# EUROPEAN PATENT APPLICATION

(11) **EP 4 729 516 A1**
(43) Date of publication of application: **22.04.2026**
(21) Application number: 24825214.0
(22) Date of filing: 18.06.2024
(51) Int. Cl.: C07D 409/06, C07D 233/64, C07D 405/06, C07C 13/44, C07C 13/465, C07C 13/48, A61K 31/381, A61K 31/382, A61K 31/4178, A61P 25/04, A61P 25/20

(54) **SUBSTITUTED IMIDAZOLE DERIVATIVE, INTERMEDIATE, PREPARATION METHOD THEREFOR AND USE THEREOF**

(30) Priority: 19.06.2023 CN 202310724980; 02.02.2024 CN 202410149322
(71) Applicant: Jiangsu NHWA Pharmaceutical Co., Ltd, Xuzhou, Jiangsu 221000 (CN)
(72) Inventor: SUN, Qingdi, Xuzhou, Jiangsu 221000 (CN); DOU, Fei, Xuzhou, Jiangsu 221000 (CN); XU, Xiangqing, Xuzhou, Jiangsu 221000 (CN); LU, Aijun, Xuzhou, Jiangsu 221000 (CN); MA, Zhen, Xuzhou, Jiangsu 221000 (CN); LI, Xuewei, Xuzhou, Jiangsu 221000 (CN); MIAO, Shen, Xuzhou, Jiangsu 221000 (CN); MEI, Zhanbiao, Xuzhou, Jiangsu 221000 (CN); WANG, Dongli, Xuzhou, Jiangsu 221000 (CN); JING, Peng, Xuzhou, Jiangsu 221000 (CN)
(74) Representative: Lavoix
(86) International application number: PCT/CN2024/099712
(87) International publication number: WO 2024/260319

(57) **Abstract**

The present invention relates to the field of medicines, and particularly relates to a compound represented by general formula (I) or a pharmaceutically acceptable salt, a stereoisomer, a tautomer and a composition containing the compound, an intermediate thereof, a preparation method therefor and a use thereof in the field of medicines.

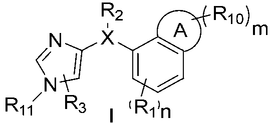

## Description

The present application claims priority to Chinese Patent Application No. 202310724980.9 filed on June 19, 2023 and entitled "SUBSTITUTED IMIDAZOLE DERIVATIVE, INTERMEDIATE, PREPARATION METHOD THEREFOR AND USE THEREOF" and Chinese Patent Application 202410149322.6 filed on February 2, 2024 and entitled "SUBSTITUTED IMIDAZOLE DERIVATIVE, INTERMEDIATE, PREPARATION METHOD THEREFOR, AND USE THEREOF", which are incorporated herein by reference in their entirety.

### TECHNICAL FIELD

The present disclosure pertains to the field of medicine, and particularly relates to a substituted imidazole derivative or a pharmaceutically acceptable salt thereof, a stereoisomer thereof, or a tautomer thereof, and a composition comprising the compound, an intermediate thereof, a preparation method therefor, and use thereof in the field of medicine.

### BACKGROUND

Adrenergic receptors are G-protein-coupled receptors, which are classified into adrenergic α receptors and β receptors. The α receptors include α1 and α2 receptors, and the β receptors include β1, β2, and β3 receptors. The α2 receptors are widely distributed in the central and peripheral nervous system and other organs and tissues (blood vessels, liver, kidney, pancreas, platelets, etc.). α2A, α2B, and α2C are three subtypes of the α2 receptors (Bylund et al., Mol. Pharmacol., 1992, 42, 1-5). α2A in the brain is mainly concentrated in the pons and medulla oblongata and is involved in the transmission of sympathetic nerve signals from the center to the periphery. Stimulation of presynaptic α2A can regulate the release of adrenaline through a negative feedback mechanism; stimulation of postsynaptic α2A can cause hyperpolarization of nerve cell membranes.

Effects of α2 receptor agonists on analgesia, anaesthesia, and sedation are well documented (Pertovaara, A., Progress in Neurobiology, 40, 691 (1993)). For example, systemic administration of clonidine has been shown to produce antinociception in a variety of species including human patients, in addition to its well-known sedative effects. Intrathecal and epidural administration of clonidine has also proven effective in producing antinociception. Another α2 receptor agonist, medetomidine, which has better α2/α1 selectivity and is more effective than clonidine for the α2 receptor, has been widely used.

Dexmedetomidine is a highly selective α2 adrenoceptor agonist, which can produce sedative, analgesic, anti-anxiety, and sympathetic nerve inhibitory effects when acting on the central and peripheral nervous systems and other organs and tissues (Maud A.S. Weerinks, et al. Clin Pharmacokinete, 2017, 56: 893-913). Due to its remarkable effects, the compound has been widely used in clinical practice at present. Although dexmedetomidine has good analgesic and sedative effects, its adverse drug reactions, particularly hypotension and bradycardia that are common, should also be emphasized. At present, there are many cases of clinical case reports of the progression of bradycardia associated with dexmedetomidine. In particular, large doses of dexmedetomidine may stimulate α2B receptors on vascular smooth muscle, leading to vasoconstriction, which results in hypertension and a reflex heart rate decrease.

Therefore, there is a need to find an α2 adrenoceptor agonist with good circulation stability, high potency, short duration, fast onset of action *in vivo,* and weak side effects, and to apply it to the preparation of a medicament for producing sedative, and/or analgesic, and/or anesthetic effects, and/or for anti-anxiety, and/or for inhibiting sympathetic nerve activity, and/or for treating insomnia, and/or for treating a mental disorder.

### SUMMARY

The following is only an exemplary description of some aspects of the present disclosure, and therefore the present disclosure is not limited thereto.

The present disclosure aims to provide a substituted imidazole derivative with α2 adrenoceptor agonist activity, or a pharmaceutically acceptable salt, a stereoisomer, or a tautomer, and a pharmaceutical composition thereof. The compound and the pharmaceutical composition can be used for preparing a medicament associated with an α2 adrenoceptor agonist, can be further used for preparing a medicament for producing sedative, and/or analgesic, and/or anesthetic effects, and/or for anti-anxiety, and/or for inhibiting sympathetic nerve activity, and/or for treating insomnia, and/or for treating a mental disorder in a human or an animal, and can be still further used for preparing a medicament for producing sedative, and/or analgesic, and/or anesthetic effects, and/or for treating insomnia, and/or for treating a mental disorder in a human or an animal.

In one aspect, the present disclosure provides a compound represented by general formula I, which is shown below, or a pharmaceutically acceptable salt, a stereoisomer, or a tautomer, 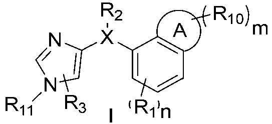
wherein X is selected from CH and N, and preferably, X is CH;
n is selected from integers of 1-3, and preferably, n is 1;
m is selected from integers of 1-8, preferably integers of 1-3;
each R₁ is independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl, and the optional substituent is independently selected from halogen and hydroxyl;
R₂ is selected from H, cyano, optionally substituted C₁-C₈ alkyl, optionally substituted C₂-C₈ alkenyl, and optionally substituted C₂-C₈ alkynyl, and the optional substituent is independently selected from halogen and hydroxyl;
R₃ is selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl, and the optional substituent is independently selected from halogen and hydroxyl;
ring A is selected from C₃-C₈ cycloalkyl, C₃-C₈ cycloalkenyl, 3- to 8-membered heterocyclyl containing 1-3 heteroatoms independently selected from oxygen, nitrogen, and sulfur, and 5- to 8-membered heteroaryl containing 1-3 heteroatoms independently selected from oxygen and sulfur, preferably C₃-C₈ cycloalkyl, 3- to 8-membered heterocyclyl containing 1 heteroatom selected from oxygen, nitrogen, and sulfur, and 5- to 8-membered heteroaryl containing 1 heteroatom selected from oxygen and sulfur; each R₁₀ is independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl;
R₁₁ is selected from H, optionally substituted C₁-C₈ alkyl, optionally substituted C₂-C₈ alkenyl, and optionally substituted C₂-C₈ alkynyl, preferably H and optionally substituted C₁-C₅ alkyl, and further preferably, R₁₁ is H; the optional substituent is selected from hydroxyl and halogen;
and the compound of formula I is not: 5-(2,3-dihydro-benzofuran-7-ylmethyl)-1-imidazole, 8-((1H-imidazol-5-yl)methyl)-1,2,3,4-tetrahydroquinoline, 5-(benzofuran-7-ylmethyl)-1H-imidazole, or 5-((5-bromobenzofuran-7-yl)methyl)-1H-imidazole.

In another aspect, the present disclosure provides a pharmaceutical composition comprising a therapeutically effective amount of the compound represented by formula I or the pharmaceutically acceptable salt thereof, the stereoisomer thereof, or the tautomer thereof described above, and optionally further comprising a pharmaceutically acceptable excipient, a carrier, an adjuvant, a vehicle, or a combination thereof.

In another aspect, the present disclosure provides use of the compound represented by formula I or the pharmaceutically acceptable salt thereof, the stereoisomer thereof, or the tautomer thereof, or the pharmaceutical composition thereof in the preparation of a medicament associated with an α2 adrenoceptor agonist, preferably in the preparation of a medicament for producing sedative, and/or analgesic, and/or anesthetic effects, and/or for anti-anxiety, and/or for inhibiting sympathetic nerve activity, and/or for treating insomnia, and/or for treating a mental disorder in a human or an animal, and further preferably in the preparation of a medicament for producing sedative, and/or analgesic, and/or anesthetic effects, and/or for treating insomnia, and/or for treating a mental disorder in a human or an animal.

In another aspect, the present disclosure provides a compound represented by formula I-A, formula I-A', formula I-B, formula I-B', formula I-C, formula I-D, or formula I-D' or a salt thereof, 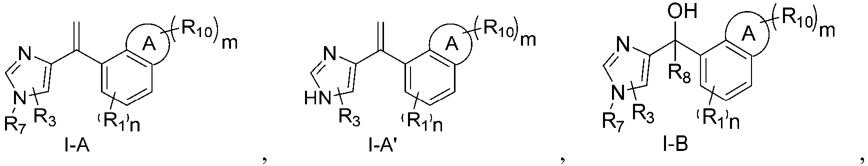 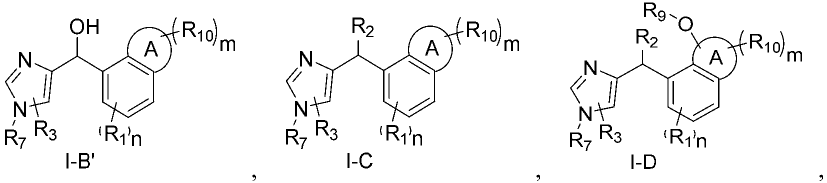 or 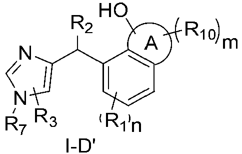
in formula I-A, n, ring A, R₁, R₃, m, and R₁₀ are as described above;
in formula I-A', n, ring A, R₁, R₃, m, and R₁₀ are as described above;
in formula I-B, n, ring A, R₁, R₃, m, and R₁₀ are as described above;
R₈ is selected from C₁-C₅ alkyl, preferably methyl, ethyl, and propyl, and further preferably, R₈ is methyl;
in formula I-B', n, ring A, R₁, R₃, m, and R₁₀ are as described above;
in formula I-C, n, ring A, R₁, R₂, R₃, m, and R₁₀ are as described above;
in formula I-D, n, ring A, R₁, R₂, R₃, m, and R₁₀ are as described above;
R₉ is selected from a hydroxyl protecting group, preferably a silyl ether hydroxyl protecting group, further preferably trimethylsilyl, tert-butyldiphenylsilyl, tert-butyldimethylsilyl, and triisopropylsilyl, and still further preferably tert-butyldimethylsilyl;
in formula I-D', n, ring A, R₁, R₂, R₃, m, and R₁₀ are as described above;
R₇ in formula I-A, formula I-B, formula I-B', formula I-C, formula I-D, or formula I-D' is selected from an amino protecting group, and preferably, R₇ is trityl.

In another aspect, the present disclosure provides a preparation method for a compound represented by formula I, which comprises the following steps: subjecting an intermediate I-B-1 to a dehydration reaction to give an intermediate I-A, then performing a hydrogenation reduction reaction to give an intermediate I-C, and subjecting the intermediate I-C to removal of an amino protecting group to give a compound represented by formula I-1; or subjecting an intermediate I-B-1 to one-step removal of an amino protecting group and water to give an intermediate I-A', and then performing a hydrogenation reduction reaction to give a compound represented by formula I-1; or subjecting an intermediate I-B' to one-step removal of an amino protecting group and a reduction reaction simultaneously to give a compound represented by formula I-2; or subjecting an intermediate I-D to removal of a hydroxyl protecting group to give an intermediate I-D', and subjecting the intermediate I-D' to removal of an amino protecting group and dehydration to give a compound represented by formula I; 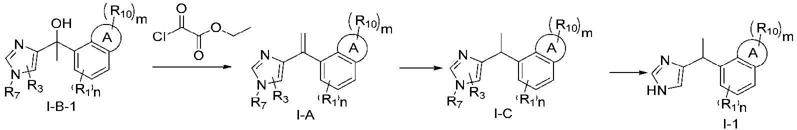 or 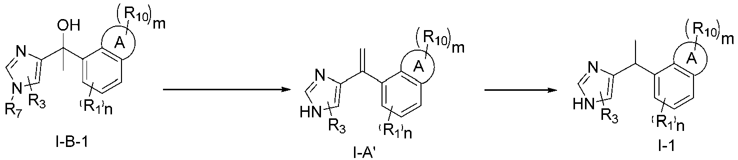 or 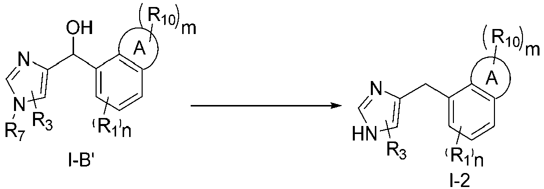 or 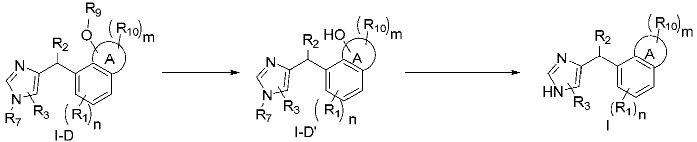
wherein n, ring A, R₁, R₂, R₃, m, and R₁₀ are as described above;
R₇ is selected from an amino protecting group, and preferably, R₇ is trityl;
R₉ is selected from a hydroxyl protecting group, preferably a silyl ether hydroxyl protecting group, further preferably trimethylsilyl, tert-butyldiphenylsilyl, tert-butyldimethylsilyl, and triisopropylsilyl, and still further preferably tert-butyldimethylsilyl.

### DETAILED DESCRIPTION

Unless otherwise specified or there is an apparent conflict in the context, all technical and scientific terms used herein have the same meanings as commonly understood by one of ordinary skill in the art to which the present disclosure pertains. In the event of a contradiction, the definition provided in the present application shall prevail. When a trade name appears herein, it is intended to refer to its corresponding commercial product or its active ingredient. All patents, published patent applications, and publications cited herein are incorporated herein by reference.

### General Terms and Definitions

The term "optional" or "optionally" means that a subsequently described event or situation may, but does not necessarily, occur, and that the description includes situations in which the event or situation occurs as well as situations in which it does not occur.

The term "optionally substituted" is used interchangeably with the term "substituted or unsubstituted", i.e., the structure or group is unsubstituted or substituted with one or more of the substituents described in the present disclosure, wherein the substitution occurs at any reasonable position on the given structure or group as permitted by valence.

Unless otherwise specified, as used herein, the attachment site of a substituent may be from any suitable position of the substituted group. When a bond of a substituent is shown to pass through a bond connecting two atoms in a ring, the substituent may be bonded to any one of the ring-forming atoms in the substitutable ring.

In general, the term "substituted" means that one or more hydrogen atoms in a given structure or group are substituted with a particular substituent. Unless otherwise indicated, substitution with one substituent may occur at each reasonable substitutable position of a group. When more than one position in a given structural formula can be substituted with one or more specific substituents selected, substitution with the substituents may occur identically or differently at each reasonable position in the structural formula.

In addition, it should be noted that unless otherwise explicitly indicated, the description "each independently" used in the present disclosure should be understood in a broad sense, and it may mean that specific items expressed by the same symbol in different groups do not affect each other, or that specific items expressed by the same symbol in the same group do not affect each other.

When the lower and upper limits of a range of numerical values are disclosed, any numerical value falling within the range and any included range are specifically disclosed. In particular, each range of values disclosed herein is to be understood as indicating each numerical value and range encompassed within a relatively broad range. When any variable (e.g., R), as well as labeled variables (e.g., R₁, R₂, R₃, R₄, R₅, R₆, R₇, etc.), occurs once or more in a composition or structure of a compound, the variable is independently defined in each case at each occurrence. For example, if a group is substituted with 0, 1, 2, or 3 R substituents, the group may optionally be substituted with up to three R substituents, and the options for each R substituent in each case are independent of each other. That is, each R substituent may be identical or different.

In each part of this specification, the substituents of the compounds disclosed in the present disclosure are disclosed according to group types or ranges. It is specifically noted that the present disclosure includes each independent secondary combination of each member of these group types and ranges. For example, the expression m-n used herein refers to the range of m to n as well as to sub-ranges consisting of the point values therein and the point values.

The term "alkyl" refers to a linear or branched saturated aliphatic hydrocarbon group consisting of carbon atoms and hydrogen atoms, which is attached to the rest of the molecule via a single bond. "Alkyl" may have 1-8 carbon atoms, i.e., "C₁-C₈ alkyl", e.g., C₁₋₅ alkyl, C₁₋₃ alkyl, C₁₋₂ alkyl, C₃ alkyl, C₄ alkyl, C₈ alkyl, C₁₋₈ alkyl, or C₃₋₈ alkyl. The alkyl may have 1-5 carbon atoms, i.e., "C₁-C₅ alkyl", or may have 1-3 carbon atoms, i.e., "C₁-C₃ alkyl", e.g., C₁₋₃ alkyl, C₁₋₂ alkyl, or C₃ alkyl. The term "C₁-C₅ alkyl" especially refers to independently disclosed methyl, ethyl, C₃ alkyl, C₄ alkyl, or C₅ alkyl. Examples of the alkyl group include, but are not limited to, methyl (Me, -CH₃), ethyl (Et, -CH₂CH₃), propyl (e.g., n-propyl (n-Pr, -CH₂CH₂CH₃), isopropyl (i-Pr, - CH(CH₃)₂)), butyl (e.g., n-butyl (n-Bu, -CH₂CH₂CH₂CH₃), isobutyl (i-Bu, - CH₂CH(CH₃)₂), sec-butyl (s-Bu, -CH(CH₃)CH₂CH₃), tert-butyl (t-Bu, -C(CH₃)₃)), pentyl (e.g., n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃)), hexyl (e.g., n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃)), heptyl, octyl, and the like. For example, the expression "C₁-C₈" or "C₁₋₈" encompasses a range of 1-8 carbon atoms and should be understood as also encompassing any sub-range and each point value therein, e.g., C₁-C₅, C₃-C₄, C₂-C₆, C₃-C₆, C₄-C₆, C₄-C₇, C₄-C₈, C₂-C₄, and the like, as well as C₁, C₂, C₃, C₄, C₅, C₆, C₇, C₈, and the like. As another example, the expression "C₁-C₅" or "C₁₋₅" encompasses a range of 1-5 carbon atoms and should be understood as also encompassing any sub-range and each point value therein, e.g., C₂-C₅, C₃-C₄, C₁-C₂, C₁-C₃, C₁-C₄, C₁-C₅, and the like, as well as C₁, C₂, C₃, C₄, C₅, and the like. As another example, the expression "C₂-C₅" or "C₂₋₅" encompasses a range of 2-5 carbon atoms and should be understood as also encompassing any sub-range and each point value therein, e.g., C₂-C₅, C₃-C₄, C₂-C₃, C₂-C₄, C₃-C₅, C₄-C₅, and the like, as well as C₂, C₃, C₄, C₅, and the like. As another example, the expression "C₁-C₈" or "C₁₋₈" encompasses a range of 1-8 carbon atoms and should be understood as also encompassing any sub-range and each point value therein, e.g., C₂-C₅, C₃-C₄, C₂-C₆, C₃-C₆, C₄-C₆, C₄-C₇, C₄-C₈, C₂-C₄, and the like, as well as C₁, C₂, C₃, C₄, C₅, C₆, C₇, C₈, and the like. As another example, the expression "three to eight membered" should be understood as encompassing any sub-range and each point value therein, e.g., three to five membered, three to six membered, three to seven membered, three to eight membered, four to five membered, four to six membered, four to seven membered, four to eight membered, five to seven membered, five to eight membered, six to seven membered, six to eight membered, and the like, as well as three membered, four membered, five membered, six membered, seven membered, eight membered, and the like. Other similar expressions herein should also be understood in a similar manner.

The term "one or more" or the similar expression "at least one" may indicate, for example, 1, 2, 3, 4, 5, 6, 7, 8, 9, 10, or more.

The term "selected from..." refers to independent selection of one or more elements from the group listed afterward, and may include a combination of two or more elements.

The term "comprise", "comprises", or "comprising" is open-ended, i.e., including what is meant by the present disclosure, but not excluding other aspects.

When it is stated that each carbon atom in a group may optionally be replaced by a heteroatom, the proviso is that the normal valency of all atoms in the group in the present case is not exceeded, and that a stable compound is formed.

When it is stated that a group is substituted with a substituent, the proviso is that the normal valency of all atoms in the group in the present case is not exceeded, and that a stable compound is formed; otherwise, the substituent is absent.

When "ring A" is defined, it does not include a double bond moiety on the benzene ring to which it is fused.

The term "heteroaryl" refers to a heteroaromatic system containing 1 to 4 heteroatoms and 5 to 14 ring atoms, wherein the heteroatom is selected from one or more of oxygen, sulfur, and nitrogen. The term "heteroaryl" may be used interchangeably with the term "heteroaromatic ring" or "heteroaromatic compound". Examples of the heteroaryl group include 5- to 14-membered heteroaryl, 5- to 8-membered heteroaryl, 5- to 10-membered monocyclic or polycyclic heteroaryl, and 5- to 6-membered monocyclic heteroaryl. "5- to 8-membered heteroaryl containing 1-3 heteroatoms independently selected from oxygen and sulfur" refers to a heteroaromatic system containing 5 to 8 ring atoms and 1-3 heteroatoms selected from oxygen and sulfur. "5- to 8-membered heteroaryl containing 1 heteroatom selected from oxygen and sulfur" refers to a heteroaromatic system containing 5 to 8 ring atoms and 1 heteroatom selected from oxygen and sulfur. The heteroaryl group may be a monocyclic or bicyclic ring system and is optionally substituted with one or more substituents described in the present disclosure.

Specific examples of the term "5- to 6-membered heteroaryl containing one oxygen atom" include, but are not limited to, furanyl and pyranyl. The heteroaryl group containing one oxygen atom may optionally be substituted with one or more substituents described in the present disclosure.

Specific embodiments of the term "5- to 6-membered heteroaryl containing one sulfur atom" include, but are not limited to, thienyl and thiopyranyl. The heteroaryl group containing one sulfur atom may optionally be substituted with one or more substituents described in the present disclosure.

The terms "heterocyclic ring" and "heterocyclyl" are used interchangeably to mean monovalent or multivalent monocyclic, bicyclic, or tricyclic ring systems containing 3-14 ring atoms, 3-8 ring atoms, or 4-6 ring atoms, wherein one or more atoms on the ring are independently replaced with heteroatoms, the heteroatom has the meaning described in the present disclosure, and the ring may be fully saturated or comprise one or more unsaturations. Unless otherwise stated, a -CH₂- group on heterocyclyl may optionally be replaced with -C(=O)-. The sulfur atom of the ring may optionally be oxidized to a S-oxide. The nitrogen atom of the ring may optionally be oxidized to a N-oxide. Examples of the heterocyclyl include 3- to 14-membered heterocyclyl, 3- to 8-membered heterocyclyl, 4- to 6-membered heterocyclyl, and 5- to 10-membered monocyclic or bicyclic heterocyclyl. "3- to 8-membered heterocyclyl containing 1-3 heteroatoms independently selected from oxygen, nitrogen, and sulfur" refers to a heterocyclic system having 3 to 8 ring atoms and containing 1-3 heteroatoms selected from oxygen, nitrogen, and sulfur. "3- to 8-membered heterocyclyl containing 1 heteroatom selected from oxygen, nitrogen, and sulfur" refers to a heterocyclic system having 3 to 8 ring atoms and containing 1 heteroatom selected from oxygen, sulfur, and nitrogen. The heterocyclyl group may be a monocyclic or bicyclic ring system and is optionally substituted with one or more substituents described in the present disclosure. Specific examples of the term "4- to 6-membered heterocyclyl containing one nitrogen atom" include, but are not limited to, azetidinyl, tetrahydropyrrolyl, hexahydropyridinyl, dihydropyrrolyl, and tetrahydropyridinyl. The heterocyclyl group containing one nitrogen atom may optionally be substituted with one or more substituents described in the present disclosure. The statement that the heterocyclyl group may be substituted with a substituent means that all substitutable positions on the "4- to 6-membered heterocyclyl containing one nitrogen atom" may be substituted with a substituent.

Specific embodiments of the term "4- to 6-membered heterocyclyl containing one sulfur atom" include, but are not limited to, thietanyl, tetrahydrothienyl, dihydrothienyl, tetrahydrothiopyranyl, dihydrothiopyranyl, 2H-thiopyranyl, and 4H-thiopyranyl. The heterocyclyl group containing one sulfur atom may optionally be substituted with one or more substituents described in the present disclosure.

Specific examples of the term "4- to 6-membered heterocyclyl containing one oxygen atom" include, but are not limited to, oxetanyl, tetrahydrofuranyl, dihydrofuranyl, tetrahydropyranyl, dihydropyranyl, 2H-pyranyl, and 4H-pyranyl. The heterocyclyl group containing one oxygen atom may optionally be substituted with one or more substituents described in the present disclosure.

The term "alkenyl" refers to a generic term for hydrocarbons containing a carbon-carbon double bond in the molecule, which are a type of unsaturated aliphatic hydrocarbon, for example: vinyl (-CH=CH₂). The "alkenyl" may have 2-8 carbon atoms, i.e., "C₂-C₈" alkenyl, e.g., C₂₋₈ alkenyl, C₂₋₄ alkenyl, C₂₋₅ alkenyl, C₃ alkenyl, C₄ alkenyl, C₆ alkenyl, C₂₋₆ alkenyl, C₃₋₈ alkenyl, C₃₋₆ alkenyl, etc. The alkenyl may also have 2-5 carbon atoms, i.e., "C₂-C₅ alkenyl", e.g., C₂₋₄ alkenyl, C₂₋₃ alkenyl, C₃ alkenyl, C₄ alkenyl, etc. Examples of the alkenyl group include, but are not limited to, vinyl (-CH=CH₂), propenyl (-CH=CH-CH₃), butenyl (-CH=CH-CH₂-CH₃, -CH₂-CH=CH-CH₃), pentenyl (-CH=CH-CH₂-CH₂-CH₃, -CH₂-CH₂-CH=CH-CH₃, -CH(CH₃)-CH=CH-CH₃), hexenyl, heptenyl, octenyl, and the like.

The term "alkynyl", a generic term for hydrocarbons containing a carbon-carbon triple bond in the molecule, refers to a type of unsaturated aliphatic hydrocarbon, for example: ethynyl (-C≡CH). The "alkynyl" may have 2-8 carbon atoms, i.e., "C₂-C₈" alkynyl, e.g., C₂₋₈ alkynyl, C₂₋₄ alkynyl, C₂₋₅ alkynyl, C₃ alkynyl, C₄ alkynyl, C₆ alkynyl, C₂₋₆ alkynyl, C₃₋₈ alkynyl, C₃₋₆ alkynyl, etc. The alkyne may also have 2-4 carbon atoms, i.e., "C₂-C₄ alkynyl", e.g., C₂₋₄ alkynyl, C₂₋₃ alkynyl, C₃ alkynyl, C₄ alkynyl, etc. Examples of the alkynyl group include, but are not limited to, ethynyl, propynyl, butynyl, pentynyl, hexynyl, heptynyl, octynyl, and the like.

The term "hydrogen (H)" means a single hydrogen atom, and such an atomic group may be attached to other groups, for example, to an oxygen atom to form a hydroxyl group.

The term "halogen" or "halo" should be understood to mean fluorine (F), chlorine (Cl), bromine (Br), or iodine (I). Preferably, the "halogen" or "halo" is selected from fluorine, chlorine, and bromine atoms, further preferably fluorine and chlorine, and still further preferably, the "halogen" or "halo" is fluorine.

The term "alkoxy" refers to an alkyl group attached to the rest of a molecule through an oxygen atom, wherein the alkyl group has the meaning described in the present disclosure. In one embodiment, the alkoxy group contains 1-8 carbon atoms. In one embodiment, the alkoxy group contains 1-5 carbon atoms; in another embodiment, the alkoxy group contains 1-3 carbon atoms. The alkoxy group may optionally be substituted with one or more substituents described in the present disclosure. Examples of the alkoxy group include, but are not limited to, methoxy (MeO, -OCH₃), ethoxy (EtO, -OCH₂CH₃), 1-propoxy (n-PrO, n-propoxy, -OCH₂CH₂CH₃), 2-propoxy (i-PrO, i-propoxy, -OCH(CH₃)₂), 1-butoxy (n-BuO, n-butoxy, -OCH₂CH₂CH₂CH₃), 2-methyl-1-propoxy (i-BuO, i-butoxy, -OCH₂CH(CH₃)₂), 2-butoxy (s-BuO, s-butoxy, - OCH(CH₃)CH₂CH₃), 2-methyl-2-propoxy (t-BuO, t-butoxy, -OC(CH₃)₃), and the like. The term "alkylthio" refers to an alkyl group attached to the rest of a molecule through a sulfur atom, wherein the alkyl group has the meaning described in the present disclosure. In one embodiment, the alkylthio group contains 1-8 carbon atoms. In one embodiment, the alkylthio group contains 1-5 carbon atoms. In another embodiment, the alkylthio group contains 1-3 carbon atoms. The alkylthio group may optionally be substituted with one or more substituents described in the present disclosure. Examples of the alkylthio group include, but are not limited to, methylthio (-SCH₃), ethylthio (-SCH₂CH₃), propylthio (-SCH₂CH₂CH₃, -SCH(CH₃)₂), butylthio (-SCH₂CH₂CH₂CH₃, - SCH₂CH(CH₃)₂, -SCH(CH₃)CH₂CH₃, -SC(CH₃)₃), and the like.

The term "cycloalkyl" broadly encompasses cyclic hydrocarbyl or cyclic alkenyl consisting of carbon atoms and hydrogen atoms, preferably containing 1 or 2 rings. The cycloalkyl may be of a monocyclic, fused polycyclic, bridged cyclic, or spiro cyclic structure. The cycloalkyl may have 3-8 carbon atoms, i.e., "C₃-C₈ cycloalkyl", or may also have 3-6 carbon atoms, i.e., "C₃-C₆ cycloalkyl", e.g., C₆ cycloalkyl, C₅ cycloalkyl, C₄ cycloalkyl, or C₃ cycloalkyl. Examples of the cycloalkyl include C₃-C₈ cycloalkyl and C₃-C₆ cycloalkyl, and specifically include: cyclopropyl, cyclopropenyl, cyclobutenyl, cyclobutyl, cyclopentyl, cyclohexyl, cyclopentenyl, cyclopentadienyl, cyclohexenyl, cyclohexadienyl, and the like. The term also encompasses the case where the C atom may be substituted with oxo (=O). Typical cycloalkyl refers to a cyclic saturated hydrocarbon group consisting of carbon atoms and hydrogen atoms. Further, the term "cycloalkenyl" typically refers to an alicyclic hydrocarbon containing a carbon-carbon double bond in a molecule consisting of carbon atoms and hydrogen atoms. The cycloalkenyl may have 3-8 carbon atoms, i.e., "C₃-C₈ cycloalkenyl", or may also have 3-6 carbon atoms, i.e., "C₃-C₆ cycloalkenyl", e.g., C₆ cycloalkenyl, C₅ cycloalkenyl, C₄ cycloalkenyl, or C₃ cycloalkenyl. Examples of the cycloalkenyl include C₃-C₈ cycloalkenyl and C₃-C₆ cycloalkenyl, and specifically include: cyclopropenyl, cyclobutenyl, cyclopentenyl, cyclohexenyl, cyclohexadienyl, and the like. The term also encompasses the case where the C atom may be substituted with oxo (=O).

The term "hydroxyl" refers to an -OH group.

Examples of the term "amino protecting group" include, but are not limited to: trityl, benzyloxycarbonyl, tert-butoxycarbonyl, fluorenylmethoxycarbonyl, allyloxycarbonyl, p-methoxybenzyl, and benzyl.

The term "hydroxyl protecting group" is preferably a silyl ether hydroxyl protecting group, and examples include, but are not limited to: trimethylsilyl (TMS), tert-butyldiphenylsilyl (TBDPS), tert-butyldimethylsilyl (TBS/TBDMS), and triisopropylsilyl (TIPS).

The term "stereoisomer" refers to a compound having the same chemical structure, but having a different arrangement of atoms or groups in space. Stereoisomers include enantiomers, diastereomers, conformational isomers (rotamers), geometric isomers (cis/trans isomers), atropisomers, and the like.

For example, stereoisomers of 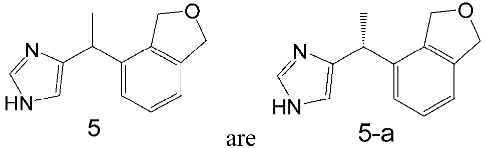 and 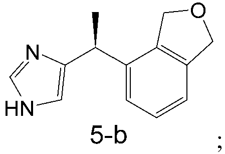 stereoisomers of 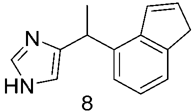 are 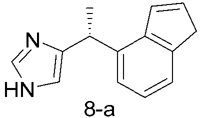 and 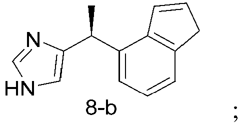 stereoisomers of 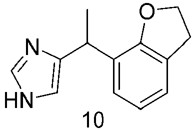 are

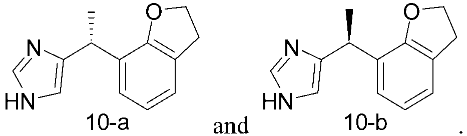

The term "tautomer" refers to a structural isomer having different energies that are interconvertible via a low energy barrier. If tautomerism is possible (e.g., in a solution), a chemical equilibrium of tautomers can be achieved. For example, proton tautomers (also known as prototropic tautomers) include interconversion via migration of a proton, such as keto-enol isomerization and imine-enamine isomerization.

The term "pharmaceutically acceptable salt" refers to an organic salt or an inorganic salt of the compound of the present disclosure.

The term "pharmaceutically acceptable carrier" refers to those substances that do not have a significant irritating effect on organisms and do not impair the biological activity and properties of the active compound. The term "pharmaceutically acceptable carrier" includes, but is not limited to, a glidant, a sweetener, a diluent, a preservative, a dye/colorant, a flavoring agent, a surfactant, a wetting agent, a dispersant, a disintegrant, a stabilizer, a solvent, or an emulsifier.

The following detailed description of the present disclosure is intended to illustrate nonlimiting embodiments by examples and to enable others skilled in the art to more fully understand the technical solutions of the present disclosure, their principles, and their practical applications, so that others skilled in the art may modify and implement the present disclosure in various forms to allow it to be optimally adapted to the requirements of particular use.

### Compound of Formula I

The present disclosure provides a compound represented by formula I or a pharmaceutically acceptable salt thereof, a stereoisomer thereof, or a tautomer thereof, 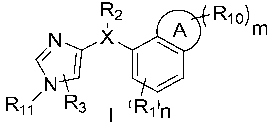
wherein X is selected from CH and N, and preferably, X is CH;
n is selected from integers of 1-3, and preferably, n is 1;
m is selected from integers of 1-8, preferably integers of 1-3;
each R₁ is independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl, and the optional substituent is independently selected from halogen and hydroxyl;
R₂ is selected from H, cyano, optionally substituted C₁-C₈ alkyl, optionally substituted C₂-C₈ alkenyl, and optionally substituted C₂-C₈ alkynyl, and the optional substituent is independently selected from halogen and hydroxyl;
R₃ is selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl, and the optional substituent is independently selected from halogen and hydroxyl;
ring A is selected from C₃-C₈ cycloalkyl, C₃-C₈ cycloalkenyl, 3- to 8-membered heterocyclyl containing 1-3 heteroatoms independently selected from oxygen, nitrogen, and sulfur, and 5- to 8-membered heteroaryl containing 1-3 heteroatoms independently selected from oxygen and sulfur, preferably C₃-C₈ cycloalkyl, 3- to 8-membered heterocyclyl containing 1 heteroatom selected from oxygen, nitrogen, and sulfur, and 5- to 8-membered heteroaryl containing 1 heteroatom selected from oxygen and sulfur; each R₁₀ is independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl;
R₁₁ is selected from H, optionally substituted C₁-C₈ alkyl, optionally substituted C₂-C₈ alkenyl, and optionally substituted C₂-C₈ alkynyl, preferably H and optionally substituted C₁-C₅ alkyl, and further preferably, R₁₁ is H; the optional substituent is selected from hydroxyl and halogen;
and the compound of formula I is not: 5-(2,3-dihydro-benzofuran-7-ylmethyl)-1-imidazole, 8-((1H-imidazol-5-yl)methyl)-1,2,3,4-tetrahydroquinoline, 5-(benzofuran-7-ylmethyl)-1H-imidazole, or 5-((5-bromobenzofuran-7-yl)methyl)-1H-imidazole.

In one embodiment, the 3- to 8-membered heterocyclyl containing one heteroatom selected from oxygen, nitrogen, and sulfur is 3- to 8-membered heterocyclyl containing one sulfur atom, 3- to 8-membered heterocyclyl containing one oxygen atom, or 3- to 8-membered heterocyclyl containing one nitrogen atom.

In one embodiment, the 3- to 8-membered heterocyclyl containing one sulfur atom is 4- to 6-membered heterocyclyl containing one sulfur atom. In one preferred embodiment, the 3- to 8-membered heterocyclyl containing one sulfur atom and/or the 4- to 6-membered heterocyclyl containing one sulfur atom are selected from thietanyl, tetrahydrothienyl, dihydrothienyl, tetrahydrothiopyranyl, and dihydrothiopyranyl.

In one embodiment, the 3- to 8-membered heterocyclyl containing one oxygen atom is 4- to 6-membered heterocyclyl containing one oxygen atom. In one preferred embodiment, the 3- to 8-membered heterocyclyl containing one oxygen atom and/or the 4- to 6-membered heterocyclyl containing one oxygen atom are selected from oxetanyl, tetrahydrofuranyl, dihydrofuranyl, tetrahydropyranyl, and dihydropyranyl.

In one embodiment, the 3- to 8-membered heterocyclyl containing one nitrogen atom is 4- to 6-membered heterocyclyl containing one nitrogen atom. In one preferred embodiment, the 3- to 8-membered heterocyclyl containing one nitrogen atom and/or the 4- to 6-membered heterocyclyl containing one nitrogen atom are selected from azetidinyl, tetrahydropyrrolyl, dihydropyrrolyl, hexahydropyridinyl, tetrahydropyridinyl, and dihydropyridinyl.

In one embodiment, the 5- to 8-membered heteroaryl containing one heteroatom selected from oxygen and sulfur is 5- to 8-membered heteroaryl containing one oxygen atom or 5- to 8-membered heteroaryl containing one sulfur atom.

In one embodiment, the 5- to 8-membered heteroaryl containing one oxygen atom is 5-to 6-membered heteroaryl containing one oxygen atom. In one preferred embodiment, the 5- to 8-membered heteroaryl containing one oxygen atom and/or the 5- to 6-membered heteroaryl containing one oxygen atom are selected from furanyl and pyranyl.

In one embodiment, the 5- to 8-membered heteroaryl containing one sulfur atom is 5-to 6-membered heteroaryl containing one sulfur atom. In one preferred embodiment, the 5- to 8-membered heteroaryl containing one sulfur atom and/or the 5- to 6-membered heteroaryl containing one sulfur atom are selected from thienyl and thiopyranyl.

In one embodiment, the C₁-C₈ alkyl is selected from C₁-C₅ alkyl and C₁-C₃ alkyl. In one specific embodiment, the C₁-C₈ alkyl, the C₁-C₅ alkyl, and the C₁-C₃ alkyl are each independently selected from methyl, ethyl, propyl, butyl, and pentyl. In one more specific embodiment, the C₁-C₈ alkyl, the C₁-C₅ alkyl, and the C₁-C₃ alkyl are each independently selected from methyl, ethyl, and propyl.

In one embodiment, the propyl includes, but is not limited to, n-propyl (n-Pr, - CH₂CH₂CH₃) or isopropyl (i-Pr, -CH(CH₃)₂). The butyl includes, but is not limited to, n-butyl (n-Bu, -CH₂CH₂CH₂CH₃), isobutyl (i-Bu, -CH₂CH(CH₃)₂), sec-butyl (s-Bu, - CH(CH₃)CH₂CH₃), or tert-butyl (t-Bu, -C(CH₃)₃). The pentyl includes, but is not limited to, n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), or 2-methyl-1-butyl (-CH₂CH(CH₃)CH₂CH₃).

In one embodiment, the C₁-C₈ alkoxy is selected from C₁-C₅ alkoxy and C₁-C₃ alkoxy. In one specific embodiment, the C₁-C₈ alkoxy, the C₁-C₅ alkoxy, and the C₁-C₃ alkoxy are each independently selected from methoxy, ethoxy, propoxy, butoxy, and pentyloxy. In one more specific embodiment, the C₁-C₈ alkoxy, the C₁-C₅ alkoxy, and the C₁-C₃ alkoxy are each independently selected from methoxy, ethoxy, and propoxy.

In one embodiment, the C₃-C₈ cycloalkyl is C₃-C₆ cycloalkyl. In one preferred embodiment, the C₃-C₈ cycloalkyl and the C₃-C₆ cycloalkyl are selected from cyclopropyl, cyclobutyl, cyclopentyl, cyclohexyl, cyclobutenyl, cyclopentenyl, cyclopentadienyl, cyclohexenyl, and cyclohexadienyl. In one more preferred embodiment, the C₃-C₈ cycloalkyl and the C₃-C₆ cycloalkyl are selected from cyclopropyl, cyclobutenyl, cyclobutyl, cyclopentyl, cyclohexyl, cyclopentenyl, and cyclohexenyl.

In one embodiment, the C₃-C₈ cycloalkenyl is C₃-C₆ cycloalkenyl. In one preferred embodiment, the C₃-C₈ cycloalkenyl and the C₃-C₆ cycloalkenyl are selected from cyclobutenyl, cyclopentenyl, cyclohexenyl, and cyclohexadienyl. In one more preferred embodiment, the C₃-C₈ cycloalkenyl and the C₃-C₆ cycloalkenyl are selected from cyclobutenyl, cyclopentenyl, and cyclohexenyl.

In one embodiment, a C atom in the C₃-C₈ cycloalkyl and/or the C₃-C₆ cycloalkyl is substituted with oxo (=O).

In one embodiment, a C atom in the C₃-C₈ cycloalkenyl and/or the C₃-C₆ cycloalkenyl is substituted with oxo (=O). In one embodiment, the halogen is selected from fluorine, chlorine, bromine, and iodine. In one preferred embodiment, the halogen is selected from fluorine, chlorine, and bromine. In one more preferred embodiment, the halogen is selected from fluorine and chlorine. In one particularly preferred embodiment, the halogen is fluorine.

In one embodiment, the C₁-C₈ alkylthio is selected from C₁-C₅ alkylthio and C₁-C₃ alkylthio. In one specific embodiment, the C₁-C₈ alkylthio, the C₁-C₅ alkylthio, and the C₁-C₃ alkylthio are each independently selected from methylthio, ethylthio, propylthio, butylthio, and pentylthio. In one more specific embodiment, the C₁-C₈ alkylthio, the C₁-C₅ alkylthio, and the C₁-C₃ alkylthio are each independently selected from methylthio, ethylthio, and propylthio.

In one embodiment, the C₂-C₈ alkenyl is selected from C₂-C₄ alkenyl.

In one embodiment, the C₂-C₈ alkynyl is selected from C₂-C₄ alkynyl.

In one embodiment, ring A is selected from C₃-C₈ cycloalkyl, C₃-C₈ cycloalkenyl, 4- to 6-membered heterocyclyl containing 1 heteroatom selected from oxygen, nitrogen, and sulfur, and 5- to 6-membered heteroaryl containing 1 heteroatom selected from oxygen and sulfur, preferably azetidinyl, oxetanyl, thietanyl, thienyl, furanyl, thiopyranyl, pyranyl, tetrahydropyrrolyl, hexahydropyridinyl, dihydropyrrolyl, tetrahydropyridinyl, tetrahydrothienyl, dihydropyridinyl, dihydrothienyl, tetrahydrothiopyranyl, dihydrothiopyranyl, tetrahydrofuranyl, dihydrofuranyl, tetrahydropyranyl, dihydropyranyl, cyclopropyl, cyclobutyl, cyclopentyl, cyclohexyl, cyclobutenyl, cyclopentenyl, cyclopentadienyl, cyclohexenyl, and cyclohexadienyl, and further preferably tetrahydropyrrolyl, hexahydropyridinyl, dihydropyrrolyl, tetrahydropyridinyl, thienyl, furanyl, thiopyranyl, pyranyl, dihydropyridinyl, tetrahydrothiopyranyl, dihydrothiopyranyl, tetrahydrothienyl, dihydrothienyl, tetrahydrofuranyl, dihydrofuranyl, cyclobutyl, cyclobutenyl, cyclopentyl, cyclohexyl, cyclopentenyl, cyclohexenyl, and cyclopentadienyl.

In one embodiment, R₁ is selected from H, halogen, optionally substituted C₁-C₈ alkyl, optionally substituted C₁-C₈ alkoxy, hydroxyl, optionally substituted C₁-C₈ alkylthio, and optionally substituted C₃-C₆ cycloalkyl, preferably H, halogen, optionally substituted C₁-C₅ alkyl, optionally substituted C₁-C₅ alkoxy, hydroxyl, optionally substituted C₁-C₈ alkylthio, and optionally substituted C₃-C₆ cycloalkyl, further preferably H, halogen, optionally substituted C₁-C₅ alkyl, and hydroxyl, and further preferably H, halogen, and optionally substituted C₁-C₅ alkyl, and further preferably, R₁ is H or -F; the optional substituent is selected from hydroxyl and halogen, and preferably, the optional substituent is F.

In one embodiment, R₁ is selected from H and halogen.

In one embodiment, R₁ is H.

In one embodiment, R₁ is halogen (e.g., fluorine).

In one embodiment, R₃ is selected from H, halogen, optionally substituted C₁-C₈ alkyl, optionally substituted C₁-C₈ alkoxy, hydroxyl, optionally substituted C₁-C₈ alkylthio, and optionally substituted C₃-C₆ cycloalkyl, preferably H, halogen, optionally substituted C₁-C₅ alkyl, optionally substituted C₁-C₅ alkoxy, hydroxyl, and optionally substituted C₃-C₆ cycloalkyl, and further preferably H, halogen, and optionally substituted C₁-C₅ alkyl, and still further preferably, R₃ is H; the optional substituent is selected from hydroxyl and halogen, and preferably, the optional substituent is F.

In one embodiment, R₃ is H.

In one embodiment, each R₁₀ is independently selected from H, halogen, optionally substituted C₁-C₈ alkyl, optionally substituted C₁-C₈ alkoxy, hydroxyl, optionally substituted C₁-C₈ alkylthio, and optionally substituted C₃-C₆ cycloalkyl, preferably H, halogen, optionally substituted C₁-C₅ alkyl, optionally substituted C₁-C₅ alkoxy, hydroxyl, optionally substituted C₁-C₅ alkylthio, and optionally substituted C₃-C₆ cycloalkyl, and further preferably H, halogen, and optionally substituted C₁-C₅ alkyl; the optional substituent is independently selected from halogen, C₁-C₈ alkyl, and hydroxyl, preferably halogen, C₁-C₅ alkyl, and hydroxyl, and further preferably, the optional substituent is halogen, still further preferably F. In one embodiment, R₂ is selected from H and optionally substituted C₁-C₈ alkyl, preferably H and optionally substituted C₁-C₅ alkyl; the optional substituent is independently selected from halogen and hydroxyl.

In one embodiment, R₂ is selected from optionally substituted C₁-C₅ alkyl; the optional substituent is independently selected from halogen and hydroxyl. In one preferred embodiment, R₂ is selected from C₁-C₅ alkyl.

In one embodiment, the compound represented by formula I is represented by formula I-a' or formula I-b':

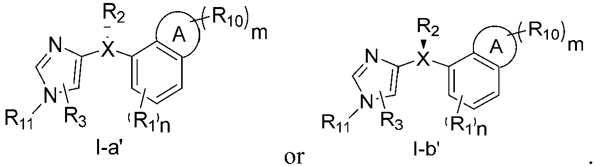

In one embodiment, ring A is selected from 5- to 6-membered heterocyclyl containing one oxygen atom and 5- to 6-membered heteroaryl containing one oxygen atom, preferably 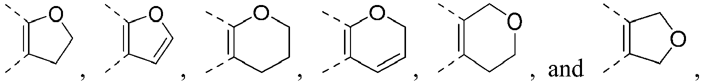 and further preferably 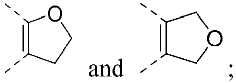 R₂ is selected from optionally substituted C₁-C₈ alkyl; the optional substituent is independently selected from halogen and hydroxyl.

In one embodiment, ring A is selected from 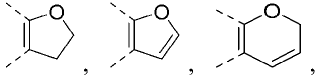 and

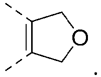

In one embodiment, ring A is selected from 4- to 6-membered heterocyclyl containing one sulfur atom and 5- to 6-membered heteroaryl containing one sulfur atom, preferably 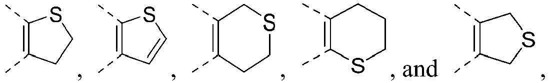 and further preferably

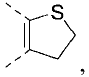

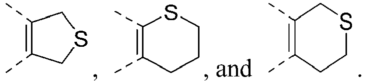

In one embodiment, ring A is selected from

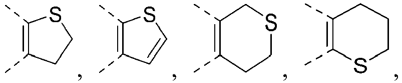

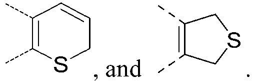

In one embodiment, ring A is selected from 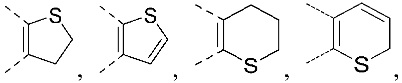 and

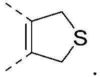

In one embodiment, ring A is selected from 4- to 6-membered heterocyclyl containing one nitrogen atom, preferably

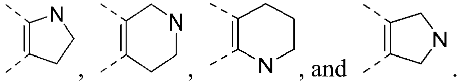

In one embodiment, ring A is selected from 4- to 6-membered heterocyclyl containing one nitrogen atom, preferably 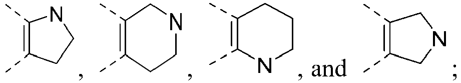 and R₂ is selected from optionally substituted C₁-C₈ alkyl; the optional substituent is independently selected from halogen and hydroxyl.

In one embodiment, ring A is

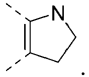

In one embodiment, ring A is selected from C₃-C₈ cycloalkyl, preferably 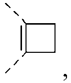 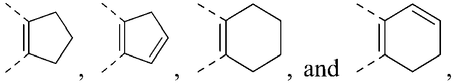 and further preferably 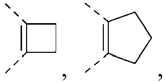 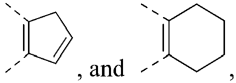 and still further preferably, ring A is

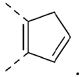

In one specific embodiment, the compound represented by formula I is represented by formula II: 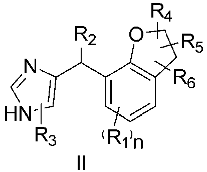
wherein R₂ is selected from optionally substituted C₁-C₈ alkyl, and the optional substituent is independently selected from halogen and hydroxyl;
n, R₁, and R₃ are as described above;
R₄, R₅, and R₆ are each independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl.

In one embodiment, R₄, R₅, and R₆ are each independently selected from H, halogen, and optionally substituted C₁-C₈ alkyl; the optional substituent is independently selected from halogen and hydroxyl.

In one specific embodiment, the compound represented by formula I is represented by formula III: 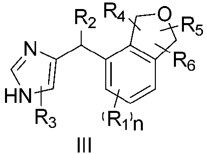
wherein, R₂ is selected from optionally substituted C₁-C₈ alkyl, and the optional substituent is independently selected from halogen, C₁-C₈ alkoxy, and hydroxyl;
n, R₁, and R₃ are as described above;
R₄, R₅, and R₆ are each independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl.

In one embodiment, R₄, R₅, and R₆ are each independently selected from H, halogen, and optionally substituted C₁-C₈ alkyl; the optional substituent is independently selected from halogen and hydroxyl.

In one specific embodiment, the compound represented by formula I is represented by formula IV: 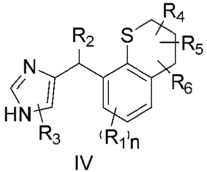
wherein n, R₁, R₂, and R₃ are as described above;
R₄, R₅, and R₆ are each independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl.

In one embodiment, R₄, R₅, and R₆ are each independently selected from H, halogen, and optionally substituted C₁-C₈ alkyl; the optional substituent is independently selected from halogen and hydroxyl.

In one specific embodiment, the compound represented by formula I is represented by formula V: 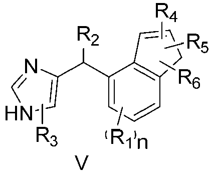
wherein n, R₁, R₂, and R₃ are as described above;
R₄, R₅, and R₆ are each independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl.

In one embodiment, R₄, R₅, and R₆ are each independently selected from H, halogen, and optionally substituted C₁-C₈ alkyl; the optional substituent is independently selected from halogen and hydroxyl.

In one specific embodiment, the compound represented by formula I is represented by formula VI: 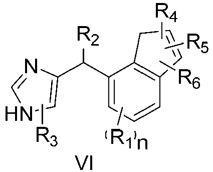
wherein n, R₁, R₂, and R₃ are as described above;
R₄, R₅, and R₆ are each independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl.

In one embodiment, R₄, R₅, and R₆ are each independently selected from H, halogen, and optionally substituted C₁-C₈ alkyl; the optional substituent is independently selected from halogen and hydroxyl.

In one specific embodiment, the compound of formula I is selected from any one of the following compounds:

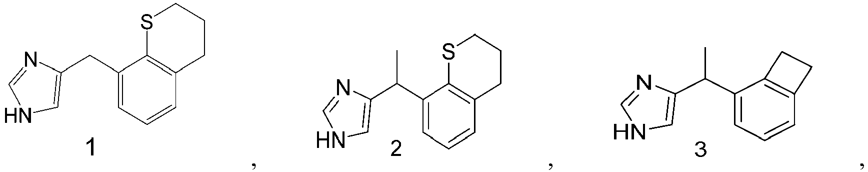

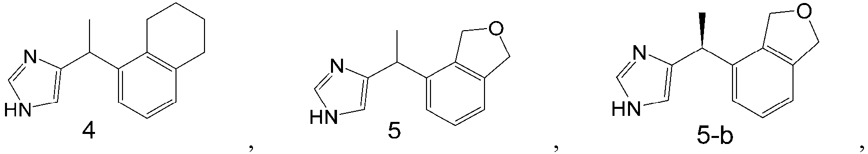

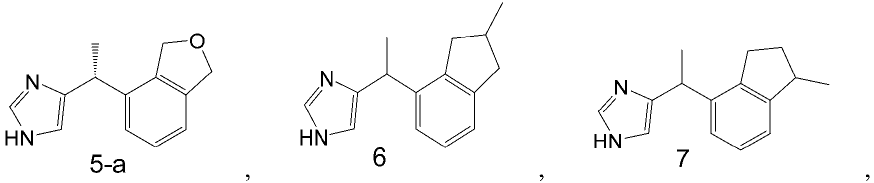

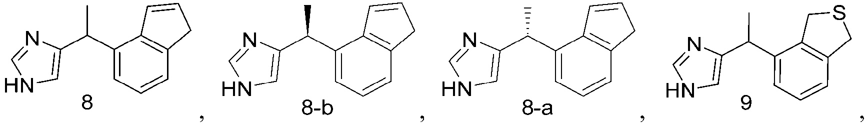

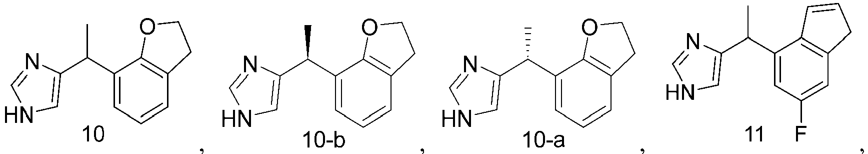

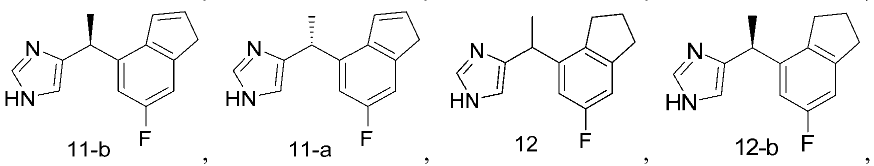

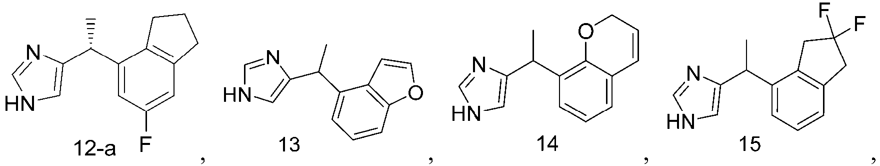

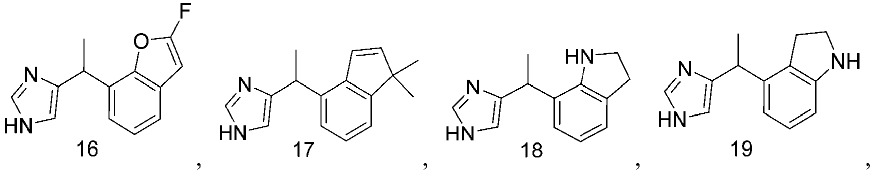

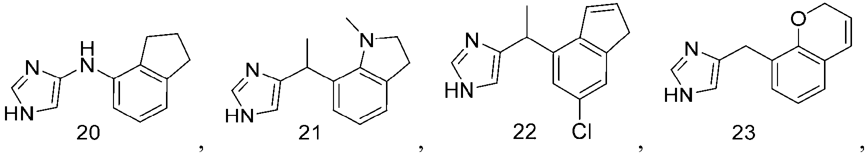

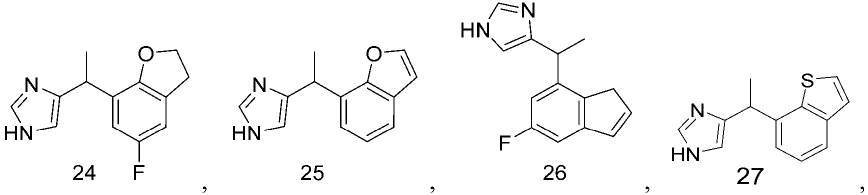

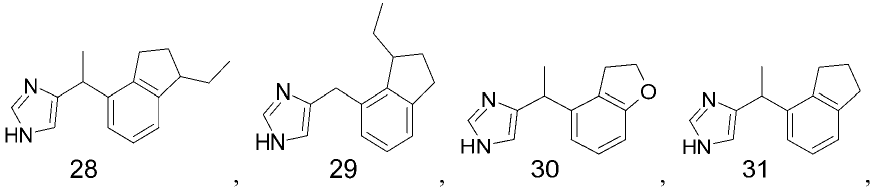

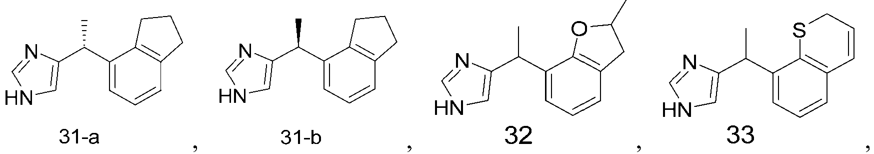

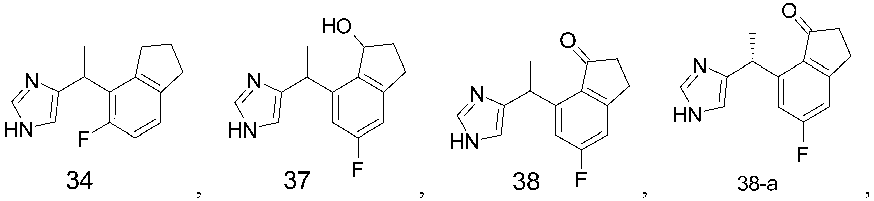

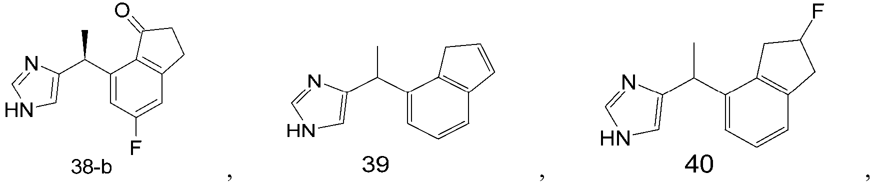

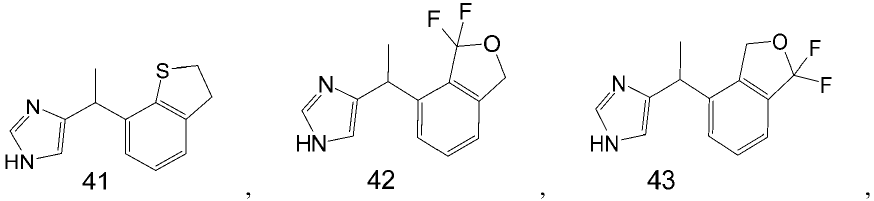

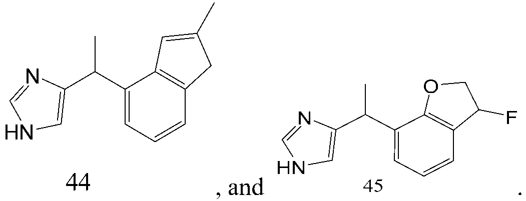

### Intermediate

The present disclosure provides a compound represented by formula I-A, formula I-A', formula I-B, formula I-B', formula I-C, formula I-D, or formula I-D' or a salt thereof, 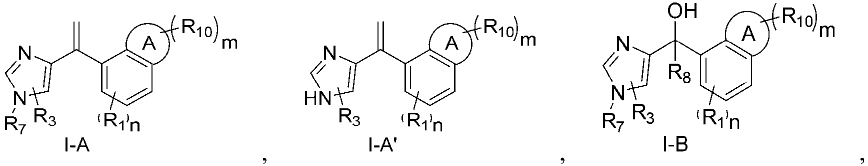 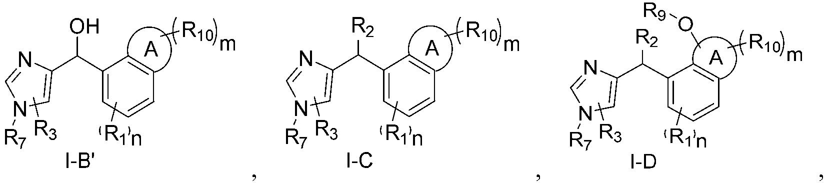 or 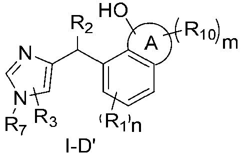
in formula I-A, n, ring A, R₁, R₃, m, and R₁₀ are as described above;
in formula I-A', n, ring A, R₁, R₃, m, and R₁₀ are as described above;
in formula I-B, n, ring A, R₁, R₃, m, and R₁₀ are as described above;
R₈ is selected from C₁-C₅ alkyl, preferably methyl, ethyl, and propyl, and further preferably, R₈ is methyl;
in formula I-B', n, ring A, R₁, R₃, m, and R₁₀ are as described above;
in formula I-C, n, ring A, R₁, R₂, R₃, m, and R₁₀ are as described above;
in formula I-D, n, ring A, R₁, R₂, R₃, m, and R₁₀ are as described above;
R₉ is selected from a hydroxyl protecting group, preferably a silyl ether hydroxyl protecting group, further preferably trimethylsilyl, tert-butyldiphenylsilyl, tert-butyldimethylsilyl, and triisopropylsilyl, and still further preferably tert-butyldimethylsilyl;
in formula I-D', n, ring A, R₁, R₂, R₃, m, and R₁₀ are as described above;
R₇ in formula I-A, formula I-B, formula I-B', formula I-C, formula I-D, or formula I-D' is selected from an amino protecting group, and preferably, R₇ is trityl.

### Preparation Method

The present disclosure provides a preparation method for a compound represented by formula I, which comprises the following steps: subjecting an intermediate I-B-1 to a dehydration reaction to give an intermediate I-A, then performing a hydrogenation reduction reaction to give an intermediate I-C, and subjecting the intermediate I-C to removal of an amino protecting group to give a compound represented by formula I-1; or subjecting an intermediate I-B-1 to one-step removal of an amino protecting group and water to give an intermediate I-A', and then performing a hydrogenation reduction reaction to give a compound represented by formula I-1; or subjecting an intermediate I-B' to one-step removal of an amino protecting group and a reduction reaction simultaneously to give a compound represented by formula I-2; or subjecting an intermediate I-D to removal of a hydroxyl protecting group to give an intermediate I-D', and subjecting the intermediate I-D' to removal of an amino protecting group and dehydration to give a compound represented by formula I; 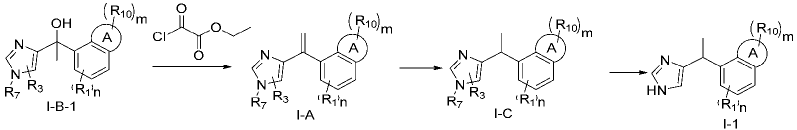 or 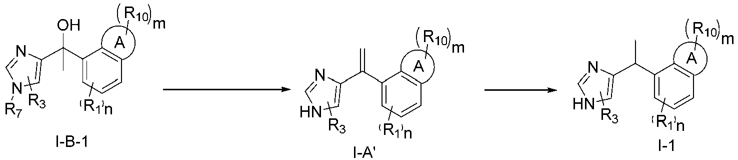 or 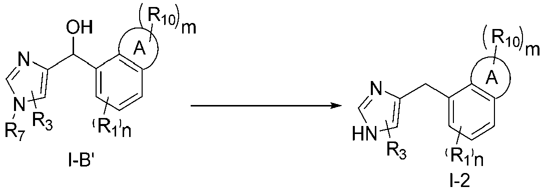 or 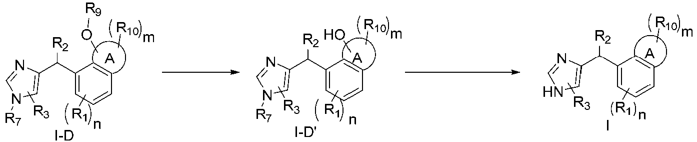
wherein n, ring A, R₁, R₂, R₃, m, and R₁₀ are as described above;
R₇ is selected from an amino protecting group, and preferably, R₇ is trityl;
R₉ is selected from a hydroxyl protecting group, preferably a silyl ether hydroxyl protecting group, further preferably trimethylsilyl, tert-butyldiphenylsilyl, tert-butyldimethylsilyl, and triisopropylsilyl, and still further preferably tert-butyldimethylsilyl.

### Beneficial Technical Effects of the Present Disclosure

Compared with the prior art, the technical solutions of the present disclosure have the following advantages:
The present disclosure relates to a class of novel structural compounds. These compounds exhibit good α2 adrenoceptor agonist activity, can be used for preparing a medicament associated with an α2 adrenoceptor agonist, can be further used for preparing a medicament for producing sedative, and/or analgesic, and/or anesthetic effects, and/or for anti-anxiety, and/or for inhibiting sympathetic nerve activity, and/or for treating insomnia, and/or for treating a mental disorder in a human or an animal, and can be still further used for preparing a medicament for producing sedative, and/or analgesic, and/or anesthetic effects, and/or for treating insomnia, and/or for treating a mental disorder in a human or an animal. These compounds have relatively good pharmacodynamic activity, and also exhibit good pharmacokinetic properties, good circulation stability, high potency, short duration, fast onset of action *in vivo,* weak side effects, and relatively good clinical application prospects.

### Example

Examples of the present disclosure are described in detail below. The examples described below are exemplary and are intended to explain the present disclosure only, and they should not be construed as limiting the present disclosure. Unless otherwise indicated, the proportions, percentages, and the like referred to herein are calculated by weight.

### Synthetic Examples

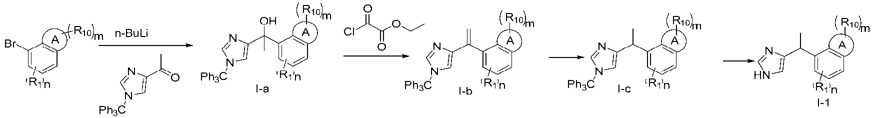

### Synthesis scheme 1 for some examples

A bromo-aromatic cyclic compound undergoes lithium-halogen exchange under the action of butyllithium, followed by a nucleophilic attack on 1-trityl-4-acetylimidazole to give an intermediate I-a. I-a undergoes dehydration under the action of ethyl oxalyl monochloride/triethylamine to give an intermediate I-b. I-b undergoes hydrogenation reduction under the action of Pd/C to give an intermediate I-c. I-c undergoes removal of a protecting group under the action of trifluoroacetic acid to give a compound represented by formula I-1.

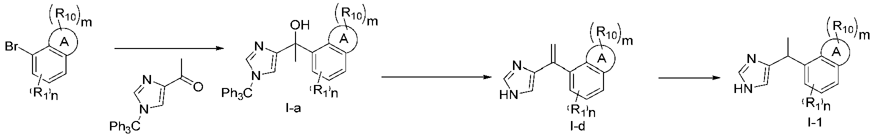

### Synthesis scheme 2 for some examples

A bromo-aromatic cyclic compound undergoes lithium-halogen exchange under the action of butyllithium, followed by a nucleophilic attack on 1-trityl-4-acetylimidazole to give an intermediate I-a. I-a undergoes one-step removal of trityl and water under the action of trifluoroacetic acid/triethylsilane to give an intermediate I-d. I-d undergoes hydrogenation reduction under the action of Pd/C to give a compound represented by formula I-1.

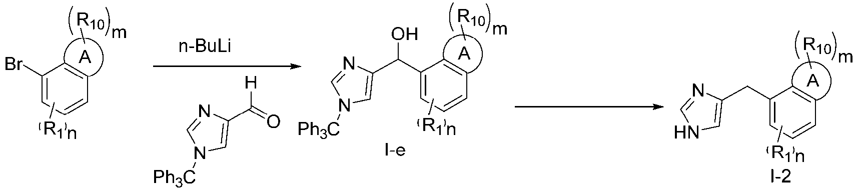

### Synthesis scheme 3 for some examples

A bromo-aromatic cyclic compound undergoes lithium-halogen exchange under the action of butyllithium, followed by a nucleophilic attack on 1-trityl-4-formylimidazole to give an intermediate I-e. I-e undergoes one-step removal of trityl and reduction under the action of trifluoroacetic acid/triethylsilane to give a compound represented by formula I-2.

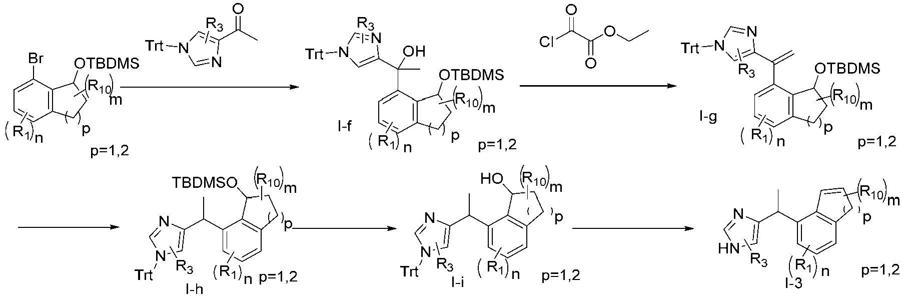

### Synthesis scheme 4 for some examples

A bromo-aromatic cyclic compound undergoes lithium-halogen exchange under the action of butyllithium, followed by a nucleophilic attack on 1-trityl-4-acetylimidazole to give an intermediate I-f. I-f undergoes dehydration under the action of ethyl oxalyl monochloride/triethylamine to give an intermediate I-g. I-g undergoes hydrogenation reduction under the action of Pd/C to give an intermediate I-h. I-h undergoes removal of a protecting group under the action of TBAF to give an intermediate I-i. I-i undergoes removal of a protecting group and dehydration under the action of p-toluenesulfonic acid to give a compound represented by formula I-3.

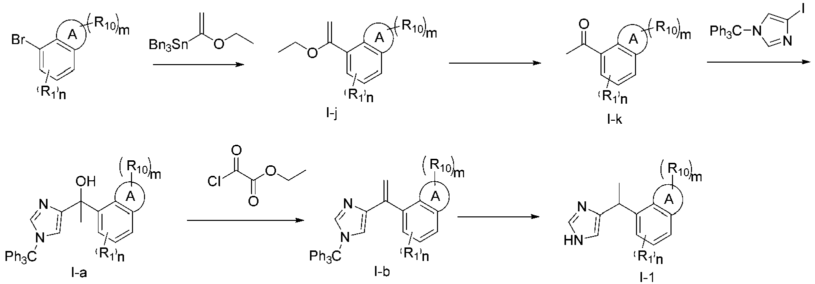

### Synthesis scheme 5 for some examples

A bromo-aromatic cyclic compound undergoes a coupling reaction with tribenzyl(1-ethoxyvinyl)stannane under the catalytic action of tetrakis(triphenylphosphine)palladium(0) to give an intermediate I-j. I-j undergoes hydrolysis under the action of hydrochloric acid to give an intermediate I-k. I-k undergoes a nucleophilic reaction with 4-iodo-1-(trityl)imidazole under the action of i-PrMgBr to give an intermediate I-a. I-a undergoes dehydration under the action of ethyl oxalyl monochloride/triethylamine to give an intermediate I-b. I-b undergoes hydrogenation reduction under the action of Pd/C to give an intermediate I-c. I-c undergoes removal of a protecting group under the action of trifluoroacetic acid to give a compound represented by formula I-1.

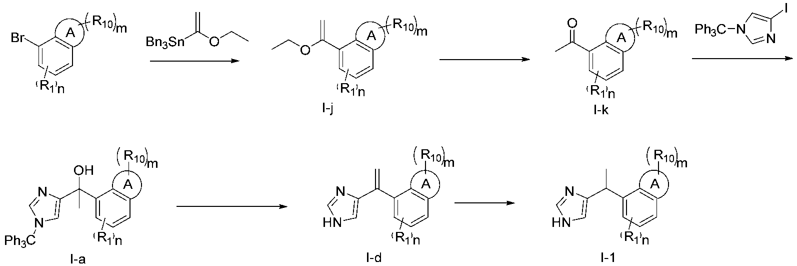

### Synthesis scheme 6 for some examples

A bromo-aromatic cyclic compound undergoes a coupling reaction with tribenzyl(1-ethoxyvinyl)stannane under the catalytic action of tetrakis(triphenylphosphine)palladium(0) to give an intermediate I-j. I-j undergoes hydrolysis under the action of hydrochloric acid to give an intermediate I-k. I-k undergoes a nucleophilic reaction with 4-iodo-1-(trityl)imidazole under the action of i-PrMgBr to give an intermediate I-a. I-a undergoes dehydration under the action of trifluoroacetic acid/triethylsilane to give an intermediate I-b. I-b undergoes hydrogenation reduction under the action of Pd/C to give an intermediate I-c. I-c undergoes removal of a protecting group under the action of trifluoroacetic acid to give a compound represented by formula I-1.

### Example 1. Preparation of 4-[1-(Thiochroman-8-yl)methyl]-1H-imidazole

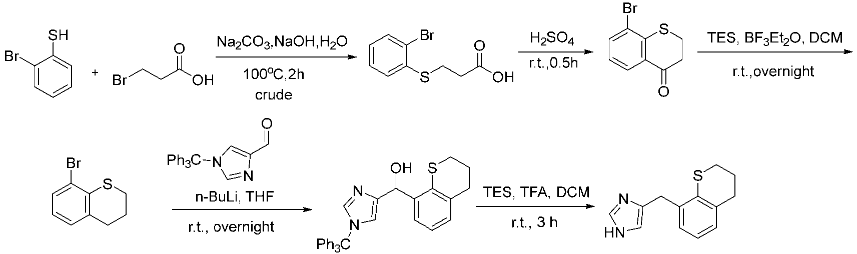

1.1 A solution of 3-bromopropionic acid (2.43 g, 15.86 mmol, 1 equiv) and Na₂CO₃ (1.68 g, 15.867 mmol, 1 equiv) in H₂O (30 mL) was added dropwise to a stirred mixture of 2-bromothiophenol (3.0 g, 15.867 mmol, 1 equiv) and NaOH (634.6 mg, 15.867 mmol, 1 equiv) in H₂O (30 mL) at 0 °C under nitrogen atmosphere. The resulting mixture was stirred at 100 °C for another 2 h. The resulting mixture was cooled to room temperature and then acidified to pH = 1 with 2 N HCl (aq). The resulting mixture was extracted with ethyl acetate (2 × 200 mL). The organic layer was washed with NaCl (aq) (3 × 200 mL) and dried over anhydrous Na2SO4. After filtration, the filtrate was concentrated under reduced pressure to give 3-(2-bromophenylthio)propanoic acid (4.02 g, crude). (ES,m/z): [M+1]=260.95, 262.95.

1.2 3-(2-Bromophenylthio)propanoic acid (4.02 g, 15.39 mmol, 1 equiv) and H₂SO₄ (40.20 mL, 754.20 mmol, 48.99 equiv) were added to a 250 mL round-bottom flask at room temperature. The resulting mixture was stirred at room temperature for 30 min under nitrogen atmosphere. The reaction was quenched by adding water/ice (150 mL) at 0 °C, and the aqueous layer was extracted with EtOAc (2 × 300 mL). The organic layer was washed with 100 mL of sodium bicarbonate (aq) and brine (2 × 400 mL) and dried over anhydrous Na₂SO₄. After filtration, the filtrate was concentrated under reduced pressure. The residue was purified by silica gel column chromatography and eluted with PE/EA (4:1) to give 8-bromo-2,3-dihydro-1-benzothiopyran-4-one (2.63 g). (ES,m/z): [M+1]= 242.94, 244.94.

1.3 8-Bromo-2,3-dihydro-1-benzothiopyran-4-one (2.63 g, 10.82 mmol, 1 equiv) and triethylsilane (24 mL) were dissolved in DCM (53 mL), and the mixture was treated for 1 min under nitrogen atmosphere. BF₃·Et₂O (24 mL, 189.39 mmol, 17.51 equiv) was added dropwise at 0 °C. The reaction mixture was then naturally warmed to room temperature. The resulting mixture was then stirred at room temperature overnight. The reaction was quenched by adding water (100 mL) at 0 °C. The resulting mixture was extracted with CH₂Cl₂ (2 × 200 mL), and the organic phases were combined, washed with water (3 × 200 mL), and dried over anhydrous Na₂SO₄. After filtration, the filtrate was concentrated under reduced pressure. The residue was purified by silica gel column chromatography and eluted with PE/EA (8:1) to give 8-bromo-3,4-dihydro-2H-1-benzothiopyran (2.04 g). (ES,m/z): [M+1]= 228.96, 230.96.

1.4 A solution of 8-bromo-3,4-dihydro-2H-1-benzothiopyran (500 mg, 2.18 mmol, 1 equiv) in THF (10 mL) was reacted with n-butyllithium (1.05 mL, 2.618 mmol, 1.2 equiv, 2.5 M in n-hexane) at -78 °C under nitrogen atmosphere for 1 h, and then 1-trityl-1*H*-imidazole-4-carbaldehyde (769.0 mg, 2.182 mmol, 1 equiv) was dissolved in THF (2 mL) and added dropwise to the system. The mixture was naturally warmed to room temperature and stirred overnight. The reaction was quenched by adding saturated ammonium chloride (aq). The resulting mixture was extracted with EtOAc (2 × 70 mL). The organic phases were combined, washed with water (3 × 100 mL), and dried over anhydrous Na₂SO₄. After filtration, the filtrate was concentrated under reduced pressure. The residue was purified by silica gel column chromatography and eluted with PE/EA (1:1) to give thiochroman-8-yl(1-trityl-1*H*-imidazol-4-yl)methanol (300 mg), (ES,m/z):[M+1]=489.65.

1.5 TFA (665.0 mg, 5.830 mmol, 10 equiv) was added dropwise to a solution of thiochroman-8-yl(1-trityl-1*H*-imidazol-4-yl)methanol (285 mg, 0.583 mmol, 1 equiv) and triethylsilane (339.1 mg, 2.915 mmol, 5 equiv) in DCM (6 mL) at room temperature under nitrogen atmosphere, and the resulting mixture was stirred at room temperature for another 3 h. The reaction was monitored by LCMS. The resulting mixture was concentrated under reduced pressure. The crude product (285 mg) was subjected to prep-HPLC to give 4-[1-(thiochroman-8-yl)methyl]-1*H*-imidazole (32.6 mg). (ES,m/z):[M+1]=231.00. ¹H NMR (400 MHz, DMSO-*d*₆) δ 7.52 (d, *J =* 1.1 Hz, 1H), 6.99 - 6.78 (m, 3H), 6.69 (s, 1H), 3.60 (s, 2H), 3.14 - 2.89 (m, 2H), 2.85 - 2.66 (m, 2H), 2.12 - 1.87 (m, 2H).

### Example 2. Preparation of 4-(1-[Thiochroman-8-yl)ethyl]-1H-imidazole

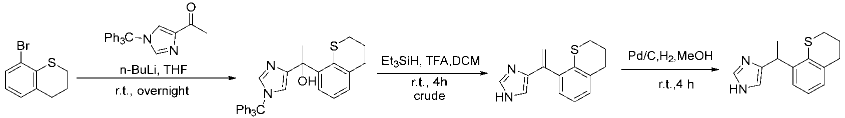

2.1 The compound was prepared according to the method of Example 1, except that 1-trityl-1H-imidazole-4-carbaldehyde was replaced by 1-[1-(trityl)imidazol-4-yl]ethanone (commercially available) to give 1-(3,4-dihydro-2H-1-benzothiopyran-8-yl)-1-[1-(trityl)imidazol-4-yl]ethanol (160 mg). (ES,m/z): [M+1]=503.68.

2.2 TFA (0.3 mL, 1.930 mmol, 10 equiv) was added to a solution of 1-(3,4-dihydro-2H-1-benzothiopyran-8-yl)-1-[1-(trityl)imidazol-4-yl]ethanol (97 mg, 0.193 mmol, 1 equiv) in DCM (2 mL) at room temperature under nitrogen atmosphere. After 1 min, triethylsilane (0.54 mL, 0.965 mmol, 5 equiv) was added dropwise at room temperature, and the resulting mixture was stirred at room temperature for 4 h and concentrated under reduced pressure to give 4-[1-(3,4-dihydro-2H-1-benzothiopyran-8-yl)vinyl]-1H-imidazole (250 mg, crude). (ES,m/z): [M+1]=243.34.

2.3 Pd/C (100 mg, 10% w/t) was added to a solution of 4-[1-(3,4-dihydro-2H-1-benzothiopyran-8-yl)vinyl]-1H-imidazole (250 mg, 1.032 mmol, 1 equiv) in MeOH (6 mL) at room temperature. The mixture was hydrogenated under a hydrogen pressure of 10 atm for 4 h, filtered through a pad of celite, concentrated under reduced pressure, and purified by prep-HPLC to give 4-[1-(3,4-dihydro-2H-1-benzothiopyran-8-yl)ethyl]-1H-imidazole (23.9 mg). (ES,m/z): [M+1]=245.20. ¹H NMR (400 MHz, DMSO-d6) δ 7.49 (d, J = 10.5 Hz, 1H), 6.98 - 6.62 (m, 4H), 4.32 (dq, J = 29.2, 6.9 Hz, 1H), 3.09 - 2.95 (m, 2H), 2.78 (q, J = 6.0 Hz, 2H), 2.04 - 1.92 (m, 2H), 1.45 (dd, J = 16.1, 7.1 Hz, 3H).

### Example 3. Preparation of 4-(1-(Bicyclo[4.2.0]octa-1(6),2,4-trien-2-yl)ethyl)-1H-imidazole

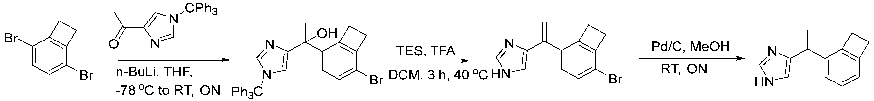

3.1 n-Butyllithium (1.3 mL, 3.17 mmol, 1.5 equiv, 2.5 M in hexane) was added dropwise to a stirred mixture of 2,5-dibromobicyclo[4.2.0]octa-1(6),2,4-triene (550 mg, 2.11 mmol, 1 equiv) in THF (1 mL) at -78 °C under nitrogen atmosphere. After the addition was completed, the mixture was stirred for 0.5 h. A solution of 1-[1-(trityl)imidazol-4-yl]ethanone (743 mg, 2.11 mmol, 1 equiv) in THF (2 mL) was added dropwise to the mixture described above at -78 °C within 3 min. The resulting mixture was stirred at room temperature overnight under nitrogen atmosphere. The reaction was quenched with a saturated aqueous ammonium chloride solution (30 mL) at room temperature. The resulting mixture was extracted with ethyl acetate (3 × 20 mL), and the organic phases were combined, washed with saturated brine (1 × 20 mL), and dried over anhydrous Na₂SO₄. The resulting mixture was concentrated under reduced pressure. The residue was purified by silica gel column chromatography and eluted with PE/EA (4:1) to give 1-(5-bromobicyclo[4.2.0]octa-1(6),2,4-trien-2-yl)-1-(1-trityl-1H-imidazol-4-yl)ethan-1-ol (216 mg). (ES, m/z): [M+1]=535, 537.

3.2. TFA (5 mL) and triethoxysilane (5 mL) were added dropwise to a stirred solution of 1-(5-bromobicyclo[4.2.0]octa-1(6),2,4-trien-2-yl)-1-(1-trityl-1H-imidazol-4-yl)ethan-1-ol (216 mg, 0.403 mmol, 1 equiv) in DCM (5 mL) at room temperature under nitrogen atmosphere. The resulting mixture was stirred at 40 °C for 3 h under nitrogen atmosphere. The resulting mixture was concentrated under reduced pressure to give 4-(1-(bicyclo[4.2.0]octa-1(6),2,4-trien-2-yl)ethyl)-1H-imidazole (230 mg, crude). The resulting crude mixture was directly used in the next step without further purification. (ES, m/z): [M+1]=275, 277.

3.3 Pd/C (44 mg, 10% w/t) was added to a stirred solution of 4-(1-(bicyclo[4.2.0]octa-1(6),2,4-trien-2-yl)ethyl)-1H-imidazole (216 mg, 0.779 mmol, 1 equiv) in MeOH (5 mL) at room temperature under hydrogen atmosphere. The resulting mixture was stirred at room temperature overnight under hydrogen atmosphere. The resulting mixture was filtered through a pad of celite, and the filter cake was washed with methanol (3 × 5 mL). The filtrate was concentrated under reduced pressure. The residue was purified by reversed-phase flash chromatography to give 4-(1-(bicyclo[4.2.0]octa-1(6),2,4-trien-2-yl)ethyl)-1H-imidazole (31.8 mg). (ES,m/z): [M+1]=199. ¹H NMR (400 MHz, Chloroform-*d*) δ 7.52 (s, 1H), 7.08 (t, *J =* 7.5 Hz, 1H), 6.98 - 6.50 (m, 3H), 4.01 (q, *J* = 7.1 Hz,1H), 3.06 - 2.83 (m, 4H), 1.57 (d, *J=* 6.8 Hz, 3H).

### Example 4. Preparation of 4-(1-(5,6,7,8-Tetrahydronaphthalen-1-yl)ethyl)-1H-imidazole

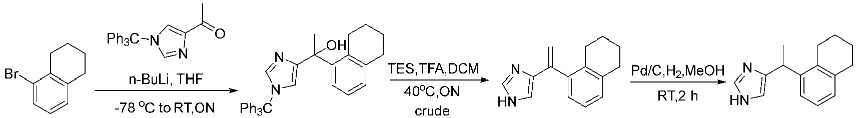

The compound was prepared according to the method of Example 3, except that 2,5-dibromobicyclo[4.2.0]octa-1(6),2,4-triene was replaced by 5-bromo-1,2,3,4-tetrahydronaphthalene (commercially available) to give 4-(1-(5,6,7,8-tetrahydronaphthalen-1-yl)ethyl)-1*H*-imidazole, (ES,m/z):[M+1]= 227.15. ¹H NMR (400 MHz, Chloroform-d) δ 7.66 (s, 1H), 7.03 (t, J = 7.5 Hz, 1H), 6.95 (s, 1H), 6.88 (d, J = 7.5 Hz, 1H), 6.73 (s, 1H), 4.33 (q, J = 7.2, 6.8 Hz, 1H), 2.81 - 2.61 (m, 4H), 1.85 - 1.68 (m, 4H), 1.55 (d, J = 6.8 Hz, 3H).

### Example 5. Preparation of 4-[1-(1,3-Dihydroisobenzofuran-4-yl)ethyl]-1H-imidazole

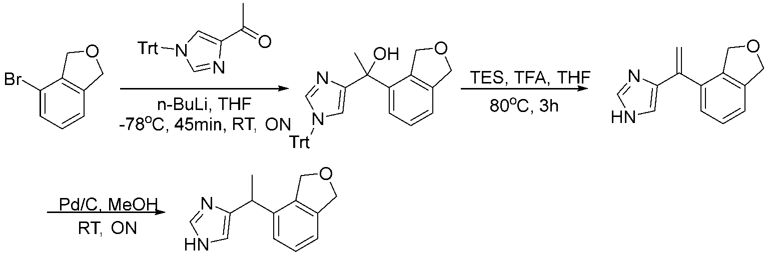

The compound was prepared according to the method of Example 3, except that 2,5-dibromobicyclo[4.2.0]octa-1(6),2,4-triene was replaced by 4-bromo-1,3-dihydroisobenzofuran (commercially available) to give 4-[1-(1,3-dihydroisobenzofuran-4-yl)ethyl]-1*H*-imidazole, (ES,m/z):[M+1]= 215. ¹H NMR (400 MHz, Methanol-d4) δ 7.56 (d, J = 1.2 Hz, 1H), 7.23 (t, J = 7.5 Hz, 1H), 7.11 (d, J = 7.5 Hz, 2H), 6.79 (t, J = 1.1 Hz, 1H), 4.91 (dt, J = 60.3, 2.1 Hz, 4H), 4.05 (q, J = 7.2 Hz, 1H), 1.56 (d, J = 7.2 Hz, 3H).

Meanwhile, the resolution of its stereoisomers 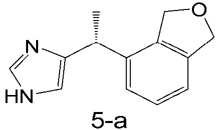 and 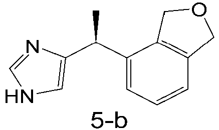 was performed.

### Example 6. Preparation of 4-(1-(2-Methyl-2,3-dihydro-1H-inden-4-yl)ethyl)-1H-imidazole

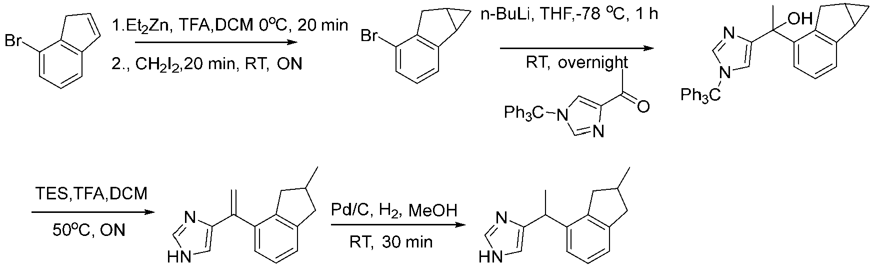

6.1 TFA (8.77 g, 76.900 mmol, 5 equiv) was added dropwise to a solution of diethylzinc (76.90 mL, 76.900 mmol, 5 equiv) in DCM (30 mL) at 0 °C under nitrogen atmosphere, and after the addition was completed, the mixture was stirred for 20 min, followed by addition of diiodomethane (20.60 g, 76.900 mmol, 5 equiv) at room temperature for 20 min. 4-Bromo-3H-indene (3 g, 15.380 mmol, 1 equiv) was added to the mixture described above at room temperature. The mixture was then stirred at room temperature overnight. The reaction was quenched with a saturated aqueous ammonium chloride solution (50 mL). The mixture was extracted with ethyl acetate (3 × 20 mL) at room temperature, and the organic layers were combined, washed with saturated brine (3 × 20 mL), and dried over anhydrous Na₂SO₄. After filtration, the filtrate was concentrated under reduced pressure. The residue was purified by silica gel column chromatography and eluted with PE/EA (5:1) to give 5-bromo-1H,1aH,6H,6aH-cyclopropa[a]indene (1.65 g). (ES, m/z): [M]= 208, 210.

6.2 n-Butyllithium (4.30 mL, 10.761 mmol, 1.5 equiv, 2.5 M in hexane) was added dropwise to a solution of 5-bromo-1H,1aH,6H,6aH-cyclopropa[a]indene (1.5 g, 7.174 mmol, 1 equiv) in tetrahydrofuran (20 mL) at -78 °C under nitrogen atmosphere. After the dropwise addition was completed, the mixture was stirred for 1 h, and then a solution of 1-[1-(trityl)imidazol-4-yl]ethanone (2.78 g, 7.891 mmol, 1.1 equiv) in THF (5 mL) was added. After the addition was completed, the mixture was slowly warmed to room temperature and stirred overnight. The reaction was quenched with a saturated aqueous ammonium chloride solution (50 mL) at 0 °C. The resulting mixture was extracted with EtOAc (3 × 20 mL). The organic phases were combined, washed with saturated brine (3 × 20 mL), and dried over anhydrous Na₂SO₄. After filtration, the filtrate was concentrated under reduced pressure. The residue was purified by silica gel column chromatography and eluted with PE/EA (5:1) to give 1-(1H,1aH,6H,6aH-cyclopropa[a]inden-5-yl)-1-[1-(trityl)imidazol-4-yl]ethanol (1.8 g). (ES, m/z): [M+1]=483.

The compound was prepared according to the method of Example 2, except that 1-(3,4-dihydro-2*H*-1-benzothiopyran-8-yl)-1-[1-(trityl)imidazol-4-yl]ethanol was replaced by 1-(1H,1aH,6H,6aH-cyclopropa[a]inden-5-yl)-1-[1-(trityl)imidazol-4-yl]ethanol (prepared in-house) to give 4-(1-(2-methyl-2,3-dihydro-1*H*-inden-4-yl)ethyl)-1*H-*imidazole, (ES,m/z):[M+1]= 227. ¹H NMR (400 MHz, Methanol-d4) δ 8.27 (dd, J = 2.6, 1.4 Hz, 1H), 7.16 - 6.89 (m, 3H), 6.77 (ddt, J = 11.2, 6.5, 3.0 Hz, 1H), 4.16 (q, J = 7.1 Hz, 1H), 3.04 - 2.78 (m, 2H), 2.53 - 2.04 (m, 3H), 1.49 (dd, J = 7.2, 4.1 Hz, 3H), 1.02 (d, J = 6.1 Hz, 3H).

### Example 7. Preparation of 4-(1-(1-Methyl-2,3-dihydro-1H-inden-4-yl)ethyl)-1H-imidazole

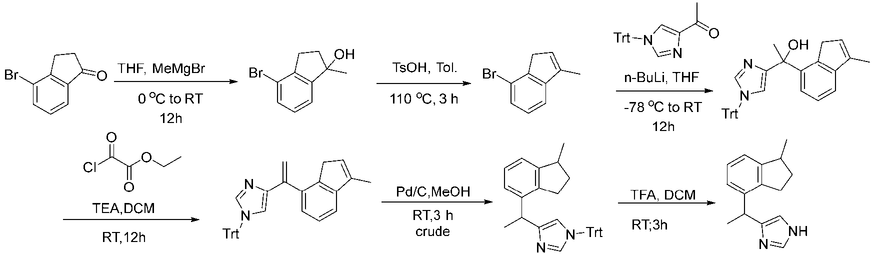

7.1 Methylmagnesium bromide (1.90 mL, 5.7 mmol, 1.2 equiv, 3 M in THF) was added dropwise to a stirred solution of 4-bromo-2,3-dihydroinden-1-one (1 g, 4.738 mmol, 1 equiv) in THF (25 mL) at 0 °C under nitrogen atmosphere. The resulting mixture was stirred at room temperature overnight under nitrogen atmosphere. The reaction was quenched by adding saturated ammonium chloride (10 mL). The resulting mixture was extracted with EtOAc (3 × 50 mL) and dried over anhydrous Na₂SO₄. After filtration, the filtrate was concentrated under reduced pressure. The residue was purified by silica gel column chromatography and eluted with PE/EA (4:1) to give 4-bromo-1-methyl-2,3-dihydroinden-1-ol (0.97 g) as a colorless oil. (ES,m/z): [M]=226, 228.

7.2 4-Bromo-1-methyl-2,3-dihydroinden-1-ol (400 mg, 1.761 mmol, 1 equiv), toluene (8 mL), and p-toluenesulfonic acid (30.33 mg, 0.176 mmol, 0.10 equiv) were added to a 20 mL sealed tube at room temperature. The resulting mixture was stirred at 110 °C for 3 h. The resulting mixture was cooled to room temperature and then concentrated under reduced pressure. The residue was purified by silica gel column chromatography and eluted with PE/EA (5:1) to give 4-bromo-1-methyl-3H-indene (300 mg) as a colorless oil. (ES,m/z): [M]= 208, 210.

7.3 n-Butyllithium (1.00 mL, 2.5 mmol, 1.5 equiv, 2.5 M in hexane) was added dropwise to a solution of 4-bromo-1-methyl-3H-indene (350 mg, 1.674 mmol, 1 equiv) in THF (10 mL) at -78 °C under nitrogen atmosphere. After the addition was completed, the mixture was stirred for 1 h, and 1-[1-(trityl)imidazol-4-yl]ethanone (707.9 mg, 2.009 mmol, 1.2 equiv) was then added. After the addition was completed, the mixture was warmed to room temperature and stirred overnight. The reaction was quenched with a saturated aqueous ammonium chloride solution (10 mL) at room temperature. The resulting mixture was extracted with ethyl acetate (3 × 100 mL), and the organic phases were combined, washed with brine (1 × 100 mL), and dried over anhydrous Na₂SO₄. After filtration, the filtrate was concentrated under reduced pressure. The residue was purified by silica gel column chromatography and eluted with PE/EA (1:1) to give 1-(1-methyl-3H-inden-4-yl)-1-[1-(trityl)imidazol-4-yl]ethanol (700 mg). (ES, m/z): [M+1]=483.

7.4 TEA (1.01 g, 9.944 mmol, 8 equiv) was added to a solution of 1-(1-methyl-3H-inden-4-yl)-1-[1-(trityl)imidazol-4-yl]ethanol (600 mg, 1.243 mmol, 1 equiv) in DCM (8 mL) at room temperature under nitrogen atmosphere, and the mixture was stirred for 2 min and cooled to 0 °C. Then ethyl oxalyl monochloride (339.5 mg, 2.486 mmol, 2 equiv) was added, and the resulting mixture was stirred at room temperature overnight and concentrated under reduced pressure. The residue was prepared by a preparative column to give 4-[1-(1-methyl-3H-inden-4-yl)vinyl]-1-(trityl)imidazole (120 mg). (ES, m/z): [M+1]=465.

7.5 4-[1-(1-Methyl-3H-inden-4-yl)vinyl]-1-(trityl)imidazole (60 mg, 0.129 mmol, 1 equiv), MeOH (2 mL), and Pd/C (30 mg, 10% w/t) were added to a 20 mL round-bottom flask at room temperature. The resulting mixture was stirred for 3 h under hydrogen atmosphere. The resulting mixture was filtered through a pad of celite, and the filter cake was washed with methanol (3 × 10 mL). The filtrate was concentrated under reduced pressure to give 4-[1-(1-methyl-2,3-dihydro-1H-inden-4-yl)ethyl]-1-(trityl)imidazole (20 mg, crude). (ES, m/z): [M+1]=469.

7.6 4-[1-(1-Methyl-2,3-dihydro-1H-inden-4-yl)ethyl]-1-(trityl)imidazole (60 mg, 0.128 mmol, 1 equiv) and DCM (3 mL)/TFA (1 mL) were added to a 20 mL round-bottom flask at room temperature. The resulting mixture was stirred at room temperature for 3 h. The resulting mixture was concentrated under reduced pressure. The residue was purified by reversed-phase flash chromatography to give 4-[1-(1-methyl-2,3-dihydro-1*H*-inden-4-yl)ethyl]-1*H*-imidazole (5.1 mg). (ES, m/z): [M+1]=227. ¹H NMR (400 MHz, Methanol-d4) δ 7.53 (s, 1H), 7.17 - 6.84 (m, 3H), 6.70 (s, 1H), 4.30 - 4.07 (m, 1H), 2.98 - 2.64 (m, 2H), 2.29 (s, 1H), 1.57 - 1.47 (m, 3H), 1.34 - 1.21 (m,5H).

### Example 8. Preparation of 4-[1-(1H-Inden-4-yl)ethyl]-1H-imidazole

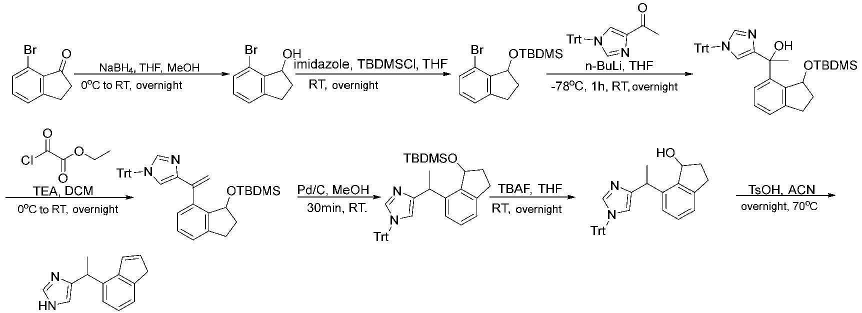

8.1 NaBH₄ (10.75 g, 284.280 mmol, 6 equiv) was added portionwise to a solution of 7-bromo-2,3-dihydroinden-1-one (11 g, 52.118 mmol, 1 equiv) in THF (100 mL) and methanol (50 mL) at 0 °C. The resulting mixture was stirred at room temperature overnight. The reaction was quenched with ice water (100 mL) at room temperature, and the resulting mixture was extracted with ethyl acetate (3 × 100 mL). The organic phases were combined, washed with brine (1 × 100 mL), and dried over anhydrous Na₂SO₄. After filtration, the filtrate was concentrated under reduced pressure. The residue was purified by silica gel column chromatography and eluted with PE/EA (1:1) to give 7-bromo-2,3-dihydro-1H-inden-1-ol (9.8 g) as a colorless liquid. (ES, m/z) = 212, 214.

8.2 Imidazole (4.10 g, 60.260 mmol, 1.2 equiv) and 7-bromo-2,3-dihydro-1H-inden-1-ol (10.7 g, 50.217 mmol, 1 equiv) were dissolved in THF (100 mL) at room temperature under nitrogen atmosphere, and then tert-butyldimethylsilyl chloride (15.14 g, 100.434 mmol, 2 equiv) was added portionwise at room temperature. The resulting mixture was stirred at room temperature overnight under nitrogen atmosphere. The reaction was quenched by adding water (100 mL) at room temperature. The resulting mixture was extracted with ethyl acetate (3 × 100 mL), and the organic phases were combined. The organic layer was washed with water (1 × 100 mL) and dried over anhydrous Na₂SO₄. After filtration, the filtrate was concentrated under reduced pressure. The residue was purified by silica gel column chromatography and eluted with PE/EA (10: 1) to give [(7-bromo-2,3-dihydro-1H-inden-1-yl)oxy](tert-butyl)dimethylsilane (15.2 g) as a light yellow oil.

8.3 n-Butyllithium (11.00 mL, 27.45 mmol, 1.5 equiv, 2.5 M in hexane) was added dropwise to a solution of [(7-bromo-2,3-dihydro-1H-inden-1-yl)oxy](tert-butyl)dimethylsilane (6 g, 18.330 mmol, 1 equiv) in THF (100 mL) at -78 °C under nitrogen atmosphere. After the addition was completed, the mixture was stirred for 1 h, and 1-[1-(trityl)imidazol-4-yl]ethanone (9.69 g, 27.495 mmol, 1.2 equiv) was then added. After the addition was completed, the mixture was warmed to room temperature and stirred overnight. The reaction was quenched with a saturated aqueous ammonium chloride solution (200 mL) at room temperature. The resulting mixture was extracted with ethyl acetate (3 × 150 mL), and the organic phases were combined, washed with brine (1 × 200 mL), and dried over anhydrous Na₂SO₄. After filtration, the filtrate was concentrated under reduced pressure. The residue was purified by silica gel column chromatography and eluted with PE/EA (1:1) to give 1-[3-[(tert-butyldimethylsilyl)oxy]-2,3-dihydro-1H-inden-4-yl]-1-(1-trityl-1H-imidazol-4-yl)ethanol (10.2g). (ES, m/z): [M+1]=601.

8.4 TEA (4.04 g, 39.940 mmol, 4 equiv) was added to a solution of 1-[3-[(tert-butyldimethylsilyl)oxy]-2,3-dihydro-1H-inden-4-yl]-1-(1-trityl-1H-imidazol-4-yl)ethanol (6 g, 9.985 mmol, 1 equiv) in DCM (70 mL) at room temperature under nitrogen atmosphere, and the mixture was stirred for 2 min and cooled to 0 °C. Then ethyl oxalyl monochloride (2.73 g, 19.970 mmol, 2 equiv) was added. The resulting mixture was stirred at room temperature overnight and quenched by adding water (100 mL). The resulting mixture was extracted with ethyl acetate (3 × 150 mL), and the organic phases were combined, washed with brine (1 × 100 mL), and dried over anhydrous Na₂SO₄. After filtration, the filtrate was concentrated under reduced pressure. The residue was purified by silica gel column chromatography and eluted with PE/EA (5:1) to give 4-(1-{3-[(tert-butyldimethylsilyl)oxy]-2,3-dihydro-1H-inden-4-yl}vinyl)-1-(trityl)imidazole (4.0 g) as a yellow solid, (ES, m/z): [M+1] = 583.

8.5 4-(1-{3-[(tert-Butyldimethylsilyl)oxy]-2,3-dihydro-1H-inden-4-yl1}vinyl)-1-(trityl)imidazole (4 g, 6.863 mmol, 1 equiv), MeOH (30 mL), and Pd/C (0.15 g, 10% w/t) were added to a 100 mL round-bottom flask at room temperature. The resulting mixture was stirred for 3 h under hydrogen atmosphere. The resulting mixture was filtered through a pad of celite, and the filter cake was washed with methanol (3 × 50 mL). The filtrate was concentrated under reduced pressure to give 4-(1-{3-[(tert-butyldimethylsilyl)oxy]-2,3-dihydro-1H-inden-4-yl}ethyl)-1-(trityl)imidazole (2.1 g). (ES, m/z): [M+1]= 585.

8.6 TBAF (39.46 mL, 39.460 mmol, 5 equiv) was added to a solution of 4-(1-{3-[(tert-butyldimethylsilyl)oxy]-2,3-dihydro-1H-inden-4-yl}ethyl)-1-(trityl)imidazole (4.6 g, 7.892 mmol, 1 equiv) in tetrahydrofuran (40 mL) at room temperature under nitrogen atmosphere. The resulting mixture was stirred at room temperature overnight under nitrogen atmosphere. The reaction was quenched by adding water (100 mL) at room temperature. The resulting mixture was extracted with ethyl acetate (3 × 80 mL), and the organic phases were combined. The organic layer was washed with water (1 × 100 mL) and dried over anhydrous Na₂SO₄. After filtration, the filtrate was concentrated under reduced pressure. The residue was purified by reversed-phase flash chromatography to give 7-{1-[1-(trityl)imidazol-4-yl]ethyl}-2,3-dihydro-1H-inden-1-ol (1.8 g) as a grayish-white solid. (ES, m/z): [M+1]=471.

8.7 TsOH (0.10 g, 0.595 mmol, 0.2 equiv) was added to a solution of 7-{1-[1-(trityl)imidazol-4-yl]ethyl}-2,3-dihydro-1H-inden-1-ol (1.4 g, 2.975 mmol, 1 equiv) in ACN (20 mL) at room temperature under nitrogen atmosphere. The resulting mixture was stirred at 70 °C overnight under nitrogen atmosphere. The resulting mixture was concentrated under reduced pressure. The residue was purified by reversed-phase flash chromatography to give 4-[1-(1H-inden-4-yl)ethyl]-1H-imidazole (389 mg). (ES, m/z):[M+1]=211. ¹H NMR (400 MHz, Methanol-d4) δ 8.35 - 7.87 (m, 1H), 7.35 (d, J = 7.3 Hz, 1H), 7.29 - 7.18 (m, 1H), 7.12 (t, J = 7.4 Hz, 1H), 6.98 (dt, J = 13.5, 7.5 Hz, 2H), 6.56 (ddd, J = 9.3, 5.8, 3.6 Hz, 1H), 4.63 - 4.27 (m, 1H), 3.39 (s, 2H), 1.64 (d, J = 6.3 Hz, 3H).

Meanwhile, the resolution of its stereoisomers 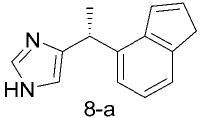 and 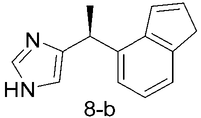 was performed.

### Example 9. Preparation of 4-[1-(1,3-Dihydrobenzo[c]thiophen-4-yl)ethyl]-1H-imidazole

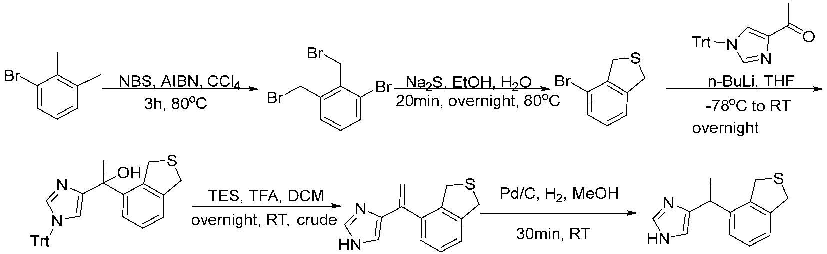

9.1 NBS (3.85 g, 21.614 mmol, 2 equiv) was added portionwise to a stirred solution of 3-bromo-1,2-xylene (2.0 g, 10.807 mmol, 1 equiv) and AIBN (0.41 g, 2.486 mmol, 0.23 equiv) in CCl₄ (30 mL) at room temperature under nitrogen atmosphere. The resulting mixture was stirred at 80 °C for 3 h under nitrogen atmosphere. The mixture was cooled to room temperature. The reaction was quenched with water/ice (100 mL) at 0 °C. The resulting mixture was extracted with dichloromethane (3 × 100 mL), and the organic layers were combined, washed with water (1 × 100 mL), dried over anhydrous Na₂SO₄, and filtered. The filtrate was concentrated under reduced pressure. The residue was purified by silica gel column chromatography and eluted with PE/EA (12:1) to give 1-bromo-2,3-bis(bromomethyl)benzene (1.7 g) as a colorless oil. (ES,m/z): [m/z]= 339.8, 341.8, 343.8, 345.8.

9.2 Sodium sulfide (182.0 mg, 2.333 mmol, 1 equiv) was dissolved in H₂O (2.4 mL), and the mixture was stirred at 80 °C for 20 min under nitrogen atmosphere, followed by dropwise addition of a solution of 1-bromo-2,3-bis(bromomethyl)benzene (800 mg, 2.333 mmol, 1 equiv) in ethanol (8.4 mL) at 80 °C. The resulting mixture was stirred at 80 °C overnight under nitrogen atmosphere. The mixture was cooled to room temperature. The reaction was quenched by adding water (30 mL) at room temperature. The resulting mixture was extracted with ethyl acetate (3 × 20 mL), and the organic layers were combined, washed with water (1 × 30 mL), and dried over anhydrous Na₂SO₄. After filtration, the filtrate was concentrated under reduced pressure. The residue was purified by silica gel column chromatography and eluted with PE/EA (12:1) to give 4-bromo-1,3-dihydro-2-benzo[c]thiophene (220 mg) as a colorless oil. (ES,m/z): [M]=214, 216.

The compound was prepared according to the method of Example 3, except that 2,5-dibromobicyclo[4.2.0]octa-1(6),2,4-triene was replaced by 4-bromo-1,3-dihydro-2-benzo[c]thiophene to give 4-[1-(1,3-dihydrobenzo[c]thiophen-4-yl)ethyl]-1H-imidazole. (ES,m/z): [M+1]=231. ¹H NMR (400 MHz, Chloroform-d) δ7.46 (s, 1H), 7.05 (dt, J = 15.8, 7.5 Hz, 2H), 6.87 (d, J = 7.3 Hz, 1H), 6.65 (s, 1H), 4.50 - 3.74 (m, 5H), 1.50 (d, J = 7.1 Hz, 3H).

### Example 10. Preparation of 4-[1-(2,3-Dihydrobenzofuran-7-yl)ethyl]-1H-imidazole

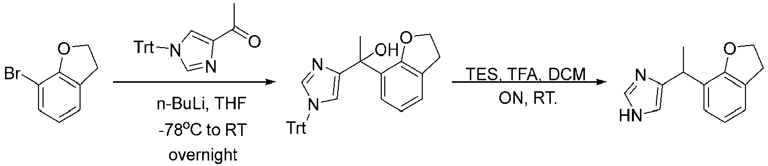

The compound was prepared according to the method of Example 1, except that 8-bromo-3,4-dihydro-2H-1-benzothiopyran was replaced by 7-bromo-2,3-dihydrobenzofuran, and 1-trityl-1H-imidazole-4-carbaldehyde was replaced by 1-[1-(trityl)imidazol-4-yl]ethanone to give 4-[1-(2,3-dihydrobenzofuran-7-yl)ethyl]-1H-imidazole. (ES,m/z): [M+1]=215. ¹H NMR (400 MHz, Methanol-d₄) δ 7.55 (d, J = 1.2 Hz, 1H), 7.03 (dd, J = 7.2, 1.4 Hz, 1H), 6.82 (d, J = 7.7 Hz, 1H), 6.78 - 6.69 (m, 2H), 4.52 (t, J = 8.7 Hz, 2H), 4.28 (q, J = 7.2 Hz, 1H), 3.17 (t, J = 8.7 Hz, 2H), 1.54 (d, J = 7.1 Hz, 3H).

Meanwhile, the resolution of its stereoisomers 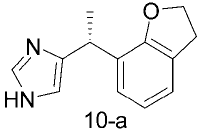 and 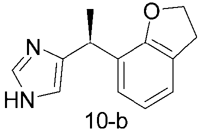 was performed.

### Example 11. 4-[1-(6-Fluoro-1H-inden-4-yl)ethyl]-1H-imidazole

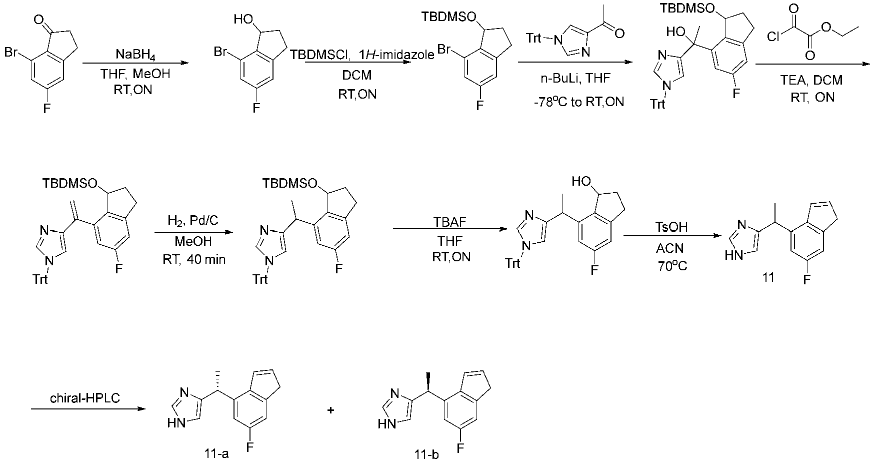

11.1 NaBH₄ (3.30 g, 87.316 mmol) was added to a mixed solution of 7-bromo-5-fluoro-2,3-dihydroinden-1-one (5 g, 21.829 mmol) dissolved in THF (40 mL) and MeOH (20 mL) at 0 °C under nitrogen atmosphere for treatment. The resulting mixture was stirred at room temperature overnight under nitrogen atmosphere. The reaction was quenched by adding water (50 mL) at room temperature. The resulting mixture was extracted with CH₂Cl₂ (3 × 50 mL). The combined organic layer was washed with water (1 × 50 mL) and dried over anhydrous Na₂SO₄. After filtration, the filtrate was concentrated under reduced pressure. The residue was purified by silica gel column chromatography and eluted with PE/EA (5:1) to give 7-bromo-5-fluoro-2,3-dihydro-1H-inden-1-ol (4.6 g) as a yellow oil. (ES, m/z): [M-18+1]=213, 215.

11.2 7-Bromo-5-fluoro-2,3-dihydro-1*H*-inden-1-ol (5 g, 21.639 mmol) and imidazole (2.95 g, 43.278 mmol) were added to a DCM (50 mL) solution at room temperature under nitrogen atmosphere, and the mixture was stirred. tert-Butyldimethylsilyl chloride (3.91 g, 25.967 mmol) was then added portionwise. The resulting mixture was stirred at room temperature overnight under nitrogen atmosphere and then concentrated under reduced pressure. The residue was purified by silica gel column chromatography and eluted with PE/EA (12:1) to give [(7-bromo-5-fluoro-2,3-dihydro-1*H*-inden-1-yl)oxy](tert-butyl)dimethylsilane (7 g) as a colorless oil.

11.3 [(7-Bromo-5-fluoro-2,3-dihydro-1*H*-inden-1-yl)oxy](tert-butyl)dimethylsilane (5.4 g, 15.637 mmol) was added to a THF (60 mL) solution under nitrogen atmosphere, and n-BuLi (9.38 mL, 23.456 mmol, 2.5 M in n-hexane) was added at -78 °C. After 40 min of the reaction, a solution of 1-[1-(trityl)imidazol-4-yl]ethanone (5.51 g, 15.637 mmol) in THF (20 mL) was added dropwise at -78 °C. The resulting mixture was stirred at room temperature overnight under nitrogen atmosphere. The reaction was quenched by adding a saturated aqueous NH₄Cl solution (200 mL) at room temperature. The resulting mixture was extracted with CH₂Cl₂ (3 × 200 mL). The combined organic layer was washed with water (1 × 200 mL) and dried over anhydrous Na₂SO₄. After filtration, the filtrate was concentrated under reduced pressure. The residue was purified by silica gel column chromatography and eluted with PE/EA (1:1) to give 1-[3-[(tert-butyldimethylsilyl)oxy]-6-fluoro-2,3-dihydro-1*H*-inden-4-yl]-1-[1-(trityl)imidazol-4-yl]ethanol (5.2 g) as a yellow oil. (ES, m/z): [M+1]=619.

11.4 1-[3-[(tert-Butyldimethylsilyl)oxy]-6-fluoro-2,3-dihydro-1*H*-inden-4-yl]-1-[1-(trityl)imidazol-4-yl]ethanol (5 g, 8.079 mmol) was added to a DCM (50 mL) solution at room temperature under nitrogen atmosphere. Triethylamine (6.54 g, 64.632 mmol) was added, and the mixture was reacted for 1 min. Ethyl oxalyl monochloride (4.41 g, 32.316 mmol) was then added dropwise at 0 °C. The resulting mixture was stirred at room temperature overnight under nitrogen atmosphere and then concentrated under reduced pressure. The residue was purified by silica gel column chromatography and eluted with PE/EA (5:1) to give 4-[1-[3-[(tert-butyldimethylsilyl)oxy]-6-fluoro-2,3-dihydro-1H-inden-4-yl]vinyl]-1-(trityl)imidazole (3.8 g) as a yellow oil. (ES, m/z): [M+1]=601.

11.5 10% Pd/C (0.04 g) and 4-[1-[3-[(tert-butyldimethylsilyl)oxy]-6-fluoro-2,3-dihydro-1*H*-inden-4-yl]vinyl]-1-(trityl)imidazole (1 g, 1.664 mmol) were added to a MeOH (10 mL) solution at room temperature. The reaction solution was stirred at room temperature for 40 min under hydrogen atmosphere. The resulting reaction solution was filtered through celite, and the filter cake was washed with MeOH (3 × 10 mL). The filtrate was concentrated under reduced pressure. The residue was purified by silica gel column chromatography and eluted with PE/EA (5:1) to give 4-[1-[3-[(tert-butyldimethylsilyl)oxy]-6-fluoro-2,3-dihydro-1*H*-inden-4-yl]ethyl]-1-(trityl)imidazole (900 mg) as a yellow oil. (ES, m/z): [M+1]=603.

11.6 Tetrabutylammonium fluoride (18.41 mL, 18.411 mmol, 1 M in THF) was added to a solution of 4-[1-[3-[(tert-butyldimethylsilyl)oxy]-6-fluoro-2,3-dihydro-1*H*-inden-4-yl]ethyl]-1-(trityl)imidazole (3.7 g, 6.137 mmol) in THF (60 mL) at room temperature. The resulting mixture was stirred at room temperature overnight under nitrogen atmosphere. The reaction was quenched by adding water (100 mL) at room temperature. The resulting mixture was extracted with EtOAc (3 × 100 mL). The combined organic layer was washed with water (1 × 100 mL) and dried over anhydrous Na₂SO₄. After filtration, the filtrate was concentrated under reduced pressure. The residue was purified by silica gel column chromatography and eluted with PE/EA (5:1) to give 5-fluoro-7-[1-[1-(trityl)imidazol-4-yl]ethyl]-2,3-dihydro-1*H*-inden-1-ol (2.1 g) as a colorless oil. (ES, m/z): [M+1]=489.

11.7 p-Toluenesulfonic acid (888.1 mg, 5.158 mmol) was added to a solution of 5-fluoro-7-[1-[1-(trityl)imidazol-4-yl]ethyl]-2,3-dihydro-1*H*-inden-1-ol (4.2 g, 8.596 mmol) in acetonitrile (80 mL) at room temperature. The resulting mixture was stirred at 70 °C for 3 h under nitrogen atmosphere. The mixture was cooled to room temperature. The resulting mixture was concentrated under reduced pressure. The residue was purified by reversed-phase flash chromatography under the following conditions: column: C18 silica gel; mobile phase: MeCN-water (0.1% NH₃.H₂O + 10 mmol/L NH₄HCO₃), with a gradient of 10% to 50% within 10 min; detector: UV 220 nm to give 4-[1-(6-fluoro-1*H*-inden-4-yl)ethyl]-1*H*-imidazole (1.9 g). (ES, m/z): [M+1]=229. ¹H NMR (400 MHz, Methanol-*d*₄) δ 7.57 (d, *J =* 1.2 Hz, 1H), 7.05 (dd, *J* = 8.6, 2.3 Hz, 1H), 6.97 (dt, *J =* 4.5, 2.0 Hz, 1H), 6.80 (d, *J =* 1.1 Hz, 1H), 6.75 (dd, *J* = 10.9, 2.4 Hz, 1H), 6.52 (dt, *J* = 5.8, 2.1 Hz, 1H), 4.44 (q, *J* = 7.2 Hz, 1H), 3.38 (d, *J* = 2.2 Hz, 2H), 1.60 (d, *J =* 7.2 Hz, 3H).

11.8 4-[1-(6-Fluoro-1*H*-inden-4-yl)ethyl]-1*H*-imidazole (500 mg) was purified by chiral HPLC under the following conditions (Column: (S, S)-Whelk-O1, 4.6 × 50 mm, 3 µm; Gradient: isocratic % B) to give 4-[(1R)-1-(6-fluoro-1H-inden-4-yl)ethyl]-1*H-*imidazole (11-a, 271 mg) (retention time: 3.29 min) and 4-[(1S)-1-(6-fluoro-1H-inden-4-yl)ethyl]-1*H*-imidazole (11-b, 274 mg) (retention time: 4.22 min).

4-[(1R)-1-(6-Fluoro-1H-inden-4-yl)ethyl]-1*H*-imidazole (11-a) (ES, m/z): [M+1]=229, ¹H NMR (400 MHz, Methanol-*d*₄) δ 7.57 (d, *J =* 1.4 Hz, 1H), 7.06 (dd, *J =* 8.6, 2.5 Hz, 1H), 7.00 - 6.93 (m, 1H), 6.81 (s, 1H), 6.76 (d, *J* = 2.4 Hz, 1H), 6.52 (dt, *J =* 6.2, 2.2 Hz, 1H), 4.44 (q, *J* = 7.2 Hz, 1H), 3.38 (d, *J* = 2.5 Hz, 2H), 1.60 (d, *J* = 7.2 Hz, 3H).

4-[(1S)-1-(6-Fluoro-1*H*-inden-4-yl)ethyl]-1*H*-imidazole (11-b) (ES, m/z): [M+1]=229, ¹H NMR (400 MHz, Methanol-*d*₄) δ7.74 - 7.50 (m, 1H), 7.09 - 7.03 (m, 1H), 7.00 - 6.93 (m, 1H), 6.81 (d, *J =* 1.1 Hz, 1H), 6.75 (dd, *J =* 10.9, 2.4 Hz, 1H), 6.52 (ddt, *J =* 5.6, 4.0, 2.0 Hz, 1H), 4.44 (q, *J =* 7.2 Hz, 1H), 3.38 (q, *J =* 2.2 Hz, 2H), 1.60 (dd, *J* = 7.2, 1.3 Hz, 3H).

### Example 12. Preparation of 4-[1-(6-Fluoro-2,3-dihydro-1H-inden-4-yl)ethyl]-1H-imidazole

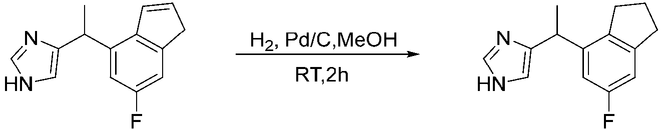

12.1 4-[1-(6-Fluoro-1*H*-inden-4-yl)ethyl]-1*H*-imidazole (50 mg, 0.219 mmol) and 10% Pd/C (10 mg) were added to MeOH (1 mL) at room temperature under hydrogen atmosphere, and the mixture was stirred for 1 h under hydrogen atmosphere. The resulting mixture was filtered, and the filter cake was washed with MeOH (3 × 10 mL). The filtrate was concentrated under reduced pressure. The residue was purified by reversed-phase flash chromatography under the following conditions: column: C18 silica gel; mobile phase: MeCN-water (0.1% NH₃.H₂O + 10 mmol/L NH₄HCO₃), with a gradient of 10% to 30% within 10 min; detector: UV 254 nm to give 4-[1-(6-fluoro-2,3-dihydro-1H-inden-4-yl)ethyl]-1H-imidazole (34.1 mg). (ES, m/z): [M+1]=231. ¹H NMR (400 MHz, Methanol-*d*₄) δ 7.57 (s, 1H), 6.80 - 6.72 (m, 2H), 6.60 (d, *J =* 10.4 Hz, 1H), 4.17 (q, *J =* 7.2 Hz, 1H), 2.92 - 2.81 (m, 3H), 2.81 (s, 1H), 2.12 - 1.99 (m, 2H), 1.53 (dd, *J* = 7.1*,* 2.1 Hz, 3H).

### Example 13. Preparation of 4-[1-(1-Benzofuran-4-yl)ethyl]-1H-imidazole

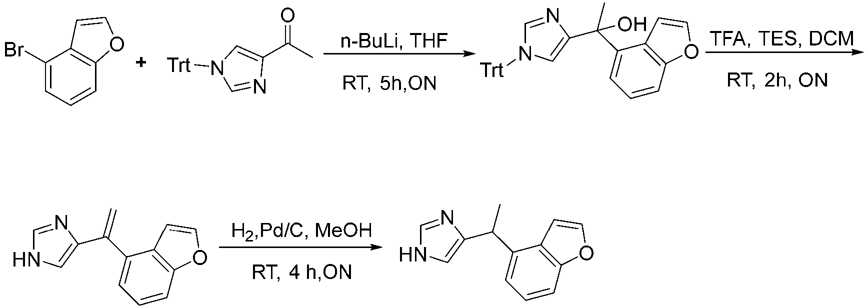

13.1 n-BuLi (0.46 mL, 1.141 mmol, 2.5 M in n-hexane) was added to a solution of 4-bromo-1-benzofuran (150 mg, 0.761 mmol, 1 equiv) in THF (4 mL) under nitrogen atmosphere. The mixture was reacted at -78 °C for 45 min, and then a solution of 1-[1-(trityl)imidazol-4-yl]ethanone (322.0 mg, 0.913 mmol, 1.2 equiv) in tetrahydrofuran (6 mL) was added dropwise at -78 °C. The resulting mixture was stirred at room temperature for another 5 h. The reaction was quenched by adding saturated NH₄Cl (aqueous solution) (10 mL) at room temperature. The resulting mixture was extracted with EtOAc (2 × 30 mL). The combined organic layer was washed with water (3 × 30 mL) and dried over anhydrous Na₂SO₄. After filtration, the filtrate was concentrated under reduced pressure. The residue was purified by reversed-phase flash chromatography under the following conditions: column: C18 silica gel; mobile phase: MeCN-water (10 mmol/L NH₄HCO₃), with a gradient of 30% to 70% within 10 min; detector: UV 220 nm to give 1-(1-benzofuran-4-yl)-1-[1-(trityl)imidazol-4-yl]ethanol (340 mg) as a pale yellow oil. (ES,m/z): [M+1]=471.

13.2 Triethylsilane (840.1 mg, 7.230 mmol) was added to a solution of 1-(1-benzofuran-4-yl)-1-[1-(trityl)imidazol-4-yl]ethanol (340 mg, 0.723 mmol) in DCM (8 mL) at room temperature under nitrogen atmosphere. After 1 min of the reaction, trifluoroacetic acid (823.8 mg, 7.230 mmol) was added dropwise at room temperature. The resulting mixture was stirred at room temperature for another 2 h. The resulting mixture was concentrated under reduced pressure. The residue was purified by reversed-phase flash chromatography under the following conditions: column: C18 silica gel; mobile phase: acetonitrile in water (10 mmol/L NH₄HCO₃), with a gradient of 10% to 50% within 10 min; detector: UV 220 nm to give 4-[1-(1-benzofuran-4-yl)vinyl]-1H-imidazole (120 mg) as a yellow oil. (ES,m/z): [M+1]=211.

13.3 10% Pd/C (10 mg) was added to a solution of 4-[1-(1-benzofuran-4-yl)vinyl]-1*H-*imidazole (50 mg, 0.238 mmol) in MeOH (2 mL) in a 25 mL round-bottom flask at room temperature under nitrogen atmosphere. The mixture was hydrogenated at room temperature for 2 h under hydrogen atmosphere using a hydrogen balloon and filtered through celite, and the filtrate was concentrated under reduced pressure. The residue was purified by reversed-phase flash chromatography under the following conditions: column: C18 silica gel; mobile phase: MeCN-water (10 mmol/L NH₄HCO₃), with a gradient of 10% to 30% within 10 min; detector: ultraviolet 220 nm to give 4-[1-(1-benzofuran-4-yl)ethyl]-1*H*-imidazole (13.6 mg). (ES,m/z):[M+1]=213. ¹H NMR (400 MHz, Methanol-*d*₄) δ 7.66 (d, *J =* 2.1 Hz, 1H), 7.56 (s, 1H), 7.34 (d, *J =* 8.2 Hz, 1H), 7.28 - 7.15 (m, 1H), 7.04 (d, *J =* 7.4 Hz, 1H), 6.85 - 6.70 (m, 2H), 4.45 (q*, J =* 7.1 Hz, 1H), 1.68 (dd, *J =* 7.3*,* 1.8 Hz, 3H).

### Example 14. Preparation of 4-[1-(2H-Chromen-8-yl)ethyl]-1H-imidazole

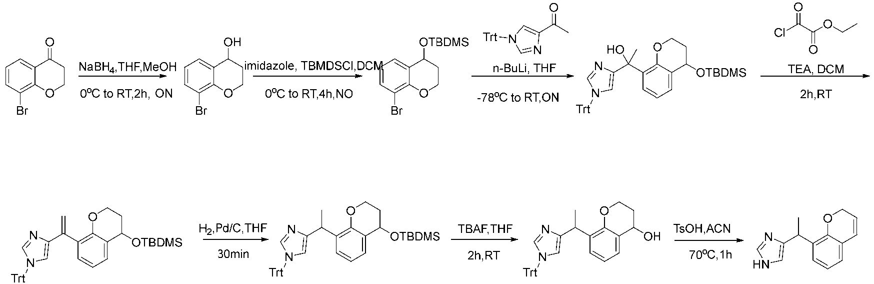

14.1 NaBH₄ (666.4 mg, 17.616 mmol) was added portionwise to a stirred mixed solution of 8-bromo-2,3-dihydro-1-benzopyran-4-one (2 g, 8.808 mmol) in MeOH (14 mL) and THF (7 mL) at 0 °C under nitrogen atmosphere. The resulting mixture was stirred at room temperature for 2 h under nitrogen atmosphere. The reaction was quenched by adding a saturated aqueous NH₄Cl solution (20 mL) at 0 °C. The resulting mixture was extracted with EtOAc (3 × 100 mL). The combined organic layer was washed with water (3 × 100 mL) and dried over anhydrous Na₂SO₄. After filtration, the filtrate was concentrated under reduced pressure. The residue was purified by silica gel column chromatography and eluted with PE/EA (5:1) to give 8-bromo-3,4-dihydro-2*H-*1-benzopyran-4-ol (1.8 g) as a yellow oil. (ES, m/z): [M-18]=211, 213.

14.2 tert-Butyldimethylsilyl chloride (1.7 g, 11.132 mmol) was added to a stirred mixture of 8-bromo-3,4-dihydro-2*H*-1-benzopyran-4-ol (1.7 g, 7.421 mmol) and imidazole (0.6 g, 8.905 mmol) in DMF (40 mL) at 0 °C under nitrogen atmosphere. The resulting mixture was stirred at room temperature for 4 h under nitrogen atmosphere. The resulting mixture was quenched with water (20 mL) and extracted with EtOAc (3 × 50 mL). The combined organic layer was washed with water (3 × 10 mL) and dried over anhydrous Na₂SO₄. After filtration, the filtrate was concentrated under reduced pressure. The residue was purified by silica gel column chromatography and eluted with PE/EA (7:1) to give [(8-bromo-3,4-dihydro-2*H*-1-benzopyran-4-yl)oxy](tert-butyl)dimethylsilane (2.2 g) as a yellow oil.

14.3 A solution of [(8-bromo-3,4-dihydro-2*H*-1-benzopyran-4-yl)oxy](tert-butyl)dimethylsilane (2.1 g, 6.116 mmol) in THF (40 mL) was treated with n-BuLi (3.6 mL, 9.174 mmol, 2.5 M in n-hexane) at -78 °C for 40 min under nitrogen atmosphere, and then a solution of 1-[1-(trityl)imidazol-4-yl]ethanone (2.4 g, 6.728 mmol) in tetrahydrofuran (10 mL) was added dropwise at -78 °C. The resulting mixture was stirred at room temperature overnight under nitrogen atmosphere. The reaction was quenched with saturated NH₄Cl (aqueous solution) at room temperature. The resulting mixture was extracted with CH₂Cl₂ (3 × 100 mL). The combined organic layer was washed with water (1 × 100 mL) and dried over anhydrous Na₂SO₄. After filtration, the filtrate was concentrated under reduced pressure. The residue was purified by reversed-phase flash chromatography under the following conditions: column: C18 silica gel; mobile phase: MeCN-water (10 mmol/L NH₄HCO₃), with a gradient of 10% to 70% within 10 min; detector: UV 254 nm to give 1-(4-[(tert-butyldimethylsilyl)oxy]-3,4-dihydro-2*H*-1-benzopyran-8-yl)-1-[1-(trityl)imidazol-4-yl]ethanol (1.2 g) as a yellow oil. (ES,m/z): [M+1]=617.

14.4 Ethyl oxalyl monochloride (730.3 mg, 5.349 mmol) was added dropwise to a stirred mixture of 1-(4-[(tert-butyldimethylsilyl)oxy]-3,4-dihydro-2*H*-1-benzopyran-8-yl)-1-[1-(trityl)imidazol-4-yl]ethanol (1.1 g, 1.783 mmol) and triethylamine (1.4 mL, 10.698 mmol) in DCM (22 mL) at room temperature under nitrogen atmosphere. The resulting mixture was stirred at room temperature for 2 h under nitrogen atmosphere. The resulting mixture was concentrated under reduced pressure. The residue was purified by silica gel column chromatography and eluted with PE/EA (12:1) to give 4-[1-[4-[(tert-butyldimethylsilyl)oxy]-3,4-dihydro-2*H*-1-benzopyran-8-yl]-vinyl]-1-(trityl)imidazole (750 mg) as a yellow oil. (ES,m/z): [M+1]=599.

14.5 10% Pd/C (177.7 mg) was added to a stirred solution of 4-[1-[4-[(tert-butyldimethylsilyl)oxy]-3,4-dihydro-2*H*-1-benzopyran-8-yl]-vinyl]-1-(trityl)imidazole (500 mg, 0.835 mmol) in THF (10 mL) at room temperature. The resulting mixture was stirred for 30 min at room temperature under hydrogen atmosphere. The resulting mixture was filtered, and the filter cake was washed with THF (3 × 10 mL). The filtrate was concentrated under reduced pressure. The residue was purified by reversed-phase flash chromatography under the following conditions: column: C18 silica gel; mobile phase: MeCN-water (0.1% NH₃.H₂O), with a gradient of 10% to 50% within 10 min; detector: UV 254 nm to give 4-[1-[4-[(tert-butyldimethylsilyl)oxy]-3,4-dihydro-2*H*-1-benzopyran-8-yl]-ethyl]-1-(trityl)imidazole (420 mg) as a yellow oil. (ES, m/z): [M+1] = 601.

14.6 Tetrabutylammonium fluoride (2.0 mL, 1.998 mmol, 1 M in THF) was added dropwise to a stirred mixture of 4-[1-[4-[(tert-butyldimethylsilyl)oxy]-3,4-dihydro-2*H-*1-benzopyran-8-yl]-ethyl]-1-(trityl)imidazole (400 mg, 0.666 mmol) in THF (8 mL) at room temperature under nitrogen atmosphere. The resulting mixture was stirred at room temperature for 2 h under nitrogen atmosphere. The reaction was quenched by adding a saturated NH₄Cl (aqueous solution) (100 mL) at room temperature. The resulting mixture was extracted with EtOAc (3 × 200 mL). The combined organic layer was washed with water (3 × 100 mL) and dried over anhydrous Na₂SO₄. After filtration, the filtrate was concentrated under reduced pressure. The residue was purified by reversed-phase flash chromatography under the following conditions: column: C18 silica gel; mobile phase: acetonitrile in water (0.1% NH₃.H₂O), with a gradient of 10% to 50% within 10 min; detector: ultraviolet 254 nm to give 8-[1-[1-(trityl)imidazol-4-yl]ethyl]-3,4-dihydro-2*H*-1-benzopyran-4-ol (180 mg) as a yellow oil. (ES, m/z): [M+1] = 487. 14.7 p-Toluenesulfonic acid (113.2 mg, 0.656 mmol) was added portionwise to a stirred mixture of 8-[1-[1-(trityl)imidazol-4-yl]ethyl]-3,4-dihydro-2H-1-benzopyran-4-ol (80 mg, 0.164 mmol) in acetonitrile (2 mL) at room temperature under nitrogen atmosphere. The resulting mixture was stirred at 70 °C for 1 h under nitrogen atmosphere. The resulting mixture was concentrated under vacuum. The crude product (50 mg) was purified by prep-HPLC (column: XBridge Prep OBD C18 column 30 × 150 mm, 5 µm; mobile phase A: water (10 mmol/L NH₄HCO₃ + 0.1% NH₃.H₂O), mobile phase B: methanol; flow rate: 60 mL/min; gradient: 20% B to 38% B within 10 min; wavelength: 254 nm/220 nm; RT1 (min): 11.82/15.28) to give 4-[1-(2H-chroman-8-yl)ethyl]-1*H-*imidazole (14.3 mg). (ES, m/z): [M+1]=227. ¹H NMR (400 MHz, Methanol-*d*₄) δ 7.54 (d, *J* = 1.2 Hz, 1H), 6.87 - 6.79 (m, 2H), 6.79 - 6.71 (m, 2H), 6.44 (dq, *J* = 9.8, 1.7 Hz, 1H), 5.82 (dtd, *J* = 9.5, 3.6, 1.8 Hz, 1H), 4.77 (dt, *J* = 3.5, 1.6 Hz, 2H), 4.44 (q, *J* = 7.1 Hz, 1H), 1.51 (d, *J =* 7.2 Hz, 3H).

### Example 15. Preparation of 4-[1-(2,2-Difluoro-1,3-dihydroinden-4-yl)ethyl]-1H-imidazole

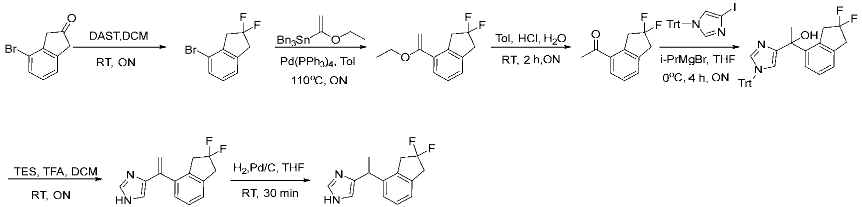

15.1 Diethylaminosulfur trifluoride (421.8 mg, 11.372 mmol) was added to a solution of 4-bromo-1,3-dihydroinden-2-one (600 mg, 2.843 mmol) in DCM (5 mL) at room temperature. The resulting mixture was stirred at room temperature overnight under nitrogen atmosphere. The reaction was quenched by adding ice water (10 mL) at 0 °C. The resulting mixture was extracted with EtOAc (3 × 10 mL). The combined organic layer was washed with water (1 × 10 mL) and dried over anhydrous Na₂SO₄. After filtration, the filtrate was concentrated under reduced pressure. The residue was purified by reversed-phase flash chromatography under the following conditions: column: C18 silica gel; mobile phase: MeCN-water (0.1% TFA), with a gradient of 10% to 50% within 10 min; detector: UV 220 nm to give 4-bromo-2,2-difluoro-1,3-dihydroindene (400 mg) as a pale brown oil.

15.2 Tribenzyl(1-ethoxyvinyl)stannane (834.8 mg, 1.802 mmol) was added to a solution of 4-bromo-2,2-difluoro-1,3-dihydroindene (350 mg, 1.502 mmol) in toluene (4 mL) at room temperature under nitrogen atmosphere. After 1 min of the reaction, Pd(PPh₃)₄ (173.5 mg, 0.150 mmol) was added portionwise at room temperature. The resulting mixture was stirred at 110 °C overnight under nitrogen atmosphere. After the reaction was completed, the mixture was cooled to room temperature. The resulting mixture was concentrated under reduced pressure to give 4-(1-ethoxyvinyl)-2,2-difluoro-1,3-dihydroindene (340 mg, crude product) as a brown oil. (ES,m/z): [M+1]=225.

15.3 H₂O (1 mL) was added to a solution of 4-(1-ethoxyvinyl)-2,2-difluoro-1,3-dihydroindene (350 mg, 1.561 mmol) in toluene (5 mL) at room temperature under nitrogen atmosphere. After 1 min of the reaction, a 4 M hydrogen chloride solution (5 mL) was added dropwise at room temperature. The resulting mixture was stirred at room temperature for 2 h under nitrogen atmosphere. The reaction was quenched by adding water (20 mL) at room temperature. The resulting mixture was extracted with EtOAc (3 × 20 mL). The combined organic layer was washed with water (1 × 20 mL) and dried over anhydrous Na₂SO₄. After filtration, the filtrate was concentrated under reduced pressure. The residue was purified by silica gel column chromatography and eluted with PE/EA (5:1) to give 1-(2,2-difluoro-1,3-dihydroinden-4-yl)ethanone (280 mg) as a pale yellow oil. (ES,m/z): [M+1]=197.

15.4 i-PrMgBr (1.83 mL, 4.584 mmol, 1 M in THF) was added to a solution of 4-iodo-1-(trityl)imidazole (1 g, 2.292 mmol) in THF (10 mL) at 0 °C under nitrogen atmosphere. After 30 min of the reaction, a solution of 1-(2,2-difluoro-1,3-dihydroinden-4-yl)ethanone (0.30 g, 1.528 mmol) in THF (2 mL) was added portionwise at 0 °C. The resulting mixture was stirred at room temperature for 4 h under nitrogen atmosphere. The reaction was quenched by adding saturated NH₄Cl (aqueous solution) (10 mL) at room temperature. The resulting mixture was extracted with EtOAc (3 × 10 mL). The combined organic layer was washed with water (1 × 10 mL) and dried over anhydrous Na₂SO₄. After filtration, the filtrate was concentrated under reduced pressure. The residue was purified by silica gel column chromatography and eluted with PE/EA (1:1) to give 1-(2,2-difluoro-1,3-dihydroinden-4-yl)-1-[1-(trityl)imidazol-4-yl]ethanol (260 mg) as a yellow oil. (ES, m/z): [M+1]=507.

15.5 A solution of 1-(2,2-difluoro-1,3-dihydroinden-4-yl)-1-[1-(trityl)imidazol-4-yl]ethanol (230 mg, 0.454 mmol) in DCM (2 mL) was added to trifluoroacetic acid (1.03 g, 9.080 mmol) at room temperature under nitrogen atmosphere. After 1 min of the reaction, triethylsilane (1.05 g, 9.080 mmol) was added dropwise at room temperature. The resulting mixture was stirred at room temperature overnight under nitrogen atmosphere. The resulting mixture was concentrated under reduced pressure. The residue was purified by reversed-phase flash chromatography under the following conditions: column: C18 silica gel; mobile phase: MeCN-water (10 mmol/L NH₄HCO₃), with a gradient of 10% to 50% within 10 min; detector: UV 220 nm to give 4-[1-(2,2-difluoro-1,3-dihydroinden-4-yl)vinyl]-1*H*-imidazole (30 mg) as a colorless oil. (ES, m/z): [M+1]=247.

15.6 Pd/C (3.9 mg, 10% w/t) was added to a solution of 4-[1-(2,2-difluoro-1,3-dihydroinden-4-yl)vinyl]-1*H*-imidazole (30 mg, 0.122 mmol) in tetrahydrofuran (3 mL) at room temperature. The resulting mixture was stirred for 30 min at room temperature under hydrogen atmosphere. The resulting mixture was filtered through celite, and the filter cake was washed with THF (3 × 10 mL). The filtrate was concentrated under reduced pressure. The residue was purified by reversed-phase flash chromatography under the following conditions: column: C18 silica gel; mobile phase: MeCN-water (0.1% NH₃.H₂O), with a gradient of 10% to 50% within 10 min; detector: UV 220 nm to give 4-[1-(2,2-difluoro-1,3-dihydroinden-4-yl)ethyl]-1*H*-imidazole (12 mg). (ES, m/z): [M+1]=249. ¹H NMR (400 MHz, Methanol-*d*₄) δ 7.56 (d, *J =* 1.2 Hz, 1H), 7.18 (t, *J =* 7.6 Hz, 1H), 7.07 (dd, *J =* 12.7, 7.6 Hz, 2H), 6.76 (d, *J =* 1.3 Hz, 1H), 4.11 (q, *J* = 7.2 Hz, 1H), 3.43 - 3.31 (m, 4H), 1.56 (d, *J* = 7.2 Hz, 3H).

### Example 16. Preparation of 4-[1-(2-Fluoro-1-benzofuran-7-yl)ethyl]-1H-imidazole

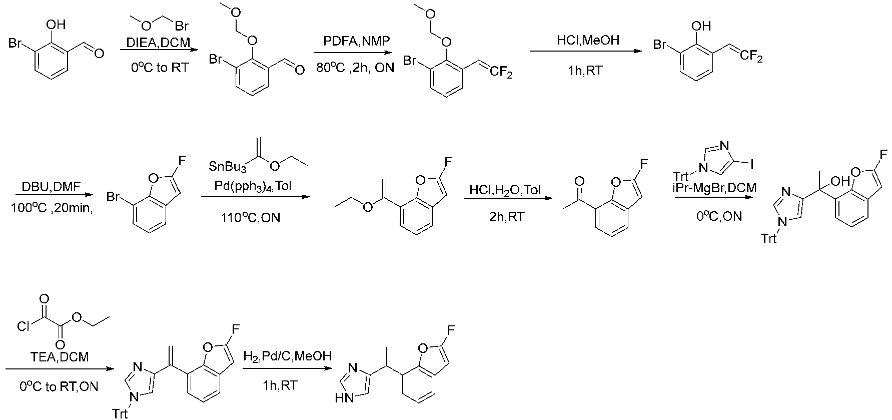

16.1 Bromomethyl methyl ether (3.7 g, 29.847 mmol) was added dropwise to a stirred solution of 3-bromo-2-hydroxylbenzaldehyde (2 g, 9.949 mmol) and DIEA (3.9 g, 29.847 mmol) in DCM at 0 °C. The resulting mixture was stirred at room temperature overnight. The resulting mixture was quenched with H₂O (20 mL) and extracted with DCM (3 × 100 mL). The combined organic layer was washed with H₂O (1 × 100 mL) and dried over anhydrous Na₂SO₄. After filtration, the filtrate was concentrated under reduced pressure. The residue was purified by silica gel column chromatography and eluted with PE/EA (5:1) to give 3-bromo-2-(methoxymethoxy)benzaldehyde (2.3 g) as a yellow oil. (ES,m/z): [M+1]=245, 247.

16.2 3-Bromo-2-(methoxymethoxy)benzaldehyde (1.5 g, 6.121 mmol) and ethyl 2,2-difluoro-2-(triphenylphosphonio)acetate (4.3 g, 12.242 mmol) were added to NMP (30 mL), and the resulting mixture was stirred at 80 °C for 2 h under N₂ atmosphere. The resulting mixture was diluted with water (20 mL) and extracted with ethyl acetate (3 × 100 mL). The combined organic layer was washed with water (1 × 100 mL) and dried over anhydrous Na₂SO₄. After filtration, the filtrate was concentrated under reduced pressure. The residue was purified by silica gel column chromatography and eluted with PE/EA (3:1) to give 1-bromo-3-(2,2-difluorovinyl)-2-(methoxymethoxy)benzene (1.3 g) as a colorless oil.

16.3 1-Bromo-3-(2,2-difluorovinyl)-2-(methoxymethoxy)benzene (2 g, 7.166 mmol) and HCl (261.2 g, 7.166 mmol) were added to MeOH (40 mL), and the resulting mixture was stirred at room temperature for 1 h. The resulting mixture was diluted with water (10 mL) and concentrated under vacuum. The residue was purified by reversed-phase chromatography under the following conditions: chromatographic column: C18 silica gel; mobile phase: acetonitrile-water, with a gradient of 10%-60% within 10 min; detector: ultraviolet 254 nm to give 2-bromo-6-(2,2-difluorovinyl)phenol (900 mg) as a colorless oil. (ES,m/z): [M+1]=234, 236.

16.4 2-Bromo-6-(2,2-difluorovinyl)phenol (700 mg, 2.98 mmol) and DBU (544.1 mg, 3.574 mmol) were added to DMF (6 mL), and the resulting mixture was stirred at 100 °C overnight. The mixture was irradiated with microwaves at 100 °C for 20 min. The resulting mixture was cooled to room temperature, then diluted with water (50 mL), and extracted with ethyl acetate (3 × 100 mL). The combined organic layer was washed with water (3 × 100 mL) and dried over anhydrous Na₂SO₄. After filtration, the filtrate was concentrated under reduced pressure. The residue was purified by reversed-phase chromatography under the following conditions: chromatographic column: C18 silica gel; mobile phase: acetonitrile-water, with a gradient of 10%-50% within 10 min; detector: ultraviolet 254 nm to give 7-bromo-2-fluoro-1-benzofuran (170 mg) as a yellow oil.

16.5 7-Bromo-2-fluoro-1-benzofuran (117 mg, 0.544 mmol, 1 equiv), tributyl(1-ethoxyvinyl)tin (235.8 mg, 0.653 mmol, 1.2 equiv), and Pd(PPh₃)₄ (62.88 mg, 0.054 mmol, 0.1 equiv) were added to toluene (3 mL). The resulting mixture was stirred at 110 °C overnight under N₂ atmosphere. The resulting mixture was cooled to room temperature. The resulting mixture was directly used in the next step without further purification. (ES,m/z): [M+1]= 207.

16.6 7-(1-Ethoxyvinyl)-2-fluoro-1-benzofuran (120 mg, 0.582 mmol) was added to a mixture of hydrochloric acid (0.2 mL)/toluene (2.4 mL), and the mixture was stirred at room temperature for 2 h. The resulting mixture was extracted with EtOAc (3 × 50 mL). The combined organic layer was washed with H₂O (0.2 mL) (3 × 50 mL) and dried over anhydrous Na₂SO₄. After filtration, the filtrate was concentrated under reduced pressure.

The residue was purified by silica gel column chromatography and eluted with PE/EA (5:1) to give 1-(2-fluoro-1-benzofuran-7-yl)ethanone (88 mg) as a yellow oil. (ES,m/z): [M+1]= 179.

16.7 4-Iodo-1-(trityl)imidazole (281.6 mg, 0.645 mmol) and bromo(isopropyl)magnesium (0.645 mL, 0.645 mmol, 1 M in THF) were added to DCM (0.8 mL), and the resulting mixture was stirred at 0 °C for 20 min under N₂ atmosphere. Then 1-(2-fluoro-1-benzofuran-7-yl)ethanone (115 mg, 0.645 mmol) was added dropwise. The resulting mixture was stirred at 0 °C overnight under nitrogen atmosphere. The resulting mixture was extracted with DCM (3 × 50 mL). The combined organic layer was washed with brine (1 × 50 mL) and dried over anhydrous Na₂SO₄. After filtration, the filtrate was concentrated under reduced pressure. The residue was purified by reversed-phase chromatography under the following conditions: chromatographic column: C18 silica gel; mobile phase: acetonitrile-water (10 mmol/L NH₄HCO₃), with a gradient of 10%-50% within 10 min; detector: ultraviolet 254 nm to give 1-(2-fluoro-1-benzofuran-7-yl)-1-[1-(trityl)imidazol-4-yl]ethanol (64 mg) as a yellow oil. (ES,m/z): [M+1]= 489.

16.8 1-(2-Fluoro-1-benzofuran-7-yl)-1-[1-(trityl)imidazol-4-yl]ethanol (63 mg, 0.129 mmol, 1 equiv) and triethanolamine (78.2 mg, 0.774 mmol, 6 equiv) were added to DCM (2 mL), and then ethyl acetoacetate (52.8 mg, 0.387 mmol, 3 equiv) was added dropwise at 0 °C. The resulting mixture was stirred at room temperature overnight under N₂ atmosphere. The resulting mixture was concentrated under vacuum. The residue was purified by silica gel column chromatography (PE/EA = 8:1) to give 4-[1-(2-fluoro-1-benzofuran-7-yl)vinyl]-1-(trityl)imidazole (35 mg) as a colorless oil. (ES,m/z): [M+1]= 471.

16.9 4-[1-(2-Fluoro-1-benzofuran-7-yl)vinyl]-1-(trityl)imidazole (40 mg, 0.175 mmol) and Pd/C (3.7 mg, 10% w/t) were added to MeOH (4 mL), and the resulting mixture was stirred at room temperature for 1 h under hydrogen atmosphere. The resulting mixture was filtered, and the filter cake was washed with MeOH (3 × 5 mL). The filtrate was concentrated under reduced pressure. The crude product (40 mg) was purified by preparative high-performance liquid chromatography under the following conditions (chromatographic column: Column: XBridge Prep OBD C18 chromatographic column 30 × 150 mm, 5 µm; mobile phase A: water (10 mmol/L NH₄HCO₃ + 0.1% NH₃.H₂O), mobile phase B: MeOH; flow rate: 60 mL/min; gradient: 40% B to 58% B within 10 min; wavelength: 254 nm/220 nm; RT1 (min): 12.33) to give 4-[1-(2-fluoro-1-benzofuran-7-yl)ethyl]-1H-imidazole (5 mg). (ES,m/z): [M+1]= 231. ¹H NMR (400 MHz, Methanol-*d*₄) δ 7.58 (s, 1H), 7.35 (dd, *J =* 7.7, 1.2 Hz, 1H), 7.16 (t, *J =* 7.7 Hz, 1H), 7.03 (dd, *J =* 7.7, 1.3 Hz, 1H), 6.85 (s, 1H), 6.00 (d, *J =* 6.4 Hz, 1H), 4.57 (q, *J* = 7.2 Hz, 1H), 1.68 (d, *J =* 7.3 Hz, 3H).

### Example 17. Preparation of 4-[1-(1,1-Dimethyl-1H-inden-4-yl)ethyl]-1H-imidazole

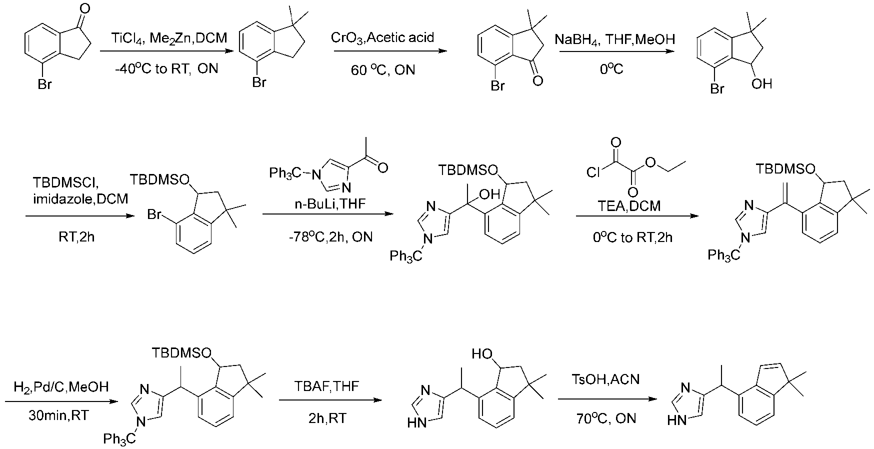

17.1 TiCl₄ (29.9 mL, 29.849 mmol) and dimethylzinc (42.6 mL, 42.642 mmol, 1 M in hexane) were added to DCM (10 mL). The resulting mixture was stirred at -40 °C for 20 min under N₂ atmosphere, and then a solution of 4-bromo-1-indenone (3 g, 14.214 mmol) in DCM (10 mL) was added dropwise. The resulting mixture was stirred at room temperature overnight under nitrogen atmosphere. The resulting mixture was quenched with a saturated aqueous NH₄Cl solution and extracted with EtOAc (3 × 200 mL). The combined organic layer was washed with brine (2 × 100 mL) and dried over anhydrous Na₂SO₄. After filtration, the filtrate was concentrated under reduced pressure. The residue was purified by silica gel column chromatography and eluted with PE to give 4-bromo-1,1-dimethyl-2,3-dihydro-1H-indene (2.4 g) as a colorless oil.

17.2 4-Bromo-1,1-dimethyl-2,3-dihydro-1H-indene (2.3 g, 10.216 mmol) and chromium trioxide (6.1 g, 61.296 mmol) were added to acetic acid (10 mL), and the resulting mixture was stirred at 60 °C for 3 h under N₂ atmosphere. The resulting mixture was cooled to room temperature and then concentrated under reduced pressure. The residue was purified by reversed-phase chromatography under the following conditions: chromatographic column: C18 silica gel; mobile phase: MeCN-water (0.1% FA), with a gradient of 10%-50% within 20 min; detector: ultraviolet 254 nm to give 7-bromo-3,3-dimethyl-2H-inden-1-one (716 mg) as a brown solid. (ES,m/z): [M+1]= 239, 241.

17.3 7-Bromo-3,3-dimethyl-2H-inden-1-one (716 mg, 2.994 mmol) and NaBH₄ (679.6 mg, 17.964 mmol) were added to MeOH (3 mL)/THF (6 mL), and the resulting mixture was stirred at 0 °C for 2 h and then quenched with water. After concentration under reduced pressure, the residue was purified by reversed-phase chromatography under the following conditions: chromatographic column: C18 silica gel; mobile phase: MeCN-water (0.1% FA), with a gradient of 10% to 50% within 10 min; detector: ultraviolet 254 nm to give 7-bromo-3,3-dimethyl-1,2-dihydroinden-1-ol (210 mg) as a yellow oil. (ES,m/z): [M+1]= 223, 225.

17.4 7-Bromo-3,3-dimethyl-1,2-dihydroinden-1-ol (110 mg, 0.456 mmol, 1 equiv) and imidazole (62.1 mg, 0.912 mmol, 2 equiv) were added to DCM (2 mL) under nitrogen atmosphere, and the resulting mixture was stirred at room temperature, followed by the addition of tert-butyldimethylsilyl chloride (82.5 mg, 0.547 mmol, 1.2 equiv). The resulting mixture was stirred at room temperature for 2 h, then quenched with water, and extracted with EtOAc (3 × 50 mL). The combined organic layer was washed with brine (1 × 50 mL) and dried over anhydrous Na₂SO₄. After filtration, the filtrate was concentrated under reduced pressure. The residue was purified by silica gel column chromatography and eluted with PE/EA (5:1) to give [(7-bromo-3,3-dimethyl-1,2-dihydroinden-1-yl)oxy](tert-butyl)dimethylsilane (150 mg) as a yellow oil.

17.5 n-BuLi (0.4 mL, 0.802 mmol, 2.5 M in hexane) was added dropwise to a solution of [(7-bromo-3,3-dimethyl-1,2-dihydroinden-1-yl)oxy](tert-butyl)dimethylsilane (190 mg, 0.535 mmol) in THF (4 mL) under nitrogen atmosphere. The mixture was stirred at -78 °C for 2 h, and then a solution of 1-[1-(trityl)imidazol-4-yl]ethanone (188.4 mg, 0.535 mmol) in THF (4 mL) was added dropwise. The resulting mixture was stirred at room temperature overnight. The resulting mixture was quenched with a saturated aqueous NH₄Cl solution and extracted with DCM (3 × 100 mL). The combined organic layer was washed with water (1 × 100 mL) and dried over anhydrous Na₂SO₄. After filtration, the filtrate was concentrated under reduced pressure. The residue was purified by reversed-phase chromatography under the following conditions: chromatographic column: C18 silica gel; mobile phase: MeCN-water (10 mmol/L NH₄HCO₃), with a gradient of 10% to 70% within 10 min; detector: ultraviolet 254 nm to give 1-(3-((tert-butyldimethylsilyloxy)-1,1-dimethyl-2,3-dihydro-1H-inden-4-yl)-1-(1-trityl-1H-imidazol-4-yl)ethanol (80 mg) as a yellow oil. (ES,m/z): [M+1]= 629.

17.6 Ethyl chloroformate (104.5 mg, 0.765 mmol) was added dropwise to a solution of (3Z)-3-amino-2-(3-[(tert-butyldimethylsilyl)oxy]-1,1-dimethyl-2,3-dihydroinden-4-yl-4-[(trityl)amino]but-3-en-2-ol (158 mg, 0.255 mmol) and triethylamine (154.9 mg, 1.530 mmol) in DCM (1.5 mL) at 0 °C. The resulting mixture was stirred at room temperature for 2 h and then concentrated under reduced pressure. The residue was purified by reversed-phase chromatography under the following conditions: chromatographic column: C18 silica gel; mobile phase: MeCN-water (10 mmol/L NH₄HCO₃), with a gradient of 10% to 55% within 10 min; detector: ultraviolet 254 nm to give 4-[1-[3-[(tert-butyldimethylsilyl)oxy]-1,1-dimethyl-2,3-dihydroinden-4-yl]vinyl]-1-(trityl)imidazole (80 mg) as a colorless oil. (ES,m/z): [M+1]= 611.5.

17.7 4-[1-[3-[(tert-Butyldimethylsilyl)oxy]-1,1-dimethyl-2,3-dihydroinden-4-yl]vinyl]-1-(trityl)imidazole (80 mg, 0.131 mmol) and Pd/C (2.79 mg, 10% w/t) were added to MeOH (2 mL). The resulting mixture was stirred at room temperature for 30 min under hydrogen atmosphere. The resulting mixture was filtered through celite, and the filter cake was washed with MeOH (3 × 10 mL). The filtrate was concentrated under reduced pressure to give 4-[1-[3-[(tert-butyldimethylsilyl)oxy]-1,1-dimethyl-2,3-dihydroinden-4-yl]ethyl]-1-(trityl)imidazole. The crude product was directly used in the next step without further purification. (ES,m/z): [M+1]= 613.

17.8 4-[1-[3-[(tert-Butyldimethylsilyl)oxy]-1,1-dimethyl-2,3-dihydroinden-4-yl]ethyl]-1-(trityl)imidazole (45 mg, 0.073 mmol) and TBAF (57.6 mg, 0.219 mmol) were added to THF (1 mL). The resulting mixture was stirred at room temperature for 2 h. The mixture was quenched with water at room temperature and concentrated under reduced pressure, and the residue was purified by reversed-phase chromatography under the following conditions: chromatographic column: C18 silica gel; mobile phase: MeCN-water (10 mmol/L NH₄HCO₃), with a gradient of 10% to 40% within 10 min; detector: ultraviolet 254 nm. As a result, 7-[1-(1H-imidazol-4-yl)ethyl]-3,3-dimethyl-1,2-dihydroinden-1-ol (18 mg) was obtained as a colorless oil. (ES,m/z): [M+1]= 257. 17.9 7-[1-(1H-Imidazol-4-yl)ethyl]-3,3-dimethyl-1,2-dihydroinden-1-ol (18 mg, 0.070 mmol) and p-toluenesulfonic acid (2.4 mg, 0.014 mmol) were added to MeCN (0.4 mL), and the resulting mixture was stirred at 70 °C for 2 h under nitrogen atmosphere. The mixture is cooled to room temperature, quenched with water at room temperature, and concentrated under reduced pressure, and the residue was purified by reversed-phase chromatography under the following conditions: chromatographic column: C18 silica gel; mobile phase: MeCN-water (10 mmol/L NH₄HCO₃), with a gradient of 10% to 50% within 10 min; detector: ultraviolet 254 nm to give 4-[1-(1,1-dimethyl-1H-inden-4-yl)ethyl]-1H-imidazole (3.4 mg). (ES,m/z): [M+1]= 239. ¹H NMR (400 MHz, Methanol-*d*₄) δ 7.63 (s, 1H), 7.18 (d, *J =* 7.4 Hz, 1H), 7.10 (t, *J =* 7.5 Hz, 1H), 6.96 (d, *J =* 7.6 Hz, 1H), 6.80 (s, 1H), 6.75 (d, *J =* 5.6 Hz, 1H), 6.36 (d, *J =* 5.6 Hz, 1H), 4.40 (q, *J =* 7.2 Hz, 1H), 1.61 (d, *J =* 7.2 Hz, 3H), 1.28 (d, *J =* 4.1 Hz, 6H).

### Example 18. Preparation of 7-[1-(1H-Imidazol-4-yl)ethyl]-2,3-dihydro-1H-indole

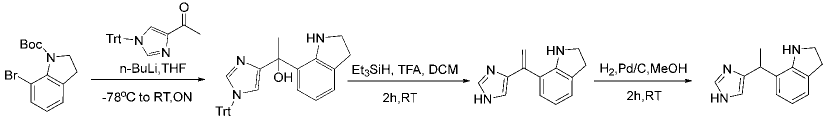

18.1 n-BuLi (0.2 mL, 0.503 mmol, 2.5 M in hexane) was added dropwise to a solution of tert-butyl 7-bromo-2,3-dehydroindole-1-carboxylate (100 mg, 0.335 mmol) in THF (1 mL) under nitrogen atmosphere. The mixture was stirred at -78 °C for 1 h, followed by the dropwise addition of 1-[1-(trityl)imidazol-4-yl]ethanone (118.2 mg, 0.335 mmol). The resulting mixture was stirred at room temperature overnight. The resulting mixture was extracted with EtOAc (3 × 100 mL). The combined organic layer was washed with brine (1 × 100 mL) and dried over anhydrous Na₂SO₄. After filtration, the filtrate was concentrated under reduced pressure. The residue was purified by reversed-phase chromatography under the following conditions: chromatographic column: C18 silica gel; mobile phase: MeCN-water (10 mmol/L NH₄HCO₃), with a gradient of 10% to 60% within 10 min; detector: ultraviolet 254 nm to give 1-(indolin-7-yl)-1-(1-trityl-1H-imidazol-4-yl)ethan-1-ol (60 mg) as a yellow oil. (ES,m/z): [M+1]=454.

18.2 1-(Indolin-7-yl)-1-(1-trityl-1H-imidazol-4-yl)ethan-1-ol (400 mg, 0.700 mmol), TFA (800.1 mg, 6.999 mmol), and Et₃SiH (813.54 mg, 7.000 mmol) were added to DCM (8 mL). The resulting mixture was stirred at room temperature for 2 h, concentrated under reduced pressure, and the residue was purified by reversed-phase chromatography under the following conditions: chromatographic column: C18 silica gel; mobile phase: MeCN-water (0.1% FA), with a gradient of 10% to 50% within 10 min; detector: ultraviolet 254 nm to give 7-[1-(1H-imidazol-4-yl)vinyl]-2,3-dihydro-1H-indole (80 mg) as a yellow oil. (ES,m/z): [M+1]=212.

18.3 7-[1-(1H-Imidazol-4-yl)vinyl]-2,3-dihydro-1H-indole (75 mg, 0.355 mmol) and Pd/C (7.56 mg, 0.071 mmol, 10% w/t) were added to MeOH (1.5 mL), and the resulting mixture was stirred at room temperature for 2 h under hydrogen atmosphere. The resulting mixture was filtered through celite, and the filter cake was washed with MeOH (3 × 10 mL). The filtrate was concentrated under reduced pressure. The crude product (60 mg) was purified by preparative high-performance liquid chromatography under the following conditions (chromatographic column: XBridge Prep OBD C18 Column 30 × 150 mm, 5 µm; mobile phase A: water (10 mmol/L NH₄HCO₃ + 0.1% NH₃.H₂O), mobile phase B: MEOH; flow rate: 60 mL/min; gradient: 40% B to 58% B within 10 min; wavelength: 254 nm/220 nm; RT1 (min): 12.33) to give 7-[1-(1H-imidazol-4-yl)ethyl]-2,3-dihydro-1H-indole (15.9 mg). (ES,m/z):[M+1]=214. ¹H NMR (400 MHz, Methanol-*d*₄) δ 7.55 (d, *J* = 1.2 Hz, 1H), 6.96 (dt, *J* = 7.3, 1.2 Hz, 1H), 6.82 (d, *J =* 7.7 Hz, 1H), 6.76 (t, *J =* 1.1 Hz, 1H), 6.66 (t, *J =* 7.5 Hz, 1H), 4.08 (q, *J =* 7.2 Hz, 1H), 3.44 (t, *J =* 8.2 Hz, 2H), 2.98 (t, *J =* 8.3 Hz, 2H), 1.56 (d, *J =* 7.2 Hz, 3H).

### Example 19. Preparation of 4-[1-(1H-Imidazol-4-yl)ethyl]-2,3-dihydro-1H-indole

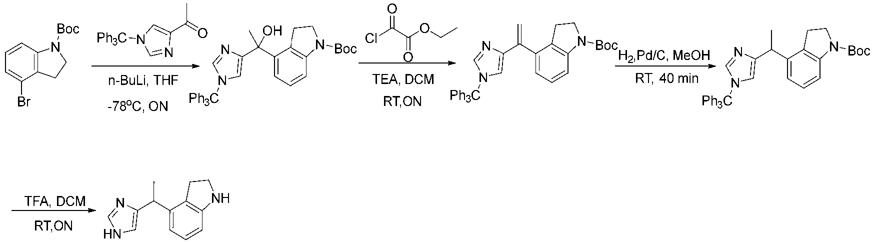

19.1 n-BuLi (221.1 mg, 3.452 mmol, 2.5 M in n-hexane) was added dropwise to a solution of tert-butyl 4-bromo-2,3-dihydroindole-1-carboxylate (500 mg, 2.301 mmol) in THF (1 mL) under nitrogen atmosphere. The mixture was stirred at -78 °C for 40 min, and then 1-[1-(trityl)imidazol-4-yl]ethanone (811.0 mg, 2.301 mmol) was added dropwise. The resulting mixture was stirred at room temperature overnight. The reaction was quenched by adding a saturated aqueous NH₄Cl solution (20 mL) at room temperature. The resulting mixture was extracted with EtOAc (3 × 20 mL). The combined organic layer was washed with water (1 × 20 mL) and dried over anhydrous Na₂SO₄. After filtration, the filtrate was concentrated under reduced pressure. The residue was purified by silica gel column chromatography and eluted with PE/EA (1:1) to give tert-butyl 4-(1-hydroxy-1-(1-trityl-1H-imidazol-4-yl)ethyl)indoline-1-carboxylate (500 mg) as a brown oil. (ES,m/z): [M+1]=572.

19.2 Ethyl oxalyl monochloride (286.6 mg, 2.100 mmol) was added dropwise to a solution of tert-butyl 4-(1-hydroxy-1-(1-trityl-1H-imidazol-4-yl)ethyl)indoline-1-carboxylate (400 mg, 0.700 mmol) in DCM (10 mL) under nitrogen atmosphere. The mixture was stirred at room temperature for 1 min. Then triethylamine (424.8 mg, 4.200 mmol) was added dropwise at 0 °C. The resulting mixture was stirred at room temperature overnight and concentrated under reduced pressure, and the residue was purified by silica gel column chromatography and eluted with PE/EA (5:1) to give tert-butyl 4-(1-(1-trityl-1H-imidazol-4-yl)vinyl)indoline-1-carboxylate (320 mg) as a yellow oil. (ES,m/z): [M+1]=554.

19.3 tert-Butyl 4-(1-(1-trityl-1H-imidazol-4-yl)vinyl)indoline-1-carboxylate (200 mg, 0.361 mmol) and 10% Pd/C (11.53 mg) were added to MeOH (5 mL). The resulting mixture was stirred at room temperature for 40 min under hydrogen atmosphere. The resulting mixture was filtered through celite, and the filter cake was washed with MeOH (3 × 10 mL). The filtrate was concentrated under reduced pressure to give tert-butyl 4-[1-[1-(trityl)imidazol-4-yl]ethyl]-2,3-dihydroindole-1-carboxylate (150 mg, crude product) as a yellow solid. (ES,m/z): [M+1]=556.

19.4 Tert-butyl 4-[1-[1-(trityl)imidazol-4-yl]ethyl]-2,3-dihydroindole-1-carboxylate (100 mg, 0.180 mmol) was dissolved in TFA (1 mL) and DCM (4 mL). The resulting mixture was stirred at room temperature overnight under nitrogen atmosphere. The resulting mixture was concentrated under reduced pressure. The residue was purified by reversed-phase chromatography under the following conditions: chromatographic column: C18 silica gel; mobile phase: MeCN-water (10 mmol/L NH₄HCO₃), with a gradient of 10% to 50% within 10 min; detector: ultraviolet 220 nm to give 4-[1-(1H-imidazol-4-yl)ethyl]-2,3-dihydro-1H-indole (15.2 mg). (ES,m/z): [M+1]=214. ¹H NMR (400 MHz, Methanol-*d*₄) δ 7.53 (s, 1H), 6.93 (t, *J =* 7.7 Hz, 1H), 6.72 (s, 1H), 6.54 (t, *J =* 7.1 Hz, 2H), 4.10 (q, *J =* 7.2 Hz, 1H), 3.42 (t, *J =* 8.3 Hz, 2H), 2.90 (ddq, *J* = 23.8, 15.8, 8.3 Hz, 2H), 1.54 (d, *J=* 7.2 Hz, 3H).

### Example 20. Preparation of N-(2,3-Dihydro-1H-inden-4-yl)-1H-imidazol-4-amine

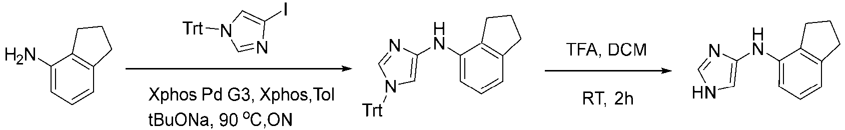

20.1 4-Iodo-1-(trityl)imidazole (200 mg, 0.458 mmol), 2,3-dihydro-1H-inden-4-amine (73.27 mg, 0.550 mmol), X-phos (21.85 mg, 0.046 mmol), sodium tert-butoxide (88.11 mg, 0.916 mmol), and Xphos Pd G3 (38.80 mg, 0.046 mmol) were added to toluene (5 mL) under nitrogen atmosphere. The resulting mixture was stirred at 90 °C overnight. The resulting mixture was cooled to room temperature and concentrated under reduced pressure. The residue was purified by reversed-phase chromatography under the following conditions: chromatographic column: C18 silica gel; mobile phase: MeCN-water (0.1% FA), with a gradient of 40%-100% within 10 min; detector: ultraviolet 254 nm to give N-(2,3-dihydro-1H-inden-4-yl)-1-(trityl)imidazol-4-amine (70 mg) as a yellow solid. (ES,m/z): [M+1]=442. 20.2 Trifluoroacetic acid (6 mL) was added dropwise to a solution of N-(2,3-dihydro-1H-inden-4-yl)-1-(trityl)imidazol-4-amine (70 mg, 0.159 mmol) in DCM (2 mL). The resulting mixture was stirred at room temperature for 2 h. The resulting mixture was concentrated under reduced pressure, and the crude product was purified by preparative high-performance liquid chromatography under the following conditions (chromatographic column: XBridge Prep Phenyl OBD Column 19 × 250 mm, 5 µm; mobile phase A: water (0.05% TFA), mobile phase B: MeOH; flow rate: 60 mL/min; gradient: 29% B to 44% B within 10 min; wavelength: 254 nm/220 nm; RT1 (min): 4.7) to give N-(2,3-dihydro-1H-inden-4-yl)-1H-imidazol-4-amine (7.4 mg). (ES,m/z): [M+1]=200. ¹H NMR (400 MHz, Methanol-*d*₄) δ 8.25 (s, 1H), 7.79 (s, 1H), 6.96 (t, *J =* 7.7 Hz, 1H), 6.70 (d, *J =* 7.3 Hz, 2H), 2.89 (t, *J =* 7.5 Hz, 2H), 2.80 (t, *J =* 7.3 Hz, 2H), 2.09 (p, *J =* 7.5 Hz, 2H).

### Example 21. Preparation of 7-(1-(1H-Imidazol-4-yl)ethyl)-1-methylindoline

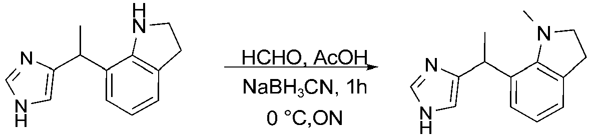

21.1 Formaldehyde (0.86 mL, 23.440 mmol) and AcOH (14.08 mg, 0.234 mmol) were added to a solution of 7-[1-(1H-imidazol-4-yl)ethyl]-2,3-dihydro-1H-indole (500 mg, 2.344 mmol) in MeOH (10 mL), and the mixture was stirred at 0 °C for 30 min under nitrogen atmosphere. Then NaBH₃CN (486.14 mg, 7.735 mmol) was added, and the mixture was stirred for another 1 h. The resulting mixture was concentrated under reduced pressure and purified by reversed-phase flash chromatography under the following conditions: column: C18 silica gel; mobile phase: water-methanol (0.1% NH₃.H₂O), with a gradient of 10% to 50% within 10 min; detector: UV 254 nm to give 7-(1-(1H-imidazol-4-yl)ethyl)-1-methylindoline (57 mg). (ES,m/z): [M+1] =228. ¹H NMR (400 MHz, Methanol-*d*₄) δ 7.56 (d, *J =* 1.2 Hz, 1H), 6.97 (dq, *J =* 7.3, 1.2 Hz, 1H), 6.89 (d, *J* = 7.7 Hz, 1H), 6.76 (t, *J =* 7.5 Hz, 1H), 6.66 (t, *J =* 1.1 Hz, 1H), 4.47 (q, *J =* 7.1 Hz, 1H), 3.42 - 3.33 (m, 1H), 3.29 - 3.20 (m, 1H), 3.02 - 2.87 (m, 2H), 2.84 (s, 3H), 1.56 (d, *J* = 7.1 Hz, 3H).

### Example 22. Preparation of 4-[1-(6-Chloro-1H-inden-4-yl)ethyl]-1H-imidazole

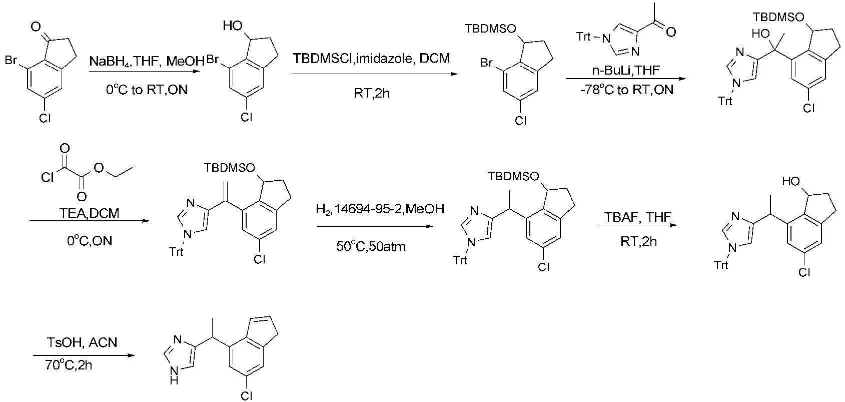

22.1 NaBH₄ (462.3 mg, 12.222 mmol) was added to a stirred solution of 7-bromo-5-chloro-2,3-dihydroinden-1-one (500 mg, 2.037 mmol) in anhydrous THF (10 mL)/MeOH (5 mL) at 0 °C. The reaction mixture was stirred at room temperature overnight. After the reaction was completed, the reaction was quenched by adding 5 mL of water. The aqueous layer was extracted with ethyl acetate (100 mL). The combined organic phase was washed with brine (100 mL), dried over anhydrous sodium sulfate, and concentrated under reduced pressure. The residue was purified by silica gel column chromatography and eluted with PE/EA (5:1) to give 7-bromo-5-chloro-2,3-dihydro-1H-inden-1-ol (400 mg) as a colorless oil. (ES,m/z): [M-18]=231.

22.2 7-Bromo-5-chloro-2,3-dihydro-1H-inden-1-ol (450 mg, 1.818 mmol), tert-butyldimethylsilyl chloride (328.8 mg, 2.182 mmol), and imidazole (247.5 mg, 3.636 mmol) were dissolved in DCM (9 mL), and the mixture was stirred at room temperature for 2 h. After the reaction was completed, the resulting mixture was quenched with water and extracted with EtOAc (3 × 100 mL). The organic layers were combined, washed with brine (1 × 100 mL), dried over anhydrous Na₂SO₄, and filtered, and the filtrate was concentrated under reduced pressure. The residue was purified by silica gel column chromatography and eluted with PE to give [(7-bromo-5-chloro-2,3-dihydro-1H-inden-1-yl)oxy](tert-butyl)dimethylsilane (500 mg) as a white solid.

22.3 n-BuLi (0.73 mL, 1.838 mmol, 2.5 M in n-hexane) was added to a solution of [(7-bromo-5-chloro-2,3-dihydro-1H-inden-1-yl)oxy](tert-butyl)dimethylsilane (443 mg, 1.225 mmol) in THF (10 mL) at -78 °C under nitrogen atmosphere, and the mixture was stirred for 1 h. Then 1-[1-(trityl)imidazol-4-yl]ethanone (431.56 mg, 1.225 mmol) was added portionwise. The resulting mixture was stirred at room temperature overnight under nitrogen atmosphere. The reaction was quenched with saturated NH₄Cl (aqueous solution) at room temperature, and the reaction solution was extracted with EtOAc (3 × 100 mL), washed with brine (1 × 100 mL), dried over anhydrous Na₂SO₄, filtered, and concentrated under reduced pressure. The resulting product was purified by reversed-phase flash chromatography (conditions were as follows: column: C18 silica gel; mobile phase: MeCN-water (10 mmol/L NH₄HCO₃), with a gradient of 10% to 50% within 10 min; detector: UV 254 nm) to give 1-[3-[(tert-butyldimethylsilyl)oxy]-6-chloro-2,3-dihydro-1H-inden-4-yl]-1-[1-(trityl)imidazol-4-yl]ethanol (350 mg) as a yellow oil. (ES,m/z): [M+1]=635.

22.4 1-[3-[(tert-Butyldimethylsilyl)oxy]-6-chloro-2,3-dihydro-1H-inden-4-yl]-1-[1-(trityl)imidazol-4-yl]ethanol (486 mg, 0.765 mmol), TEA (464.5 mg, 4.590 mmol), and ethyl oxalyl monochloride (313.3 mg, 2.295 mmol) were dissolved in DCM (10 mL), and the mixture was stirred at 0 °C for 2 h under nitrogen atmosphere. The reaction was quenched by adding water at 0 °C, and the mixture was then extracted with CH₂Cl₂ (3 × 50 mL). The organic phase was washed with brine (1 × 50 mL), dried over anhydrous Na₂SO₄, filtered, and concentrated under reduced pressure. The resulting residue was purified by reversed-phase flash chromatography under the following conditions: column: C18 silica gel; mobile phase: MeCN in water (10 mmol/L NH₄HCO₃), with a gradient of 10% to 50% within 10 min; detector: UV 254 nm to give 4-[1-[3-[(tert-butyldimethylsilyl)oxy]-6-chloro-2,3-dihydro-1H-inden-4-yl]vinyl]-1-(trityl)imidazole (350 mg) as a yellow oil. (ES,m/z): [M+1]=617.

22.5 4-[1-[3-[(tert-Butyldimethylsilyl)oxy]-6-chloro-2,3-dihydro-1H-inden-4-yl]vinyl]-1-(trityl)imidazole (349 mg, 0.565 mmol) and tris(triphenylphosphine)rhodium(I) chloride (14694-95-2) (104.6 mg, 0.113 mmol) were dissolved in MeOH (6 mL), and the mixture was stirred at 50 °C overnight under hydrogen atmosphere. The resulting mixture was filtered, and the filter cake was washed with MeOH (2 × 10 mL). The resulting filtrate was concentrated under reduced pressure. The residue was purified by silica gel column chromatography and eluted with PE/EA (5:1) to give 4-[1-[3-[(tert-butyldimethylsilyl)oxy]-6-chloro-2,3-dihydro-1H-inden-4-yl]ethyl]-1-(trityl)imidazole (277 mg) as a yellow oil. (ES,m/z): [M+1]=619.

22.6 4-[1-[3-[(tert-Butyldimethylsilyl)oxy]-6-chloro-2,3-dihydro-1H-inden-4-yl]ethyl]-1-(trityl)imidazole (340 mg, 0.549 mmol) and TBAF (430.6 mg, 1.647 mmol) were dissolved in THF (6 mL), and the mixture was stirred at room temperature for 2 h. The resulting mixture was concentrated under vacuum. The residue was purified by reversed-phase flash chromatography under the following conditions: column: C18 silica gel; mobile phase: MeCN in water (10 mmol/L NH₄HCO₃) with a gradient of 10% to 50% within 10 min; detector: UV 254 nm to give 5-chloro-7-[1-(1-trityl-1H-imidazol-4-yl)ethyl]-2,3-dihydro-1H-inden-1-ol (277 mg) as a yellow oil. (ES,m/z): [M+1]=505.

22.7 5-Chloro-7-[1-(1-trityl-1H-imidazol-4-yl)ethyl]-2,3-dihydro-1H-inden-1-ol (200 mg, 0.396 mmol) and p-toluenesulfonic acid (136.38 mg, 0.792 mmol) were dissolved in MeCN (4 mL), and the mixture was stirred at 70 °C for 2 h under nitrogen atmosphere. The resulting mixture was concentrated under vacuum. The residue was purified by reversed-phase flash chromatography under the following conditions: column: C18 silica gel; mobile phase: MeCN-water (10 mmol/L NH₄HCO₃), with a gradient of 10% to 50% within 10 min; detector: UV 254 nm to give 4-[1-(6-chloro-1H-inden-4-yl)ethyl]-1H-imidazole (58 mg). (ES,m/z): [M+1]=245. ¹H NMR (400 MHz, Methanol-*d*₄) δ 7.58 (d, *J =* 1.2 Hz, 1H), 7.31 (d, *J =* 1.8 Hz, 1H), 7.03 - 6.95 (m, 2H), 6.82 (s, 1H), 6.57 (dt, *J* = 5.7, 2.0 Hz, 1H), 4.43 (q, *J* = 7.2 Hz, 1H), 3.40 (d, *J* = 2.2 Hz, 2H), 1.61 (d, *J =* 7.2 Hz, 3H).

### Example 23. Preparation of 4-[(2H-Chroman-8-yl)methyl]-1H-imidazole

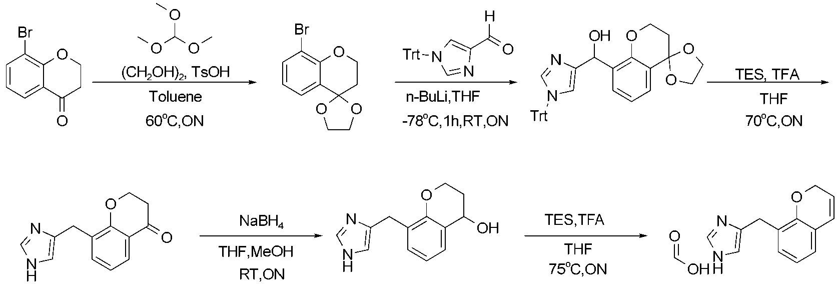

23.1 8-Bromo-2,3-dihydro-1-benzopyran-4-one (300 mg, 1.321 mmol), toluene (10 mL), ethylene glycol (0.5 mL), trimethyl orthoformate (1 mL), and p-toluenesulfonic acid (22.75 mg, 0.132 mmol) were added to a 40 mL sealed tube at room temperature. The resulting mixture was stirred at 60 °C overnight under nitrogen atmosphere. The mixture was cooled to room temperature. The resulting mixture was extracted with EtOAc (3 × 100 mL). The combined organic layer was washed with brine (3 × 100 mL) and dried over anhydrous Na₂SO₄. After filtration, the filtrate was concentrated under reduced pressure. The residue was purified by silica gel column chromatography and eluted with PE/EA (1:1) to give 8-bromo-2,3-dihydrospiro[1-benzopyran-4,2'-[1,3]dioxolane] (270 mg) as a white solid. (ES,m/z): [M+1]=271, 273.

23.2 8-Bromo-2,3-dihydrospiro[1-benzopyran-4,2'-[1,3]dioxolane] (1.5 g, 5.533 mmol) was dissolved in THF (40 mL), and n-butyllithium (2.43 mL, 6.086 mmol, 1.1 eq, 2.5 M in n-hexane) was added at -78 °C under nitrogen atmosphere. The mixture was reacted for 1 h, and a solution of 1-(trityl)imidazole-4-carbaldehyde (2.06 g, 6.086 mmol) in tetrahydrofuran (10 mL) was then added dropwise at -78 °C. The resulting mixture was stirred at room temperature overnight under nitrogen atmosphere. The reaction was quenched by adding saturated NH₄Cl (aqueous solution) (2 mL), and the resulting mixture was extracted with ethyl acetate (3 × 200 mL). The organic phases were combined, washed with brine (3 × 200 mL), dried over anhydrous sodium sulfate, filtered, and concentrated under reduced pressure. The residue was purified by reversed-phase flash chromatography under the following conditions: column: C18 silica gel; mobile phase: aqueous acetonitrile solution (10 mmol/L NH₄HCO₃), with a gradient of 50% to 70% within 10 min; detector: UV 220 nm to give 2,3-dihydrospiro[1-benzopyran-4,2'-[1,3]dioxolan]-8-yl[1-(trityl)imidazol-4-yl]methanol (1.3 g) as a white solid. (ES,m/z): [M+1]=531.

23.3 2,3-Dihydrospiro[1-benzopyran-4,2'-[1,3]dioxolan]-8-yl[1-(trityl)imidazol-4-yl]methanol (200 mg, 0.377 mmol), THF (5.00 mL), triethylsilane (438.27 mg, 3.770 mmol), and trifluoroacetic acid (429.77 mg, 37.70 mmol) were added to a 20 mL sealed tube at room temperature. The resulting mixture was stirred at 70 °C overnight under nitrogen atmosphere, cooled to room temperature, and concentrated under reduced pressure. The residue was purified by reversed-phase flash chromatography under the following conditions: column: C18 silica gel; mobile phase: acetonitrile-water solution (10 mmol/L NH₄HCO₃), with a gradient of 10% to 20% within 10 min; detector: UV 220 nm to give 8-(1H-imidazol-4-ylmethyl)-2,3-dihydro-1-benzopyran-4-one (30 mg). (ES,m/z): [M+1]=229.

23.4 NaBH₄ (124.30 mg, 3.286 mmol) was added dropwise to a stirred solution of 8-(1H-imidazol-4-ylmethyl)-2,3-dihydro-1-benzopyran-4-one (375 mg, 1.643 mmol) in THF (5 mL) and MeOH (5 mL) at 0 °C. The resulting product was stirred at room temperature for 2 h under nitrogen atmosphere. The reaction was quenched with water/ice (2 mL) at room temperature. The resulting product was concentrated under reduced pressure and purified by reversed-phase flash chromatography under the following conditions: column: C18 silica gel; mobile phase: acetonitrile-water solution (10 mmol/L NH₄HCO₃), with a gradient of 0% to 10% within 10 min; detector: ultraviolet 220 nm to give 8-(1H-imidazol-4-ylmethyl)-3,4-dihydro-2H-1-benzopyran-4-ol (355 mg). (ES,m/z): [M-1]=229.

23.5 8-(1H-Imidazol-4-ylmethyl)-3,4-dihydro-2H-1-benzopyran-4-ol (200 mg, 0.869 mmol), THF (10 mL), triethylsilane (1009.95 mg, 8.690 mmol), and TFA (990.35 mg, 8.690 mmol) were added to a 20 mL sealed tube at room temperature, and the mixture was stirred at 75 °C overnight under nitrogen atmosphere. The resulting product was concentrated under reduced pressure to give a crude product (60 mg), which was then purified by prep-HPLC (conditions: column: XBridge Prep Phenyl OBD column 19 × 250 mm, 5 µm; mobile Phase A: water (0.1% FA), mobile phase B: MeCN; flow rate: 60 mL/min; gradient: 6% B to 21% B within 10 min; wavelength: 254 nm/220 nm; RT1 (min): 5.3) to give 4-[(2H-chroman-8-yl)methyl]-1H-imidazole formate (22.7 mg). (ES,m/z): [M+1]=213. ¹H NMR (400 MHz, Methanol-d4) δ 8.49 (s, 1H), 8.19 (s, 1H), 6.97 (d, J = 8.5 Hz, 2H), 6.88 (d, J = 7.3 Hz, 1H), 6.79 (t, J = 7.5 Hz, 1H), 6.43 (d, J = 9.8 Hz, 1H), 5.81 (dt, J = 9.9, 3.6 Hz, 1H), 4.87 - 4.69 (m, 2H), 3.90 (s, 2H).

### Example 24. Preparation of 4-[1-(5-Fluoro-2,3-dihydro-1-benzofuran-7-yl)ethyl]-1H-imidazole

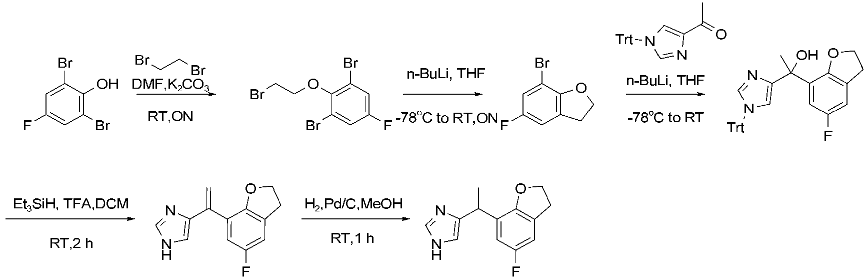

24.1 2,6-Dibromo-4-fluorophenol (2 g, 7.410 mmol) was dissolved in DMF (30 mL) under nitrogen atmosphere, and K₂CO₃ (2.1 g, 14.820 mmol) was added. The mixture was stirred for 15 min, and then dibromoethane (2.1 g, 11.115 mmol) was added dropwise. The resulting mixture was stirred at room temperature overnight, diluted by adding water (100 mL), and then extracted with ethyl acetate (3 × 100 mL). The organic phases were combined, washed with water (1 × 100 mL), and dried over anhydrous Na₂SO₄. The residue was purified by reversed-phase flash chromatography under the following conditions: column: C18 silica gel; mobile phase: acetonitrile-water (0.1% FA), with a gradient of 40% to 50% within 20 min; detector: UV 254 nm to give 1,3-dibromo-2-(2-bromoethoxy)-5-fluorobenzene (1.5 g) as a colorless oil.

24.2 A solution of 1,3-dibromo-2-(2-bromoethoxy)-5-fluorobenzene (2 g, 5.307 mmol) and n-butyllithium (2.1 mL, 5.360 mmol, 2.5 M in n-hexane) in THF (40 mL) was stirred at -78 °C for 1 h under nitrogen atmosphere. The resulting mixture was stirred at room temperature overnight under nitrogen atmosphere. The reaction was quenched with saturated NH₄Cl (aqueous solution) at room temperature and extracted with CH₂Cl₂ (3 × 100 mL). The organic phases were combined, washed with water (1 × 100 mL), dried over anhydrous Na₂SO₄, filtered, concentrated under reduced pressure, and then purified by reversed-phase flash chromatography under the following conditions: column: C18 silica gel; mobile phase: acetonitrile-water (0.1% TFA), with a gradient of 10% to 50% within 10 min; detector: UV 254 nm to give 7-bromo-5-fluoro-2,3-dihydro-1-benzofuran (300 mg) as a tan oil.

24.3 n-BuLi (0.4 mL, 1.071 mmol, 2.5 M in n-hexane) was added to a solution of 7-bromo-5-fluoro-2,3-dihydro-1-benzofuran (155 mg, 0.714 mmol) in THF (3 mL) at - 78 °C under nitrogen atmosphere. The mixture was stirred for 30 min, and then 1-[1-(trityl)imidazol-4-yl]ethanone (251.7 mg, 0.714 mmol) was added at -78 °C. The resulting mixture was stirred at room temperature for 2 h under nitrogen atmosphere. The reaction was quenched by adding saturated NH₄Cl (aqueous solution) at room temperature. The resulting mixture was extracted with CH₂Cl₂ (3 × 100 mL). The organic layers were combined, washed with water (1 × 100 mL), dried over anhydrous Na₂SO₄, filtered, concentrated under reduced pressure, and then purified by reversed-phase flash chromatography under the following conditions: column: C18 silica gel; mobile phase: MeCN-water (10 mmol/L NH₄HCO₃), with a gradient of 10% to 70% within 10 min; detector: UV 254 nm to give 1-(5-fluoro-2,3-dihydro-1-benzofuran-7-yl)-1-[1-(trityl)imidazol-4-yl]ethanol (100 mg) as a white solid. (ES,m/z): [M+1]=491. 24.4 1-(5-Fluoro-2,3-dihydro-1-benzofuran-7-yl)-1-[1-(trityl)imidazol-4-yl]ethanol (100 mg, 0.204 mmol), Et₃SiH (0.23 g, 1.978 mmol), and TFA (0.23 g, 2.040 mmol) were added to DCM (2 mL), and the mixture was stirred at room temperature for 2 h under nitrogen atmosphere. The resulting mixture was concentrated under vacuum and purified by reversed-phase flash chromatography under the following conditions: column: C18 silica gel; mobile phase: MeCN-water (10 mmol/L NH₄HCO₃), with a gradient of 10% to 30% within 10 min; detector: UV 254 nm to give 4-[1-(5-fluoro-2,3-dihydro-1-benzofuran-7-yl)vinyl]-1H-imidazole (42 mg). (ES, m/z): [m+1]=231.

24.5 4-[1-(5-Fluoro-2,3-dihydro-1-benzofuran-7-yl)vinyl]-1H-imidazole (48 mg, 0.208 mmol) and 10% Pd/C (20 mg) were added to MeOH, and the mixture was stirred at room temperature for 1 h under hydrogen atmosphere. The resulting mixture was filtered through a pad of celite, and the filter cake was washed with MeOH (3 × 10 mL). The filtrate was concentrated under reduced pressure to give a crude product (40 mg), which was purified by prep-HPLC under the following conditions (column: XBridge Shield RP18 OBD column 30 × 150 mm, 5 µm; mobile phase A: water (10 mmol/L NH₄HCO₃ + 0.1% NH₃.H₂O), mobile phase B: MeCN; flow rate: 60 mL/min; gradient: 27% B to 47% B within 10 min; wavelength: 254 nm/200 nm; RT1 (min): 10.63) to give 4-[1-(5-fluoro-2,3-dihydro-1-benzofuran-7-yl)ethyl]-1H-imidazole (13.5 mg). (ES,m/z): [M+1]= 233. ¹H NMR (400 MHz, Methanol-*d*₄) δ 7.56 (d, *J =* 1.2 Hz, 1H), 6.83 - 6.74 (m, 2H), 6.51 (dd, *J* = 10.3, 2.7 Hz, 1H), 4.55 (td, *J =* 8.8, 1.6 Hz, 2H), 4.26 (q, *J =* 7.2 Hz, 1H), 3.27 - 3.12 (m, 2H), 1.52 (d, *J =* 7.2 Hz, 3H).

### Example 25. Preparation of 4-[1-(1-Benzofuran-7-yl)ethyl]-1H-imidazole

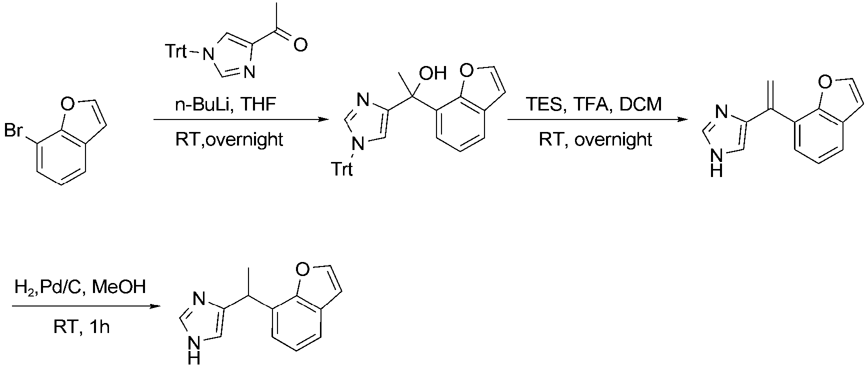

25.1 7-Bromo-1-benzofuran (500 mg, 2.538 mmol) was dissolved in THF (14 mL) at - 78 °C under nitrogen atmosphere, and the mixture was stirred for 2 min. n-BuLi (1.52 mL, 3.807 mmol, 2.5 M in n-hexane) was then added to the mixture described above over 1 min. The resulting mixture was stirred at -78 °C for another 1 h. 1-[1-(Trityl)imidazol-4-yl]ethanone (983.8 mg) was added dropwise to the mixture described above at -78 °C within 2 min. The resulting mixture was stirred at room temperature overnight. The reaction was quenched by adding NH₄Cl (aqueous solution) (10 mL) at room temperature. The resulting mixture was extracted with EtOAc (3 × 50 mL). The combined organic phase was washed with water (1 × 50 mL), dried over anhydrous Na₂SO₄, filtered, concentrated under reduced pressure, and then purified by reversed-phase flash chromatography under the following conditions: column: C18 silica gel; mobile phase: acetonitrile-water solution (10 mmol/L NH₄HCO₃), with a gradient of 70%-75% within 20 min; detector: UV 254 nm, to give 1-(1-benzofuran-7-yl)-1-[1-(trityl)imidazol-4-yl]ethanol (400 mg). (ES,m/z): [M]=470. ¹H NMR (400 MHz, Chloroform-*d*) δ 7.54 (dd, *J =* 7.6, 1.2 Hz, 1H), 7.48 (td, *J =* 3.4, 1.2 Hz, 2H), 7.38 (d, *J =* 2.4 Hz, 1H), 7.34 - 7.30 (m, 9H), 7.22 (t, *J =* 7.7 Hz, 1H), 7.14 - 7.09 (m, 6H), 6.85 (d, *J =* 1.4 Hz, 1H), 6.73 (d, *J =* 2.2 Hz, 1H), 5.29 (s, 1H), 2.01 (s, 3H).

25.2 Triethylsilane (2 mL) was added to a solution of 1-(1-benzofuran-7-yl)-1-[1-(trityl)imidazol-4-yl]ethanol (400 mg, 0.893 mmol) and TFA (2 mL) in DCM (8 mL) at room temperature, and the mixture was stirred overnight. The resulting mixture was concentrated under reduced pressure. The residue was purified by reversed-phase flash chromatography under the following conditions: column: C18 silica gel; mobile phase: acetonitrile-water (0.1% FA), with a gradient of 20% to 25% within 20 min; detector: UV 254 nm, to give 4-[1-(1-benzofuran-7-yl)vinyl]-1H-imidazole (130 mg). (ES,m/z): [2M+1]=421.

25.3 In a pressure tank, Pd/C (15 mg, 10% w/t) was added to a solution of 4-[1-(1-benzofuran-7-yl)vinyl]-1H-imidazole (120 mg, 0.571 mmol) in MeOH (3 mL). The mixture was hydrogenated at room temperature under a hydrogen pressure of 30 psi for 1 h. After the reaction was completed as monitored by LCMS, the resulting product was filtered through celite and concentrated under reduced pressure to give a crude product (120 mg), which was purified by prep-HPLC under the following conditions (column: XBridge Prep OBD C18 column 30 × 150 mm, 5 µm; mobile phase A: water (10 mmol/L NH₄HCO₃), mobile phase B: MeCN; flow rate: 60 mL/min; gradient: 19% B to 37% B within 10 min; wavelength: 254 nm/220 nm; RT1 (min): 10.63/11.72) to give 4-[1-(1-benzofuran-7-yl)ethyl]-1H-imidazole (20.7 mg). (ES,m/z): [M+1]=213. ¹H NMR (400 MHz, Methanol-*d*₄) δ 7.71 (t, *J =* 2.5 Hz, 1H), 7.56 (d, *J =* 1.3 Hz, 1H), 7.44 (dt, *J =* 7.7, 1.6 Hz, 1H), 7.13 (td, *J =* 7.6, 1.8 Hz, 1H), 7.03 (dd, *J =* 7.5, 1.2 Hz, 1H), 6.85 - 6.77 (m, 2H), 4.67 (q, *J =* 7.2 Hz, 1H), 1.69 (dd, *J =* 7.3, 1.6 Hz, 3H).

### Example 26. Preparation of 4-[1-(6-Fluoro-3H-inden-4-yl)ethyl]-1H-imidazole

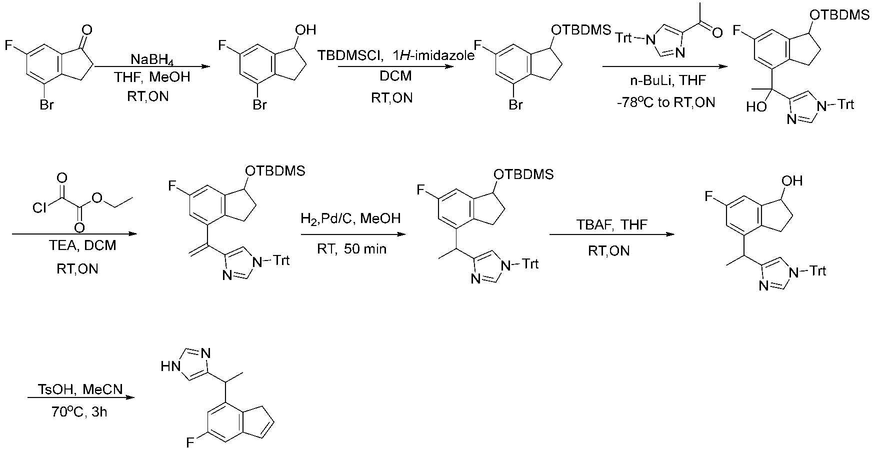

26.1 A mixed solution of 4-bromo-6-fluoro-2,3-dihydroinden-1-one (1 g, 4.366 mmol, 1 equiv) in THF (10 mL) and MeOH (5 mL) was treated with NaBH₄ (0.66 g, 17.464 mmol, 4 equiv) at 0 °C under nitrogen atmosphere. The resulting mixture was stirred at room temperature overnight under nitrogen atmosphere. The reaction was quenched by adding water (50 mL) at room temperature. The resulting mixture was extracted with CH₂Cl₂ (3 × 50 mL). The combined organic layer was washed with water (1 × 50 mL) and dried over anhydrous Na₂SO₄. After filtration, the filtrate was concentrated under reduced pressure. The residue was purified by silica gel column chromatography and eluted with PE/EA (5:1) to give 4-bromo-6-fluoro-2,3-dihydro-1*H*-inden-1-ol (950 mg) as a colorless oil. (ES,m/z): [M]=230, 232.

26.2 tert-Butyldimethylsilyl chloride (704.5 mg, 4.674 mmol) was added to a stirred solution of 4-bromo-6-fluoro-2,3-dihydro-1*H*-inden-1-ol (900 mg, 3.895 mmol) and imidazole (530.3 mg, 7.790 mmol) in DCM (20 mL) at room temperature under nitrogen atmosphere. The resulting mixture was stirred at room temperature overnight under nitrogen atmosphere. The resulting mixture was concentrated under reduced pressure. The residue was purified by silica gel column chromatography and eluted with PE/EA (12:1) to give [(4-bromo-6-fluoro-2,3-dihydro-1H-inden-1-yl)oxy](tert-butyl)dimethylsilane (1.1 g) as a colorless oil.

26.3 n-BuLi (2.08 mL, 5.213 mmol, 2.5 M in n-hexane) was added to a solution of [(4-bromo-6-fluoro-2,3-dihydro-1H-inden-1-yl)oxy](tert-butyl)dimethylsilane (1.2 g, 3.475 mmol) in THF (10 mL) at -78 °C under nitrogen atmosphere. After 40 min of the reaction, a solution of 1-[1-(trityl)imidazol-4-yl]ethanone (1.35 g, 3.823 mmol) in THF (5 mL) was added dropwise at -78 °C. The mixture was stirred at room temperature overnight under nitrogen atmosphere. The reaction was quenched by adding a saturated aqueous NH₄Cl solution (50 mL) at room temperature. The resulting mixture was extracted with CH₂Cl₂ (3 × 50 mL). The combined organic layer was washed with water (1 × 50 mL) and dried over anhydrous Na₂SO₄. After filtration, the filtrate was concentrated under reduced pressure. The residue was purified by silica gel column chromatography and eluted with PE/EA (1:1) to give 1-[1-[(tert-butyldimethylsilyl)oxy]-6-fluoro-2,3-dihydro-1*H*-inden-4-yl]-1-[1-(trityl)imidazol-4-yl]ethanol (1.4 g) as a white solid. (ES, m/z): [M+1]=619.

26.4 A solution of 1-[1-[(tert-butyldimethylsilyl)oxy]-6-fluoro-2,3-dihydro-1*H*-inden-4-yl]-1-[1-(trityl)imidazol-4-yl]ethanol (1.5 g, 2.424 mmol) in DCM (20 mL) was treated with triethylamine (1.47 g, 14.544 mmol) at room temperature for 1 min under nitrogen atmosphere, and then ethyl oxalyl monochloride (0.99 g, 7.7.272 mmol) was added dropwise at 0 °C. The resulting mixture was stirred at room temperature overnight under nitrogen atmosphere. The resulting mixture was concentrated under vacuum. The residue was purified by silica gel column chromatography and eluted with PE/EA (5:1) to give 4-[1-[1-[(tert-butyldimethylsilyl)oxy]-6-fluoro-2,3-dihydro-1*H-*inden-4-yl]vinyl]-1-(trityl)imidazole (1.1 g) as a yellow oil. (ES, m/z): [M+1]=601.

26.5 A solution of 4-[1-[1-[(tert-butyldimethylsilyl)oxy]-6-fluoro-2,3-dihydro-1*H-*inden-4-yl]vinyl]-1-(trityl)imidazole (1.5 g, 2.496 mmol) in MeOH (15 mL) was treated with Pd/C (0.13 g, 10% w/t) at room temperature. The resulting mixture was stirred at room temperature for 50 min under hydrogen atmosphere. The resulting mixture was filtered through celite, and the filter cake was washed with MeOH (3 × 10 mL). The filtrate was concentrated under reduced pressure. The residue was purified by silica gel column chromatography and eluted with PE/EA (5:1) to give 4-[1-[1-[(tert-butyldimethylsilyl)oxy]-6-fluoro-2,3-dihydro-1*H*-inden-4-yl]ethyl]-1-(trityl)imidazole (1.2 g) as a white solid. (ES, m/z): [M+1]=603.

26.6 A solution of 4-[1-[1-[(tert-butyldimethylsilyl)oxy]-6-fluoro-2,3-dihydro-1*H-*inden-4-yl]ethyl]-1-(trityl)imidazole (1 g, 1.659 mmol) in THF (10 mL) was treated with tetrabutylammonium fluoride (5.0 mL, 4.977 mmol, 1 M in THF) at room temperature. The resulting mixture was stirred at room temperature overnight under nitrogen atmosphere. The reaction was quenched by adding water (20 mL) at room temperature. The resulting mixture was extracted with EtOAc (3 × 20 mL). The combined organic layer was washed with water (1 × 20 mL) and dried over anhydrous Na₂SO₄. After filtration, the filtrate was concentrated under reduced pressure. The residue was purified by silica gel column chromatography and eluted with PE/EA (5:1) to give 6-fluoro-4-[1-[1-(trityl)imidazol-4-yl]ethyl]-2,3-dihydro-1*H*-inden-1-ol (443 mg) as a white solid. (ES, m/z): [M+1]=489.

26.7 A solution of 6-fluoro-4-[1-[1-(trityl)imidazol-4-yl]ethyl]-2,3-dihydro-1*H*-inden-1-ol (150 mg, 0.307 mmol) in acetonitrile (5 mL) was treated with p-toluenesulfonic acid (52.7 mg, 0.307 mmol) at room temperature. The resulting mixture was stirred at 70 °C for 3 h under nitrogen atmosphere. The mixture was cooled to room temperature. The resulting mixture was concentrated under reduced pressure. The residue was purified by reversed-phase flash chromatography under the following conditions: column: C18 silica gel; mobile phase: acetonitrile in water (0.1% NH₃.H₂O + 10 mmol/L NH₄HCO₃), with a gradient of 10% to 50% within 10 min; detector: UV 220 nm to give 4-[1-(6-fluoro-3*H*-inden-4-yl)ethyl]-1*H*-imidazole (16 mg). (ES, m/z): [M+1]=229. ¹H NMR (400 MHz, Methanol-*d*₄) δ 7.57 (d, *J =* 1.2 Hz, 1H), 6.95 (dd, *J* = 8.7, 2.4 Hz, 1H), 6.87 - 6.82 (m, 2H), 6.70 (dd, *J =* 10.9, 2.4 Hz, 1H), 6.64 (dt, *J =* 5.6, 2.1 Hz, 1H), 4.46 - 4.19 (m, 1H), 3.36 (dq, *J =* 23.3, 1.8 Hz, 1H), 3.26 (dq, *J =* 23.2, 1.8 Hz, 1H), 1.61 (d, *J =* 7.2 Hz, 3H).

### Example 27. Preparation of 4-[1-(Benzothiophen-7-yl)ethyl]-1H-imidazole

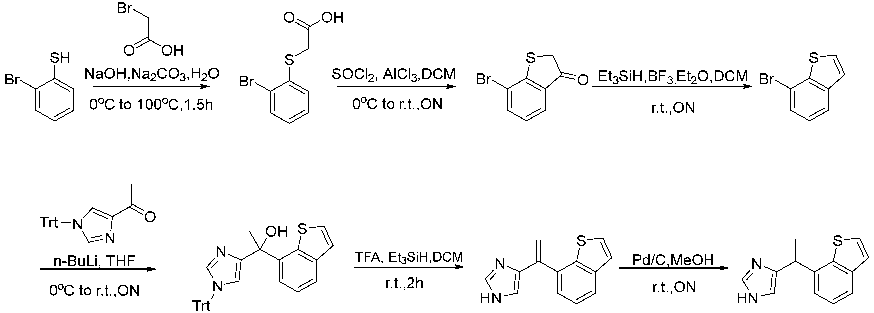

27.1 2-Bromoacetic acid (1.2 g, 8.991 mmol) and an aqueous Na₂CO₃ (952.9 mg, 8.991 mmol) solution (40 mL) were added dropwise to a mixture of 2-bromothiophenol (1.7 g, 8.991 mmol) and NaOH (359.6 mg, 8.991 mmol) in water (10 mL) at 0 °C. The resulting mixture was stirred at 100 °C for 1.5 h. The mixture was acidified to pH = 1 with HCl (aq.). The resulting mixture was extracted with EtOAc (2 × 50 mL). The combined organic layer was washed with H₂O (3 × 50 mL) and dried over anhydrous Na₂SO₄. After filtration, the filtrate was concentrated under reduced pressure to give [(2-bromophenyl)thio]acetic acid (1.9 g) as a pale yellow solid. The crude product was directly used in the next step without further purification. (ES,m/z): [M-1]=245, 247

27.2 [(2-Bromophenyl)thio]acetic acid (1.9 g, 7.689 mmol) and SOCl₂ (1.8 g, 15.378 mmol) were added to a DCM (30 mL) solution under nitrogen atmosphere, and the mixture was stirred at 0 °C for 1 h. The resulting mixture was concentrated under reduced pressure. The residue was dissolved in DCM (20 mL). AlCl₃ (4.2 g, 31.564 mmol) was slowly added to the mixture described above at 0 °C (more than 5 min). The mixture was then transferred to an environment at room temperature and stirred overnight. The reaction was quenched by adding HCl (aq.) (0.5 mL) at 0 °C. The resulting mixture was extracted with CH₂Cl₂ (3 × 50 mL). The combined organic layer was washed with H₂O (1 × 50 mL) to give 7-bromo-2H-1-benzothiophen-3-one (375.0 mg) as a pink solid. (ES,m/z): [M-1]= 227, 229.

27.3 7-Bromo-2H-1-benzothiophen-3-one (375.0 mg, 1.637 mmol), Et₃SiH (3.8 mL), and BF₃.Et₂O (3.8 mL) were added to a DCM (9 mL) solution under nitrogen atmosphere, and the mixture was stirred at room temperature overnight. The resulting mixture was diluted with water (10 mL), extracted with CH₂Cl₂ (3 × 100 mL), and dried over anhydrous Na₂SO₄. After filtration, the filtrate was concentrated under reduced pressure. The residue was purified by silica gel column chromatography and eluted with PE/EA (3:1) to give 7-bromobenzothiophene (230.0 mg) as a colorless oil. (ES,m/z): M=212, 214.

27.4 n-BuLi (0.6 mL, 1.603 mmol, 2.5 M in n-hexane) was added dropwise to a solution of 7-bromobenzothiophene (230.0 mg, 1.069 mmol) in THF (5 mL) at -78 °C under nitrogen atmosphere. The mixture was stirred for 1h, 1-[1-(trityl)imidazol-4-yl]ethanone (376.8 mg, 1.069 mmol) was then added dropwise to the mixture described above, and the mixture was transferred to an environment at room temperature and stirred overnight. The reaction was quenched by adding saturated NH₄Cl. The resulting mixture was extracted with EtOAc (3 × 50 mL). The combined organic layer was washed with H₂O (3 × 50 mL) and dried over anhydrous Na₂SO₄. After filtration, the filtrate was concentrated under reduced pressure. The residue was purified by silica gel column chromatography and eluted with PE/EA (3:1) to give 1-(1-benzothiophen-7-yl)-1-[1-(trityl)imidazol-4-yl]ethanol (210 mg) as a yellow oil. (ES,m/z): [M+1]=488. 27.5 1-(1-Benzothiophen-7-yl)-1-[1-(trityl)imidazol-4-yl]ethanol (210 mg, 0.432 mmol, 1 equiv), TFA (492.05 mg, 4.320 mmol), and Et₃SiH (250.89 mg, 2.160 mmol) were added to a DCM (4 mL) solution under nitrogen atmosphere, and the mixture was stirred at room temperature for 2 h. The mixture was concentrated under reduced pressure and purified by reversed-phase chromatography under the following conditions: chromatographic column: C18 silica gel; mobile phase: acetonitrile-water (0.1% FA), with a gradient of 10% to 40% within 10 min; detector: UV 254 nm to give 4-[1-(1-benzothiophen-7-yl)vinyl]-1H-imidazole (50 mg) as a yellow oil. (ES,m/z): [M+1]= 227.

27.6 4-[1-(1-Benzothiophen-7-yl)vinyl]-1H-imidazole (50 mg, 0.221 mmol) and 10% Pd/C (20 mg) were added to a MeOH (1 mL) solution under hydrogen atmosphere, and the mixture was stirred at room temperature overnight. The resulting mixture was filtered through a pad of Celite, and the filter cake was washed with MeOH (3 × 10 mL). The filtrate was concentrated under reduced pressure and purified by reversed-phase chromatography under the following conditions: chromatographic column: C18 silica gel; mobile phase: acetonitrile-water (0.1% FA), with a gradient of 10% to 30% within 10 min; detector: UV 254 nm to give 4-[1-(benzothiophen-7-yl)ethyl]-1H-imidazole (15.9 mg) as a colorless oil. (ES,m/z): [M+1]= 229. ¹H NMR (400 MHz, Methanol-*d*₄) δ 8.84 (s, 1H), 7.79 (d, *J =* 7.8 Hz, 1H), 7.74 (d, *J =* 7.7 Hz, 1H), 7.49 (s, 1H), 7.38 - 7.25 (m, 2H), 7.23 (s, 1H), 4.69 (q, *J =* 7.1 Hz, 1H), 1.82 (d, *J =* 7.1 Hz, 3H).

### Example 28. Preparation of 4-[1-(1-Ethyl-2,3-dihydro-1H-inden-4-yl)ethyl]-1H-imidazole

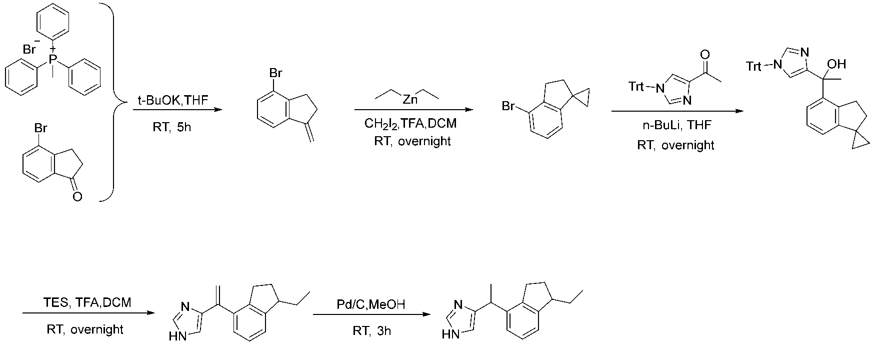

28.1 4-Bromo-2,3-dihydroinden-1-one (4 g, 18.952 mmol) and methyltriphenylphosphonium bromide (13.54 g, 37.904 mmol) were added to a THF (80 mL) solution under nitrogen atmosphere, and the mixture was stirred at 20 °C for 5 h and quenched by adding ice water at 0 °C. The resulting mixture was extracted with EtOAc (3 × 100 mL), and the combined organic layer was washed with brine (3 × 50 mL) and dried over anhydrous Na₂SO₄. After filtration, the filtrate was concentrated under reduced pressure. The residue was purified by silica gel column chromatography and eluted with PE/EA (12:1) to give 4-bromo-1-methylene-2,3-dihydroindene (3.8 g) as a yellow oil. (ES,m/z): [M]=208.

28.2 TFA (1.46 mL, 19.610 mmol) was added to a solution of diethylzinc (19.61 mL, 19.610 mmol) in DCM (20 mL) at 0 °C under nitrogen atmosphere, and the mixture was stirred for 20 min. Diiodomethane (5.25 g, 19.610 mmol) was then added dropwise at 0 °C. The resulting mixture was stirred at 0 °C for 20 min. Finally, a solution of 4-bromo-1-methylene-2,3-dihydroindene (820 mg, 3.922 mmol) in DCM (10 mL) was added dropwise to the mixture described above at 0 °C, and the mixture was stirred at room temperature overnight. The reaction was quenched by adding saturated NH₄Cl (30 mL). The resulting mixture was extracted with EtOAc (2 × 70 mL). The combined organic layer was washed with brine (3 × 100 mL) and dried over anhydrous Na₂SO₄. After filtration, the filtrate was concentrated under reduced pressure. The residue was purified by silica gel column chromatography and eluted with PE/EA (4:1) to give 4'-bromo-2',3'-dihydrospiro[cyclopropane-1,1'-indene] (250 mg) as a colorless oil. (ES,m/z): [M]=222.

28.3 n-BuLi (0.67 mL, 1.681 mmol) was added dropwise to a solution of 4'-bromo-2',3'-dihydrospiro[cyclopropane-1,1'-indene] (250 mg, 1.121 mmol) in THF (5 mL) at -78 °C under nitrogen atmosphere. The mixture was stirred for 1 h, 1-[1-(trityl)imidazol-4-yl]ethanone (473.9 mg, 1.345 mmol) was added dropwise to the mixture described above, and the mixture was transferred to an environment at room temperature and stirred overnight. The reaction was quenched by adding saturated NH₄Cl (15 mL). The resulting mixture was extracted with EtOAc (2 × 50 mL). The combined organic layer was washed with brine (3 × 50 mL) and dried over anhydrous Na₂SO₄. After filtration, the filtrate was concentrated under reduced pressure. The residue was purified by reversed-phase chromatography under the following conditions: chromatographic column: C18 silica gel; mobile phase: acetonitrile-water (10 mmol/L NH₄HCO₃), with a gradient of 30% to 80% within 10 min; detector: UV 220 nm to give 1-(2',3'-dihydrospiro[cyclopropane-1,1'-inden]-4'-yl)-1-[1-(trityl)imidazol-4-yl]ethanol (300 mg) as a light yellow solid. (ES,m/z): [M+1]=497.

28.4 1-(2',3'-Dihydrospiro[cyclopropane-1,1'-inden]-4'-yl)-1-[1-(trityl)imidazol-4-yl]ethanol (100 mg, 0.201 mmol), TFA (688.8 mg, 6.030 mmol), and Et₃SiH (702.4 mg, 6.030 mmol) were added to a DCM (3 mL) solution under nitrogen atmosphere, and the mixture was stirred at room temperature overnight. The mixture was concentrated under reduced pressure and purified by reversed-phase chromatography under the following conditions: chromatographic column: C18 silica gel; mobile phase: acetonitrile-water (10 mmol/L NH₄HCO₃), with a gradient of 40% to 70% within 10 min; detector: UV 220 nm to give 4-[1-(1-ethyl-2,3-dihydro-1H-inden-4-yl)vinyl]-1H-imidazole (80 mg) as an off-white solid. (ES,m/z): [M+1]=239.

28.5 4-[1-(1-Ethyl-2,3-dihydro-1H-inden-4-yl)vinyl]-1H-imidazole (70 mg, 0.294 mmol) and 10% Pd/C (8 mg, 10% w/t) were added to a MeOH (2 mL) solution under hydrogen atmosphere, and the mixture was stirred at room temperature for 3 h. The resulting mixture was filtered through a pad of Celite, and the filter cake was washed with MeOH (3 × 10 mL). The filtrate was concentrated under reduced pressure and purified by reversed-phase chromatography under the following conditions: chromatographic column: C18 silica gel; mobile phase: acetonitrile-water (10 mmol/L NH₄HCO₃), with a gradient of 30% to 70% within 10 min; detector: UV 220 nm to give 4-[1-(1-ethyl-2,3-dihydro-1H-inden-4-yl)ethyl]-1H-imidazole (40.7 mg). (ES,m/z):[M+1]=241. ¹H NMR (400 MHz, Methanol-*d*₄) δ 7.56 (s, 1H), 7.12 - 6.98 (m, 2H), 6.90 (dd, *J =* 11.5, 7.4 Hz, 1H), 6.72 (d, *J =* 7.8 Hz, 1H), 4.18 (d, *J =* 7.3 Hz, 1H), 3.09 - 2.81 (m, 2H), 2.74 (tt, *J =* 15.7, 8.1 Hz, 1H), 2.26 (dq, *J =* 8.2, 4.1 Hz, 1H), 1.87 (ddd, *J =* 12.4, 7.4, 4.8 Hz, 1H), 1.66 (s, 1H), 1.55 (td, *J =* 5.7, 3.0 Hz, 3H), 1.41 (q, *J* = 12.3, 9.5 Hz, 1H), 0.98 (td, *J =* 7.4, 3.7 Hz, 3H).

### Example 29. Preparation of 4-[(3-Ethyl-2,3-dihydro-1H-inden-4-yl)methyl]-1H-imidazole

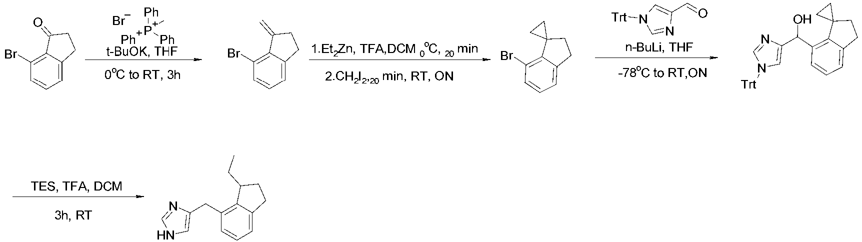

29.1 t-BuOK (5.21 mL, 1 M in THF) was added dropwise to a solution of 7-bromo-2,3-dihydroinden-1-one (1 g, 4.738 mmol) and methyltriphenylphosphonium bromide (5.92 g, 16.583 mmol) in THF (5 mL) at 0 °C under nitrogen atmosphere, and the mixture was transferred to an environment at room temperature and stirred for 3 h. The reaction was quenched by adding ice water at 0 °C. The resulting mixture was extracted with EtOAc (3 × 10 mL), and the combined organic layer was washed with brine (1 × 20 mL) and dried over anhydrous Na₂SO₄. After filtration, the filtrate was concentrated under reduced pressure. The residue was purified by silica gel column chromatography and eluted with PE/EA (10:1) to give 7-bromo-1-methylene-2,3-dihydroindene (657 mg) as a yellow oil. (ES,m/z): [M]= 208, 210.

29.2 TFA (545.34 mg, 4.785 mmol) was added to a solution of diethylzinc (4.78 mL, 4.785 mmol) in DCM (10 mL) at 0 °C under nitrogen atmosphere, and the mixture was stirred for 20 min. Diiodomethane (1280.9 mg, 4.785 mmol) was then added dropwise at room temperature, and the mixture was stirred for 20 min. Finally, a solution of 7-bromo-1-methylene-2,3-dihydroindene (500 mg, 2.563 mmol) in DCM (10 mL) was added dropwise to the mixture described above at room temperature, and the mixture was stirred at room temperature overnight. The reaction was quenched by adding saturated NH₄Cl (50 mL). The resulting mixture was extracted with EtOAc (1 × 50 mL). The combined organic layer was washed with brine (1 × 50 mL) and dried over anhydrous Na₂SO₄. After filtration, the filtrate was concentrated under reduced pressure. The residue was purified by silica gel column chromatography and eluted with PE/EA (5:1) to give 7'-bromo-2',3'-dihydrospiro[cyclopropane-1,1'-indene] (170 mg) as a light yellow oil. (ES,m/z): [M]= 222, 224.

29.3 n-BuLi (1.21 mL, 3.026 mmol, 2.5 M in n-hexane) was added dropwise to a solution of 7'-bromo-2',3'-dihydrospiro[cyclopropane-1,1'-indene] (450 mg, 2.017 mmol) in THF (10 mL) at -78 °C under nitrogen atmosphere. The mixture was stirred for 1 h, 1-[1-(trityl)imidazol-4-yl]ethanone (682.5 mg, 2.017 mmol) was added dropwise to the mixture described above, and the mixture was transferred to an environment at room temperature and stirred overnight. The reaction was quenched by adding saturated NH₄Cl (20 mL). The resulting mixture was extracted with EtOAc (3 × 20 mL). The combined organic layer was washed with brine (1 × 20 mL) and dried over anhydrous Na₂SO₄. After filtration, the filtrate was concentrated under reduced pressure. The residue was purified by silica gel column chromatography and eluted with PE/EA (1:1) to give 2',3'-dihydrospiro[cyclopropane-1,1'-inden]-7'-yl[1-(trityl)imidazol-4-yl]methanol (450 mg) as an off-white solid. (ES,m/z): [M+1]=483.

29.4 2',3'-Dihydrospiro[cyclopropane-1,1'-inden]-7'-yl[1-(trityl)imidazol-4-yl]methanol (150 mg, 0.311 mmol), TFA (567.01 mg, 4.980 mmol), and Et₃SiH (578.2 mg, 4.980 mmol) were added to a DCM (5 mL) solution under nitrogen atmosphere, and the mixture was stirred at room temperature overnight. The mixture was concentrated under reduced pressure, and the residue (160 mg) was purified by preparative high-performance liquid chromatography under the following conditions (chromatographic column: XBridge Shield RP18 OBD chromatographic column 30 × 150 mm, 5 µm; mobile phase A: water (10 mmol/L NH₄HCO₃), mobile phase B: ACN; flow rate: 60 mL/min; gradient: 26% B to 41% B within 10 min; wavelength: 254 nm/220 nm; RT1 (min): 10.23/12.13) to give 4-[(3-ethyl-2,3-dihydro-1H-inden-4-yl)methyl]-1H-imidazole (21.7 mg) as a colorless oil. (ES,m/z): [M+1]=227. ¹H NMR (400 MHz, Methanol-*d*₄) δ 7.55 (d, *J =* 1.2 Hz, 1H), 7.03 (d, *J* = 4.6 Hz, 2H), 6.91 (q, *J* = 4.1 Hz, 1H), 6.59 (s, 1H), 4.07 - 3.79 (m, 2H), 3.11 (dddd, *J =* 9.6, 8.0, 3.4, 1.5 Hz, 1H), 2.97 (ddd, *J =* 15.8, 10.5, 7.9 Hz, 1H), 2.75 (ddd, *J =* 15.9, 8.9, 1.8 Hz, 1H), 2.16 - 1.83 (m, 2H), 1.56 (dqd, *J =* 14.8, 7.5, 3.2 Hz, 1H), 1.44 - 1.20 (m, 1H), 0.92 (t, *J =* 7.4 Hz, 3H).

### Example 30. Preparation of 4-[1-(2,3-Dihydrobenzofuran-4-yl)ethyl]-1H-imidazole

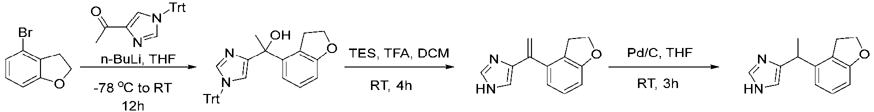

30.1 n-BuLi (0.90 mL, 2.260 mmol, 2.5 M in n-hexane) was added dropwise to a solution of 4-bromo-2,3-dihydro-1-benzofuran (300 mg, 1.507 mmol) in THF (10 mL) at -78 °C under nitrogen atmosphere. The mixture was stirred for 1h, 1-[1-(trityl)imidazol-4-yl]ethanone (584.31 mg, 1.658 mmol) was added dropwise to the mixture described above, and the mixture was transferred to an environment at room temperature and stirred overnight. The reaction was quenched by adding saturated NH₄Cl (20 mL). The resulting mixture was extracted with EtOAc (3 × 20 mL). The combined organic layer was washed with brine (1 × 20 mL) and dried over anhydrous Na₂SO₄. After filtration, the filtrate was concentrated under reduced pressure. The residue was purified by reversed-phase chromatography under the following conditions: chromatographic column: C18 silica gel; mobile phase: acetonitrile-water (10 mmol/L NH₄HCO₃), with a gradient of 50% to 55% within 10 min; detector: UV 254 nm to give 1-(2,3-dihydro-1-benzofuran-4-yl)-1-[1-(trityl)imidazol-4-yl]ethanol (430 mg) as a yellow solid. (ES, m/z): [M+1] =473.

30.2 1-(2,3-Dihydro-1-benzofuran-4-yl)-1-[1-(trityl)imidazol-4-yl]ethanol (430 mg, 0.910 mmol), TFA (1 mL), and Et₃SiH (1 mL) were added to a DCM (8 mL) solution under nitrogen atmosphere, and the mixture was stirred at room temperature overnight. The mixture was concentrated under reduced pressure, and the residue was purified by reversed-phase chromatography under the following conditions: chromatographic column: C18 silica gel; mobile phase: acetonitrile-water (0.1% NH₃.H₂O), with a gradient of 20% to 25% within 15 min; detector: UV 200 nm to give 4-[1-(2,3-dihydro-1-benzofuran-4-yl)vinyl]-1H-imidazole (170 mg) as a white solid. (ES, m/z): [M+1] =213.

30.3 4-[1-(2,3-Dihydro-1-benzofuran-4-yl)vinyl]-1H-imidazole (180 mg, 0.848 mmol) and 10% Pd/C (72.20 mg) were added to a THF (4 mL) solution under hydrogen atmosphere, and the mixture was stirred at room temperature for 3 h. The resulting mixture was filtered through a pad of Celite, and the filter cake was washed with MeOH (1 × 10 mL). The filtrate was concentrated under reduced pressure and purified by reversed-phase chromatography under the following conditions: chromatographic column: C18 silica gel; mobile phase: acetonitrile-water (0.1% NH₃.H₂O), with a gradient of 20% to 25% within 10 min; detector: UV 254 nm to give 4-[1-(2,3-dihydro-1-benzofuran-4-yl)ethyl]-1H-imidazole (129.1 mg). (ES, m/z): [M+1] =215. ¹H NMR (400 MHz, Methanol-d4) δ 7.55 (d, J = 1.2 Hz, 1H), 7.01 (t, J = 7.8 Hz, 1H), 6.76 (d, J = 1.1 Hz, 1H), 6.65 (d, J = 7.8 Hz, 1H), 6.56 (d, J = 7.9 Hz, 1H), 4.48 (t, J = 8.7 Hz, 2H), 4.10 (q, J = 7.2 Hz, 1H), 3.21 - 2.93 (m, 2H), 1.56 (d, J = 7.2 Hz, 3H).

### Example 31. Preparation of 4-[1-(2,3-Dihydro-1H-inden-4-yl)ethyl]-1H-imidazole

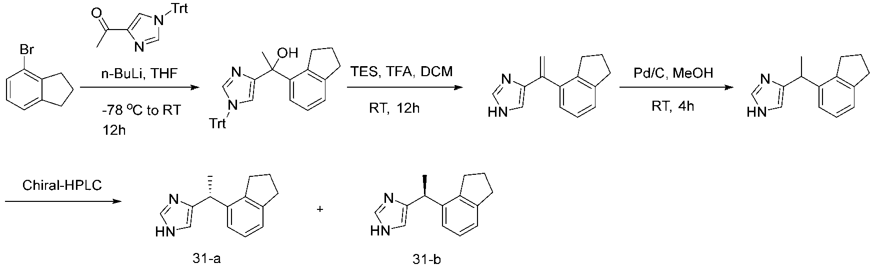

31.1 n-BuLi (0.60 mL, 10.761 mmol, 2.5 M in n-hexane) was added dropwise to a solution of 4-bromo-2,3-dihydro-1H-indene (300 mg, 1.522 mmol) in THF (8 mL) at - 78 °C under nitrogen atmosphere. The mixture was stirred for 1h, 1-[1-(trityl)imidazol-4-yl]ethanone (590.15 mg, 1.674 mmol) was added dropwise to the mixture described above, and the mixture was transferred to an environment at room temperature and stirred overnight. The reaction was quenched by adding saturated NH₄Cl (20 mL). The resulting mixture was extracted with EtOAc (3 × 20 mL). The combined organic layer was washed with brine (1 × 20 mL) and dried over anhydrous Na₂SO₄. After filtration, the filtrate was concentrated under reduced pressure. The residue was purified by reversed-phase chromatography under the following conditions: chromatographic column: C18 silica gel; mobile phase: acetonitrile-water (10 mmol/L NH₄HCO₃), with a gradient of 70% to 75% within 10 min; detector: UV 254 nm to give 1-(2,3-dihydro-1H-inden-4-yl)-1-[1-(triphenylmethyl)imidazol-4-yl]ethanol (530 mg) as a yellow solid. (ES, m/z): [M+1]=471.

31.2 1-(2,3-Dihydro-1H-inden-4-yl)-1-[1-(triphenylmethyl)imidazol-4-yl]ethanol (530 mg, 1.126 mmol), TFA (2 mL), and Et₃SiH (2 mL) were added to a DCM (6 mL) solution under nitrogen atmosphere, and the mixture was stirred at room temperature overnight. The mixture was concentrated under reduced pressure, and the residue was purified by reversed-phase chromatography under the following conditions: chromatographic column: C18 silica gel; mobile phase: acetonitrile-water (0.1% FA), with a gradient of 20% to 25% within 15 min; detector: UV 200 nm to give 4-(1-(2,3-dihydro-1H-inden-4-yl)vinyl)-1H-imidazole (170 mg) as a colorless oil. (ES, m/z): [M+1]=211.

31.3 4-(1-(2,3-Dihydro-1H-inden-4-yl)vinyl)-1H-imidazole (180 mg, 0.856 mmol) and 10% Pd/C (72.88 mg, 50% w/t) were added to a MeOH (5 mL) solution under hydrogen atmosphere, and the mixture was stirred at room temperature for 3 h. The resulting mixture was filtered through a pad of Celite, and the filter cake was washed with MeOH (3 × 10 mL). The filtrate was concentrated under reduced pressure and purified by reversed-phase chromatography under the following conditions: chromatographic column: C18 silica gel; mobile phase: acetonitrile-water (0.1% FA), with a gradient of 20% to 25% within 10 min; detector: UV 254 nm to give 4-[1-(2,3-dihydro-1H-inden-4-yl)ethyl]-1H-imidazole (97.2 mg) as a yellow oil. (ES,m/z): [M+1]=213. ¹H NMR (400 MHz, Methanol-*d*₄) δ 8.47 (d, *J =* 1.4 Hz, 1H), 7.20 (d, *J =* 1.3 Hz, 1H), 7.16 - 7.00 (m, 2H), 6.87 (dd, *J* = 6.3, 2.4 Hz, 1H), 4.30 (q, *J =* 7.2 Hz, 1H), 2.87 (dq, *J =* 19.2, 7.9 Hz, 4H), 2.06 (p, *J =* 7.4 Hz, 2H), 1.61 (d, *J =* 7.2 Hz, 3H).

4-(1-(2,3-Dihydro-1H-inden-4-yl)ethyl)-1H-imidazole (400 mg) was purified by chiral HPLC under the following conditions (column: (S,S)-WELK-O1, 2 × 25 cm, 5 µm; mobile phase A: Hex (10 mM NH₃-MeOH), mobile phase B: EtOH) to give (R)-4-[1-(2,3-dihydro-1H-inden-4-yl)ethyl]-1H-imidazole (31-a) (103.4 mg) (retention time: 1.14 min) and (S)-4-[1-(2,3-dihydro-1H-inden-4-yl)ethyl]-1H-imidazole (31-b) (89 mg) (retention time: 1.64 min).

(R)-4-[1-(2,3-Dihydro-1H-inden-4-yl)ethyl]-1H-imidazole (31-a) (ES, m/z): [M+1] = 213.

¹H NMR (400 MHz, Chloroform-*d*) *δ* 7.43 - 7.38 (m, 1H), 7.12 - 7.02 (m, 2H), 6.91 - 6.84 (m, 1H), 6.72 (s, 1H), 4.17 (q, *J =* 7.1 Hz, 1H), 2.97 - 2.69 (m, 4H), 2.10 - 1.89 (m, 2H), 1.57 (d, *J* = 7.2 Hz, 3H).

(S)-4-[1-(2,3-Dihydro-1H-inden-4-yl)ethyl]-1H-imidazole (31-b) (ES, m/z): [M+1] = 213.

¹H NMR (400 MHz, Chloroform-*d*) *δ* 9.41 (s, 1H), 7.41 (s, 1H), 7.10 - 7.00 (m, 2H), 6.88 - 6.82 (m, 1H), 6.69 (s, 1H), 4.16 (q, *J =* 7.1 Hz, 1H), 2.95 - 2.68 (m, 4H), 2.08 - 1.86 (m, 2H), 1.56 (d, *J =* 7.2 Hz, 3H).

### Example 32. Preparation of 4-[1-(2-Methyl-2,3-dihydrobenzofuran-7-yl)ethyl]-1H-imidazole

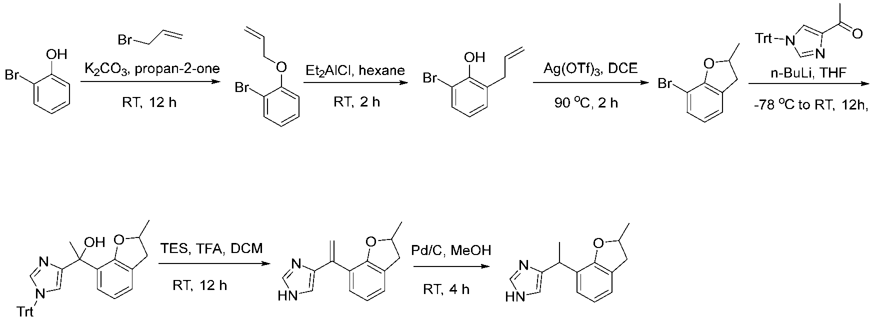

32.1 2-Bromophenol (2.2 g, 12.716 mmol) and K₂CO₃ (3.51 g, 25.432 mmol) were added to an acetone (22 mL) solution under air atmosphere, and the mixture was stirred at room temperature for 1 h. Allyl bromide (2.31 g, 19.074 mmol) was then added portionwise to the mixture described above within 1 min. The resulting mixture was stirred at room temperature overnight. The reaction was quenched by adding water (20 mL), and the mixture was extracted with EtOAc (3 × 50 mL). The combined organic layer was washed with water (1 × 50 mL) and dried over anhydrous Na₂SO₄. After filtration, the filtrate was concentrated under reduced pressure. The residue was purified by silica gel column chromatography and eluted with PE/EA (5:1) to give 1-bromo-2-(prop-2-en-1-oxy)benzene (2 g) as a colorless oil. (ES,m/z): [M] =212, 214.

32.2 A mixture of 1-bromo-2-(prop-2-en-1-oxy)benzene (1 g, 4.693 mmol) and diethylaluminum chloride (0.85 g, 7.039 mmol) in hexane (14 mL) was stirred at room temperature for 2 h under air atmosphere. The reaction was quenched by adding water (20 mL), and the mixture was extracted with EtOAc (3 × 50 mL). The combined organic layer was washed with water (1 × 50 mL) and dried over anhydrous Na₂SO₄. After filtration, the filtrate was concentrated under reduced pressure. The residue was purified by silica gel column chromatography and eluted with PE/EA (5:1) to give 2-bromo-6-(prop-2-en-1-yl)phenol (800 mg) as a yellow solid. (ES,m/z): [M] =212, 214.

32.3 A solution of 2-bromo-6-(prop-2-en-1-yl)phenol (350 mg, 1.643 mmol) and silver trifluoromethanesulfonate (84.41 mg, 0.329 mmol) in dichloroethane (8 mL) was stirred at 90 °C for 2 h under nitrogen atmosphere. The mixture was cooled to room temperature, diluted with water (10 mL), and extracted with ethyl acetate (3 × 20 mL). The combined organic layer was washed with water (1 × 50 mL) and dried over anhydrous Na₂SO₄. After filtration, the filtrate was concentrated under reduced pressure. The residue was purified by reversed-phase chromatography under the following conditions: chromatographic column: C18 silica gel; mobile phase: acetonitrile-water (0.1% NH₃.H₂O), with a gradient of 45% to 50% within 10 min; detector: UV 254 nm to give 7-bromo-2-methyl-2,3-dihydro-1-benzofuran (100 mg) as a colorless oil. (ES,m/z): [M] =212, 214.

32.4 n-BuLi (0.28 mL, 0.704 mmol, 2.5 M in n-hexane) was added dropwise to a solution of 7-bromo-2-methyl-2,3-dihydro-1-benzofuran (100 mg, 0.469 mmol) in THF (4 mL) at -78 °C under nitrogen atmosphere. The mixture was stirred for 1 h, 1-[1-(trityl)imidazol-4-yl]ethanone (181.95 mg, 0.516 mmol) was added dropwise to the mixture described above, and the mixture was transferred to an environment at room temperature and stirred overnight. The reaction was quenched by adding saturated NH₄Cl (20 mL). The resulting mixture was extracted with EtOAc (3 × 20 mL). The combined organic layer was washed with brine (1 × 20 mL) and dried over anhydrous Na₂SO₄. After filtration, the filtrate was concentrated under reduced pressure. The residue was purified by reversed-phase chromatography under the following conditions: chromatographic column: C18 silica gel; mobile phase: acetonitrile-water (10 mmol/L NH₄HCO₃), with a gradient of 50% to 55% within 10 min; detector: UV 254 nm to give 1-(2-methyl-2,3-dihydro-1-benzofuran-7-yl)-1-[1-(trityl)imidazol-4-yl]ethanol (80 mg) as a yellow solid. (ES,m/z): [M+1] =487.

32.5 1-(2-Methyl-2,3-dihydro-1-benzofuran-7-yl)-1-[1-(trityl)imidazol-4-yl]ethanol (80 mg, 0.164 mmol), TFA (1 mL), and Et₃SiH (1 mL) were added to a DCM (2 mL) solution under nitrogen atmosphere, and the mixture was stirred at room temperature for 12 h. The mixture was concentrated under reduced pressure, and the residue was purified by reversed-phase chromatography under the following conditions: chromatographic column: C18 silica gel; mobile phase: acetonitrile-water (0.1% FA), with a gradient of 20% to 25% within 15 min; detector: UV 200 nm to give 4-[1-(2-methyl-2,3-dihydro-1-benzofuran-7-yl)vinyl]-1H-imidazole (20 mg) as a yellow solid. (ES,m/z): [M+1] =227.

32.6 A solution of 4-[1-(2-methyl-2,3-dihydro-1-benzofuran-7-yl)vinyl]-1H-imidazole (20 mg, 0.088 mmol) and 10% Pd/C (7.52 mg) in MeOH (2 mL) was stirred at room temperature for 4 h under hydrogen atmosphere. The resulting mixture was filtered through a pad of Celite, and the filter cake was washed with MeOH (1 × 10 mL). The filtrate was concentrated under reduced pressure and purified by reversed-phase chromatography under the following conditions: chromatographic column: C18 silica gel; mobile phase: acetonitrile-water (0.1% NH₃.H₂O), with a gradient of 20% to 25% within 10 min; detector: UV 254 nm to give 4-[1-(2-methyl-2,3-dihydrobenzofuran-7-yl)ethyl]-1H-imidazole (14.4 mg) as a colorless oil. (ES,m/z): [M+1]=229. ¹H NMR (400 MHz, Chloroform-*d*) *δ* 7.75 (s, 1H), 7.04 - 6.97 (m, 1H), 6.90 - 6.83 (m, 1H), 6.81 (s, 1H), 6.74 (t, *J =* 7.4 Hz, 1H), 4.97 - 4.81 (m, 1H), 4.29 (p, *J =* 7.0 Hz, 1H), 3.34 - 3.22 (m, 1H), 2.83 - 2.73 (m, 1H), 1.63 - 1.56 (m, 3H), 1.45 - 1.38 (m, 3H).

### Example 33. Preparation of 4-[1-(2H-Thiochroman-8-yl)ethyl]-1H-imidazole

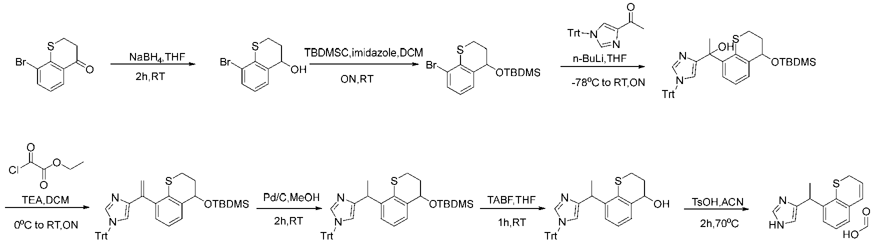

33.1 A solution of 8-bromo-2,3-dihydro-1-thiochroman-4-one (2 g, 8.226 mmol) and NaBH₄ (1.8 g, 49.356 mmol) in THF (30 mL) was stirred at room temperature for 2 h under air atmosphere. The reaction was quenched by adding water. The resulting mixture was extracted with EtOAc (3 × 100 mL). The combined organic layer was washed with water (3 × 100 mL) and dried over anhydrous Na₂SO₄. After filtration, the filtrate was concentrated under reduced pressure. The residue was purified by silica gel column chromatography and eluted with PE/EA (1:1) to give 8-bromo-3,4-dihydro-2H-1-thiochroman-4-ol (1.9 g) as a colorless oil. (ES,m/z): [M-18]=227, 229.

33.2 tert-Butyldimethylsilyl chloride (1.3 g, 8.812 mmol) was added portionwise to a mixture of 8-bromo-3,4-dihydro-2H-1-thiochroman-4-ol (1.8 g, 7.343 mmol) and imidazole (1.0 g, 14.686 mmol) in DCM (40 mL) at room temperature under air atmosphere. The resulting mixture was stirred overnight. The reaction was quenched by adding water (10 mL). The resulting mixture was extracted with EtOAc (3 × 100 mL). The combined organic layer was washed with water (3 × 100 mL) and dried over anhydrous Na₂SO₄. After filtration, the filtrate was concentrated under reduced pressure. The residue was purified by silica gel column chromatography and eluted with PE/EA (1:1) to give [(8-bromo-3,4-dihydro-2H-1-thiochroman-4-yl)oxy](tert-butyl)dimethylsilane (2.2 g) as a colorless oil.

33.3 n-BuLi (0.7 g, 11.687 mmol, 2.5 M in n-hexane) was added dropwise to a solution of [(8-bromo-3,4-dihydro-2H-1-thiochroman-4-yl)oxy](tert-butyl)dimethylsilane (2.8 g, 7.791 mmol) in THF (40 mL) at -78 °C under nitrogen atmosphere. The mixture was stirred for 1 h, a solution of 1-[1-(trityl)imidazol-4-yl]ethanone (2.8 g, 7.791 mmol) in THF (10 mL) was added dropwise to the mixture described above, and the mixture was transferred to an environment at room temperature and stirred overnight. The reaction was quenched by adding saturated NH₄Cl. The resulting mixture was extracted with EtOAc (3 × 100 mL). The combined organic layer was washed with water (3 × 100 mL) and dried over anhydrous Na₂SO₄. After filtration, the filtrate was concentrated under reduced pressure to give 1-(4-[(tert-butyldimethylsilyl)oxy]-3,4-dihydro-2H-1-thiochroman-8-yl)-1-[1-(trityl)imidazol-4-yl]ethanol (3.6 g) as a colorless oil. (ES,m/z): [M+1]=634.

33.4 Ethyl oxalyl chloride (3.06 g, 22.443 mmol) was added dropwise to a solution of 1-(4-[(tert-butyldimethylsilyl)oxy]-3,4-dihydro-2H-1-thiochroman-8-yl)-1-[1-(trityl)imidazol-4-yl]ethanol (4.6 g, 7.481 mmol) and TEA (4.54 g, 44.886 mmol) in DCM (400 mL) at 0 °C under air atmosphere. The resulting mixture was stirred at room temperature overnight. The mixture was concentrated under reduced pressure, and the residue was purified by Prep-TLC (PE/EA 8:1) to give 4-(1-(4-[(tert-butyldimethylsilyl)oxy]-3,4-dihydro-2H-1-thiochroman-8-yl)vinyl)-1-(trityl)imidazole (3.5 g) as a yellow oil. (ES,m/z): [M+1]=615.

33.5 A solution of 4-(1-(4-[(tert-butyldimethylsilyl)oxy]-3,4-dihydro-2H-1-thiochroman-8-yl)vinyl)-1-(trityl)imidazole (1.5 g, 2.439 mmol) and 10% Pd/C (52 mg) in MeOH (30 mL) was stirred at room temperature for 2 h under hydrogen atmosphere. The resulting mixture was filtered through a pad of Celite, and the filter cake was washed with MeOH (3 × 5 mL). The filtrate was concentrated under reduced pressure and purified by reversed-phase chromatography under the following conditions: chromatographic column: C18 silica gel; mobile phase: acetonitrile-water (0.1% NH₃.H₂O + 10 mmol/L NH₄HCO₃), with a gradient of 10% to 40% within 10 min; detector: UV 254 nm to give 4-(1-(4-((tert-butyldimethylsilyl)oxy)thiochroman-8-yl)ethyl)-1-trityl-1H-imidazole (220 mg) as a colorless oil. (ES,m/z): [M+1]=617.

33.6 A solution of 4-(1-(4-((tert-butyldimethylsilyl)oxy)thiochroman-8-yl)ethyl)-1-trityl-1H-imidazole (212 mg, 0.344 mmol) and TBAF (269.5 mg, 1.032 mmol) in THF (4 mL) was stirred at room temperature for 1 h under nitrogen atmosphere. The filtrate was concentrated under reduced pressure and purified by reversed-phase chromatography under the following conditions: chromatographic column: C18 silica gel; mobile phase: acetonitrile-water (10 mmol/L NH₄HCO₃), with a gradient of 10% to 50% within 10 min; detector: UV 254 nm to give 8-(1-[1-(triphenylmethyl)imidazol-4-yl]ethyl-3,4-dihydro-2H-1-benzothiopyran-4-ol (160 mg) as a yellow oil. (ES,m/z): [M+1]=504.

33.7 A solution of 8-(1-[1-(triphenylmethyl)imidazol-4-yl]ethyl-3,4-dihydro-2H-1-benzothiopyran-4-ol (100 mg, 0.199 mmol) and TsOH (7.4 mg, 0.043 mmol) in acetonitrile (2 mL) was stirred at 70 °C for 2 h under nitrogen atmosphere. The filtrate was concentrated under reduced pressure and purified by reversed-phase chromatography under the following conditions: chromatographic column: C18 silica gel; mobile phase: acetonitrile-water (10 mmol/L NH₄HCO₃), with a gradient of 10% to 50% within 10 min; detector: UV 254 nm to give 4-[1-(2H-thiochroman-8-yl)ethyl]-1H-imidazole formate (3.5 mg). (ES,m/z):[M+1]=243. ¹H NMR (400 MHz, Chloroform-*d*) δ 8.43 (s, 1H), 7.72 (d, *J =* 10.2 Hz, 1H), 7.01 (t, *J =* 7.5 Hz, 1H), 6.98 - 6.89 (m, 2H), 6.79 (s, 1H), 6.48 (d, *J =* 10.1 Hz, 1H), 5.94 (dt, *J =* 10.0, 5.0 Hz, 1H), 4.55 (q, *J =* 7.0 Hz, 1H), 3.44 - 3.28 (m, 2H), 1.58 (d, *J =* 7.1 Hz, 3H).

### Example 34: Preparation of 4-[1-(5-Fluoro-2,3-dihydro-1H-inden-4-yl)ethyl]-1H-imidazole

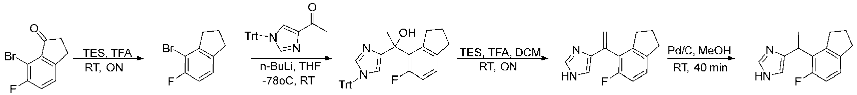

34.1 Triethylsilane (888.4 mg, 7.640 mmol) was added to a solution of 7-bromo-6-fluoro-2,3-dihydroinden-1-one (700 mg, 3.056 mmol) in trifluoroacetic acid (3 mL) at room temperature. The resulting mixture was stirred at room temperature overnight under nitrogen atmosphere. The resulting mixture was concentrated under reduced pressure. The residue was purified by silica gel column chromatography and eluted with PE/EA (5:1) to give 4-bromo-5-fluoro-2,3-dihydro-1H-indene (320 mg) as a colorless oil. (ES,m/z): [M]=214, 216.

34.2 n-BuLi (1.06 mL, 2.651 mmol, 2.5 M in n-hexane) was added dropwise to a solution of 4-bromo-5-fluoro-2,3-dihydro-1H-indene (380 mg, 1.767 mmol) in tetrahydrofuran (6 mL) at -78 °C under nitrogen atmosphere. The mixture was reacted at -78 °C for 40 min, and then a solution of 1-[1-(trityl)imidazol-4-yl]ethanone (622.7 mg, 1.767 mmol) in tetrahydrofuran (4 mL) was added dropwise. After the dropwise addition was completed, the mixture was warmed to room temperature and stirred overnight. The reaction was quenched by adding a saturated aqueous NH₄Cl solution (20 mL) at room temperature. The mixture was extracted with ethyl acetate (3 × 20 mL). The combined organic layer was washed with water (1 × 20 mL) and dried over anhydrous Na₂SO₄. After filtration, the filtrate was concentrated under reduced pressure. The residue was purified by silica gel column chromatography and eluted with PE/EA (5:1) to give 1-(5-fluoro-2,3-dihydro-1H-inden-4-yl)-1-[1-(trityl)imidazol-4-yl]ethanol (350 mg) as a white solid. (ES,m/z): [M+1]=489.

34.3 Trifluoroacetic acid (1.4 g, 12.280 mmol) was added to a solution of 1-(5-fluoro-2,3-dihydro-1H-inden-4-yl)-1-[1-(trityl)imidazol-4-yl]ethanol (300 mg, 0.614 mmol) in dichloromethane (5 mL) at room temperature under nitrogen atmosphere. After 1 min of the reaction, triethylsilane (1.427 g, 12.280 mmol) was added dropwise. The resulting mixture was stirred overnight. The resulting mixture was concentrated to dryness by rotary evaporation. The residue was purified by reversed-phase flash chromatography under the following conditions: column: C18 silica gel; mobile phase: acetonitrile in water (10 mmol/L NH₄HCO₃), with a gradient of 10% to 50% within 10 min; detector: UV 220 nm to give 4-[1-(5-fluoro-2,3-dihydro-1H-inden-4-yl)vinyl]-1H-imidazole (60 mg) as a colorless oil. (ES,m/z): [M+1]=229.

34.4 10% Pd/C (20 mg) was added to a solution of 4-[1-(5-fluoro-2,3-dihydro-1H-inden-4-yl)vinyl]-1H-imidazole (60 mg, 0.263 mmol) in methanol (5 mL) at room temperature. The reaction solution was stirred at room temperature for 40 min under hydrogen atmosphere and then filtered through a pad of celite, and the filter cake was washed with methanol (3 × 10 mL). The filtrate was concentrated under reduced pressure. The residue was purified by reversed-phase flash chromatography under the following conditions: column: C18 silica gel; mobile phase: aqueous acetonitrile solution (10 mmol/L NH₄HCO₃), with a gradient of 10% to 50% within 10 min; detector: UV 220 nm to give 4-[1-(5-fluoro-2,3-dihydro-1H-inden-4-yl)ethyl]-1H-imidazole (32 mg). (ES,m/z): [M+1]=231. ¹H NMR (400 MHz, Methanol-*d*₄) δ 7.54 (s, 1H), 7.02 (dd, *J =* 8.2, 4.8 Hz, 1H), 6.86 - 6.72 (m, 2H), 4.38 (q, *J =* 7.3 Hz, 1H), 2.84 (dt, *J =* 26.1, 7.6 Hz, 3H), 2.53 (dt, *J =* 15.6, 7.4 Hz, 1H), 2.12 - 1.84 (m, 2H), 1.61 (d, *J =* 7.3 Hz, 3H).

### Example 37. Preparation of 7-[1-(1H-Imidazol-4-yl)ethyl]-5-fluoro-2,3-dihydro-1H-inden-1-ol

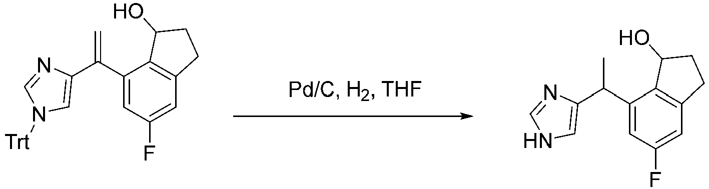

37.1 Pd/C (10%, 20 mg) was added to a solution of 5-fluoro-7-{1-[1-(trityl)imidazol-4-yl]vinyl}-2,3-dihydro-1H-inden-1-ol (100 mg, 0.20 mmol) in THF (1 mL) at room temperature. The reaction solution was purged with hydrogen and hydrogenated at room temperature overnight. The resulting mixture was filtered, and the filter cake was washed with dichloromethane. The filtrate was concentrated under reduced pressure. The residue was purified by silica gel column chromatography and eluted with PE/EA (1:1) to give 7-[1-(1H-imidazol-4-yl)ethyl]-5-fluoro-2,3-dihydro-1H-inden-1-ol (26.6 mg). (ES, m/z): [M+1]=247.1. ¹H NMR (400 MHz, DMSO-*d₆*) *δ* 11.78 (d, *J* = 22.4 Hz, 1H), 7.57 - 7.41 (m, 1H), 6.93 - 6.61 (m, 3H), 5.33 - 5.08 (m, 2H), 4.68 - 4.37 (m, 1H), 3.03 (dt, *J =* 16.0, 8.0 Hz, 1H), 2.77 - 2.64 (m, 1H), 2.36 - 2.16 (m, 1H), 1.99 - 1.84 (m, 1H), 1.52 (dd, *J =* 8.4, 7.2 Hz, 3H).

### Example 38. Preparation of 7-[1-(1H-Imidazol-4-yl)ethyl]-5-fluoro-2,3-dihydro-1H-inden-1-one

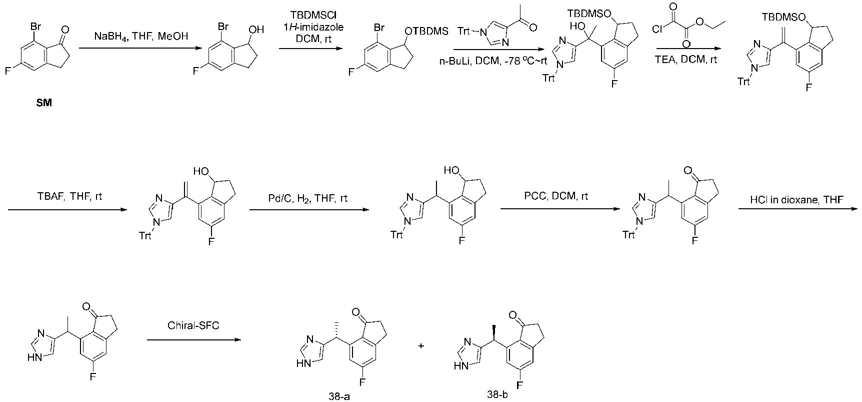

38.1 NaBH₄ (33.05 g, 873.18 mmol) was added portionwise to a mixed solution of 7-bromo-5-fluoro-2,3-dihydroinden-1-one (50.00 g, 218.25 mmol) in THF (500 mL) and MeOH (250 mL) at 0 °C under nitrogen atmosphere. The resulting mixture was stirred at 25 °C for 1 h under nitrogen atmosphere. The resulting mixture was extracted with dichloromethane (2 × 1000 mL). The combined organic layer was washed with brine and dried over anhydrous Na₂SO₄. After filtration, the filtrate was concentrated under reduced pressure to give 7-bromo-5-fluoro-2,3-dihydro-1H-inden-1-ol (53.60 g) as a yellow oil. (ES,m/z): [M]=213.

38.2 tert-Butyldimethylsilyl chloride (42.56 g, 282.47 mmol) was added to a mixture of 7-bromo-5-fluoro-2,3-dihydro-1H-inden-1-ol (53.60 g, 231.95 mmol) and imidazole (31.58 g, 463.90 mmol) in dichloromethane (800 mL) at room temperature. The resulting mixture was stirred at room temperature for 1 h under air atmosphere. The resulting mixture was extracted with EtOAc (2 × 1000 mL). The combined organic layer was washed with brine and dried over anhydrous Na₂SO₄. After filtration, the filtrate was concentrated under reduced pressure. This resulted in [(7-bromo-5-fluoro-2,3-dihydro-1H-inden-1-yl)oxy](tert-butyl)dimethylsilane (62 g) as a yellow oil. (ES, m/z): [M]=345.

38.3 n-BuLi (108 mL, 269.67 mmol, 2.5 M in n-hexane) was added dropwise to a solution of [(7-bromo-5-fluoro-2,3-dihydro-1H-inden-1-yl)oxy](tert-butyl)dimethylsilane (62.00 g, 179.74 mmol) in THF (600 mL) at -78 °C under nitrogen atmosphere. After 40 min, 1-[1-(trityl)imidazol-4-yl]ethanone (63.36 g, 179.75 mmol, 1.00 equiv) in tetrahydrofuran (200 mL) was added dropwise. The mixture was warmed to 25 °C and stirred overnight under nitrogen atmosphere. The reaction was quenched with a saturated aqueous NH₄Cl solution (1000 mL) at 25 °C. The mixture was extracted with dichloromethane (3 × 1000 mL). The combined organic layer was washed with brine and dried over anhydrous Na₂SO₄. After filtration, the filtrate was concentrated under reduced pressure. The residue was purified by silica gel column chromatography and eluted with PE/EA (1:1) to give 1-{3-[(tert-butyldimethylsilyl)oxy]-6-fluoro-2,3-dihydro-1H-inden-4-yl}-1-[1-(trityl)imidazol-4-yl]ethanol (68.00 g) as a yellow oil. (ES, m/z): [M+1] =619.

38.4 Ethyl chlorooxoacetate (60.01 g, 439.45 mmol) was added to a solution of 1-{3-[(tert-butyldimethylsilyl)oxy]-6-fluoro-2,3-dihydro-1H-inden-4-yl}-1-[1-(trityl)imidazol-4-yl]ethanol (68 g, 109.82 mmol) and triethylamine (88.91 g, 878.98 mmol) in dichloromethane (800 mL) at 0 °C under nitrogen atmosphere. The mixture was warmed to room temperature, stirred for 1 h, and extracted with dichloromethane (3 × 1000 mL). The combined organic layer was washed with brine and dried over anhydrous Na₂SO₄. After filtration, the filtrate was concentrated under reduced pressure. The residue was purified by silica gel column chromatography and eluted with PE/EA (5:1) to give 4-(1-{3-[(tert-butyldimethylsilyl)oxy]-6-fluoro-2,3-dihydro-1H-inden-4-yl}vinyl)-1-(trityl)imidazole (62.00 g) as a yellow solid. (ES, m/z): [M+1] =601.

38.5 TBAF (97.65 g, 309.50 mmol, 3 equiv) was added to a solution of 4-(1-{3-[(tert-butyldimethylsilyl)oxy]-6-fluoro-2,3-dihydro-1H-inden-4-yl}vinyl)-1-(trityl)imidazole (62 g, 103.16 mmol, 1 equiv) in THF (800 mL) at room temperature. The mixture was stirred for 2h and then extracted with dichloromethane (2 × 1000 mL). The combined organic layer was washed with brine and dried over anhydrous Na₂SO₄. After filtration, the filtrate was concentrated under reduced pressure to give 5-fluoro-7-{1-[1-(trityl)imidazol-4-yl]vinyl}-2,3-dihydro-1H-inden-1-ol (65.00 g) as a yellow solid. (ES, m/z): [M+1] =487.

38.6 Pd/C (10%, 13.00 g) was added to a solution of 5-fluoro-7-{1-[1-(trityl)imidazol-4-yl]vinyl}-2,3-dihydro-1H-inden-1-ol (65.00 g, 133.58 mmol) in THF (1000 mL) under nitrogen atmosphere. The mixture was hydrogenated at room temperature overnight under hydrogen atmosphere. The reaction solution was filtered, and the filter cake was washed with dichloromethane. The filtrate was concentrated under reduced pressure. The residue was purified by silica gel column chromatography and eluted with PE/EA (1:1) to give 5-fluoro-7-{1-[1-(trityl)imidazol-4-yl]ethyl}-2,3-dihydro-1H-inden-1-ol (46.00 g) as a grayish-white solid. (ES, m/z): [M+1] =489.

38.7 Pyridinium chlorochromate (PCC) (30.43 g, 141.22 mmol) was added to a solution of 5-fluoro-7-{1-[1-(trityl)imidazol-4-yl]ethyl}-2,3-dihydro-1H-inden-1-ol (46.00 g, 94.14 mmol) in DCM (600 mL) at room temperature under nitrogen atmosphere. After the reaction was completed, the reaction solution was extracted with dichloromethane (2 × 1000 mL). The combined organic layer was washed with brine and dried over anhydrous Na₂SO₄. After filtration, the filtrate was concentrated under reduced pressure to give 5-fluoro-7-{1-[1-(trityl)imidazol-4-yl]ethyl}-2,3-dihydroinden-1-one (52.00 g) as a yellow oil. (ES, m/z): [M+1] =487.

38.8 A solution of hydrochloric acid in dioxane was added to a solution of 5-fluoro-7-{1-[1-(trityl)imidazol-4-yl]ethyl}-2,3-dihydroinden-1-one (52.00 g, 106.7 mmol) in THF (300 mL) at room temperature under air atmosphere. After the mixture was reacted at room temperature for 2 h, the residue was purified by reversed-phase flash chromatography under the following conditions: column: C18 silica gel; mobile phase: aqueous acetonitrile, with a gradient of 10% to 50% within 40 min; detector: UV 254 nm to give 7-[1-(1H-imidazol-4-yl)ethyl]-5-fluoro-2,3-dihydro-1H-inden-1-one (10.00 g). (ES, m/z): [M+1] =245. ¹H NMR (400 MHz, DMSO-*d₆*) *δ* 11.83 (s, 1H), 7.54 (d, *J* = 10.6 Hz, 1H), 7.28 - 7.12 (m, 1H), 6.97 - 6.68 (m, 2H), 5.48 - 5.24 (m, 1H), 3.10 - 3.03 (m, 2H), 2.71 - 2.64 (m, 2H), 1.51 - 1.41 (m, 3H).

38.9 7-[1-(1H-Imidazol-4-yl)ethyl]-5-fluoro-2,3-dihydro-1H-inden-1-one (60 mg) was purified by Prep Chiral SFC under the following conditions (column: CHIRALPAK IG, 3 × 25 cm, 5 µm; mobile phase A: CO₂, mobile phase B: MEOH (0.1 % 2 M NH₃-MEOH)) to give (R)-7-(1-(1H-imidazol-4-yl)ethyl)-5-fluoro-2,3-dihydro-1H-inden-1-one (38-a, 22.2 mg) (retention time: 0.96 min) and (S)-7-(1-(1H-imidazol-4-yl)ethyl)-5-fluoro-2,3-dihydro-1H-inden-1-one (38-b, 13.7 mg) (retention time: 0.87 min).

(R)-7-(1-(1H-Imidazol-4-yl)ethyl)-5-fluoro-2,3-dihydro-1H-inden-1-one (38-a)

(ES, m/z): [M+1] =245. ¹H NMR (400 MHz, Methanol-*d4*) *δ* 7.82 (d, *J =* 1.2 Hz, 1H), 7.11 (d, *J =* 8.4 Hz, 1H), 7.02 (t, *J =* 1.2 Hz, 1H), 6.81 (dd, *J =* 10.4, 2.4 Hz, 1H), 5.48 (q, *J =* 7.2 Hz, 1H), 3.21 - 3.11 (m, 2H), 2.77 - 2.70 (m, 2H), 1.58 (d, *J =* 7.2 Hz, 3H). (S)-7-(1-(1H-Imidazol-4-yl)ethyl)-5-fluoro-2,3-dihydro-1H-inden-1-one (38-b)

(ES, m/z): [M+1] =245. ¹H NMR (400 MHz, Methanol-*d4*) *δ* 8.05 (d, *J =* 1.2 Hz, 1H), 7.17 - 7.09 (m, 2H), 6.84 (dd, *J =* 10.4, 2.4 Hz, 1H), 5.51 (q, *J =* 7.2 Hz, 1H), 3.16 (t, *J* = 6.0 Hz, 2H), 2.78 - 2.68 (m, 2H), 1.60 (d, *J =* 7.2 Hz, 3H).

### Biological Test Example

### Test Example 1. In Vitro Affinity

### 1. Experimental method

### 1.1 Preparation of buffer and test sample

Standard buffer (used for preparing α_{1A}, α_{2A}, α_{2B}, and α_{2C} receptor membranes): 146 mg of EDTA was weighed out, a 50 mM Tris-HCl buffer was added to reach a total volume of 1000 mL, and the pH was adjusted to 7.7 to make a final concentration of 0.5 mM for EDTA.

Test sample (compound): The theoretical sample weighing amount was calculated according to the designed concentration and the required volume. Generally, the test sample was dissolved in DMSO to an initial preparation dose of 5.0 × 10⁻³ M, and the solution was sequentially diluted with DMSO to 5.0 × 10⁻⁴ M-5.0 × 10⁻⁹ M; the diluted DMSO solution was diluted with Buffer to a working concentration of 5.0 × 10⁻⁵ M-5.0 × 10⁻¹¹ M; and the final concentration of DMSO in the working solution was 1% (the final concentration of DMSO in the reaction system was 0.2%). The test sample was prepared and then stored at 4 °C. After the test was completed, the test sample was discarded.

### 1.2 Preparation of receptor membranes

CHO-K1-α_{1A}, CHO-K1-α_{2A}, CHO-K1-α_{2B}, and CHO-K1-α_{2C} cells were taken out of a refrigerator at -80 °C, thawed naturally, and centrifuged at 2000 g at 4 °C for 10 min. The supernatant was discarded, and the pellet was collected. A buffer was added to the pellet, and the mixture was homogenized for 20-30 s and then centrifuged at 48000 g at 4 °C for 25 min. The supernatant was discarded, and the buffer was added again. The mixture was homogenized for 20-30 s and centrifuged at 48000 g at 4 °C for 25 min. The supernatant was discarded, and the pellet was collected and stored at -80 °C.

### 1.3 Competitive receptor binding assay

### 1.3.1 Experimental conditions for radioactive binding

**Table 1. Experimental conditions for radioactive binding**

| Receptor name | Radioligand | Non-labeled | Positive drug | Incubation condition | Receptor membrane concentration (mg/mL) |
|---|---|---|---|---|---|
| α_{1A} | ³H-Prazosin | Prazosin | Risperidone | | 2.5 |
| α_{2A} | ³H-RX821002 | Yohimbine | Yohimbine | Cell 25 °C | 5.0 |
| α_{2B} | ³H-RX821002 | Yohimbine | Yohimbine | 90 min | 10.0 |
| α_{2C} | ³H-RX821002 | Yohimbine | Yohimbine | | 5.0 |

| | | | | | |
|---|---|---|---|---|---|
| Note: Prazosin: prazosin; RX821002: 2-methoxyidazoxan monohydrochloride. | | | | | |

### 1.3.2 Procedures of receptor binding assay

Step 1: 50 µL of a vehicle (1% DMSO) was added to a total binding tube (TB), 50 µL of a non-labeled substance (with a final concentration of 1.0 × 10⁻⁵ M) was added to a non-specific binding tube (NB), and 50 µL of a test compound was added to each test compound tube (CB).

Step 2: 100 µL of a buffer was added to each reaction tube.

Step 3: The prepared membrane was suspended in a buffer to give a membrane suspension with a corresponding concentration for later use.

Step 4: 50 µL of the corresponding radioligand was added to each reaction tube.

Step 5: 50 µL of the membrane suspension was added to each reaction tube.

Step 6: Each reaction tube was incubated according to the incubation conditions. After the reaction was completed, the binding ligands were rapidly filtered under reduced pressure. The UniFilter GF/C plate was saturated with a 0.5% PEI solution 1 h in advance, fully washed with an ice-cold Tris-HCl buffer, filtered under vacuum, then placed into a thermostatic dryer, and dried for 30 min. The filter plate was taken out, and a MICROSCINT PS scintillation solution was added at 40 µL/well.

Step 7: The scintillation vial was put into a liquid scintillation counter for counting.

### 1.4 Data analysis

Based on the effect values of the compound samples at different concentration test points, the GraphPad Prism software was used to fit the action curves of the compound samples to the receptors, and the Ki values were calculated.

### 1.5 Experimental results

By way of example, the affinity of compounds 1, 3, 4, and 8 of the present disclosure for the α_{2A} receptor was comparable to that of dexmedetomidine hydrochloride, and the affinity of compounds 2 and 10 for the α_{2A} receptor is superior to that of dexmedetomidine hydrochloride; the affinity α_{1A/2A} of compound 3 is comparable to that of dexmedetomidine hydrochloride, and the affinity α_{1A/2A} of compounds 4, 8, and 10 is superior to that of dexmedetomidine hydrochloride. See Table 2 for details.

### Test Example 2. In Vitro Function

### 2. Experimental method

2.1 Objective: To evaluate the agonistic effects of dexmedetomidine hydrochloride series compounds on adrenoceptors (α_{1A}, α_{2A}, α_{2B}, and α_{2C}) by detecting changes in cAMP and IP1 concentrations in the signaling pathways of the receptors using Cisbio HTRF cAMP-Gi and IP-One kits with a microplate reader, and calculating the EC₅₀ values of the compounds.

### 2.2 Experimental materials:

Cell line:
   293-α_{1A} stably transfected cell strain (Shanghai Shujing Biopharma Co., Ltd.)
   293-α_{2A} stably transfected cell strain (Shanghai Shujing Biopharma Co., Ltd.)
   CHO-α_{2B}/Gα15 stably transfected cell strain (Nanjing GenScript Biotech Co., Ltd.)
   CHO-α_{2C}/Gα15 stably transfected cell strain (Nanjing GenScript Biotech Co., Ltd.)
Cell culture conditions:
   293-α_{1A} (DMEM, 10% FBS, 0.6 µg/mL puromycin)
   293-α_{2A} (DMEM, 10% FBS, 0.6 µg/mL puromycin)
   CHO-α_{2B}/Gα15 (F12, 10% FBS, 200 µg/mL Zeocin, 100 µg/mL Hygromycin B)
   CHO-α_{2C}/Gα15 (F12, 10% FBS, 400 µg/mL G418, 400 µg/mL Hygromycin B)
Reagents and consumables:
   F12 (meilunbio, MA0229)
   DMEM (Gibco, 8121703)
   FBS (BOVOGEN, SFBS)
   Zeocin (invitrogen, R25001)
   Hygromycin B (invitrogen, 10687010)
   Puromycin (Solarbio, 119L042)
   PBS (meilunbio, MA0015)
   Trypsin (Gibco, 25200-072)
   96 cell plate (cisbio, 66PL96025)
   cAMP-Gi kit (cisbio, 03E)
   IP-ONE Kit (cisbio, 16E)
   CO₂ incubator (Thermo, 311)
   Centrifuge (Shanghai Anting Scientific Instrument Factory, TGL-16C)
   Cell counter (Countstar, IC1000)
   Microplate reader (PerkinElmer, EnVision)

### 2.3 Experimental procedures

### 2.3.1 Agonist screening mode cAMP assay (α_{2A} adrenoceptor)

(1) Preparation of reaction buffer (1× Stimulation buffer) required for experiment: The 5× Stimulation buffer in the Cisbio cAMP-Gi kit was diluted with ddH₂O at a ratio of 1:4 for later use.
(2) Preparation of compound: The compound was diluted with DMSO to give a 5 mM stock solution, followed by a 3.16-fold dilution to give 10 gradients, and then the prepared compound was diluted with the Stimulation buffer to the corresponding concentrations (2.5×) for later use.
(3) Preparation of cells: 293-α_{2A} cells in the culture dish were digested with trypsin, eluted with a culture medium, and collected into a 5 mL centrifuge tube. The cells were centrifuged at 1000 rpm for 5 min, and the supernatant was discarded. 3 mL of PBS was added, and the mixture was gently pipetted to be mixed well. The cells were centrifuged again at 1000 rpm for 5 min, and the supernatant was discarded. The cells were resuspended in the 1× Stimulation buffer and counted using a Countstar cell counter, and the cell density was adjusted to 4 × 10⁵ cells/mL for later use.
(4) Cell addition: The cell suspension was added to assay plates at 5 µL/well (i.e., about 2000 cells/well).
(5) Compound addition: The compound diluted with the Stimulation buffer was added to the assay plates described above at 4 µL/well.
(6) Reaction and incubation: After gentle shaking, the assay plates were incubated at 37 °C for 20 min.
(7) Agonist Forskolin addition: A 10× adenylate cyclase agonist (Forskolin with a final concentration of 1 µM) solution was added at 1 µL/well.
(8) Reaction and incubation: After gentle shaking, the assay plates were incubated at 37 °C for 45 min.
(9) Detection reagent addition: cAMP-cryptate and Anti-cAMP-d2 were separately diluted according to a ratio of 1:20 with Lysis & detection buffer in the Cisbio cAMP-Gi detection kit, and the diluted cAMP-cryptate and Anti-cAMP-d2 described above were separately added to the assay plate at 5 µL/well. After shaking, the assay plates were left to stand at room temperature for 60 min.
(10) Experimental readings: The plate was read on Envision, readings from the 665 nm and 615 nm channels were taken, and the ratio of 665 nm/615 nm readings was calculated.

### 2.3.2 Agonist screening mode IP-One assay (α_{1A}, α_{2B}, and α_{2C} adrenoceptors)

(1) Preparation of reaction buffer (1× Stimulation buffer) required for experiment: The 5× Stimulation buffer in the Cisbio IP-One kit was diluted with ddH₂O at a ratio of 1:4 for later use.
(2) Preparation of compound: The compound was diluted with DMSO to give a 5 mM stock solution, followed by a 3.16-fold dilution to give 10 gradients, and then the prepared compound was diluted with the Stimulation buffer to the corresponding concentrations (2×) for later use.
(3) Preparation of cells: 293-α_{1A}, CHO-α_{2B}, and CHO-α_{2C} cells in the culture dish were digested with trypsin, eluted with a culture medium, and collected into a 5 mL centrifuge tube. The cells were centrifuged at 1000 rpm for 5 min, and the supernatant was discarded. 3 mL of PBS was added, and the mixture was gently pipetted to be mixed well. The cells were centrifuged again at 1000 rpm for 5 min, and the supernatant was discarded. The cells were resuspended in the 1× Stimulation buffer and counted using a Countstar cell counter, and the cell density was adjusted to 1.7 × 10⁶ cells/mL for later use.
(4) Cell addition: The cell suspension was added to assay plates at 7 µL/well (i.e., about 12000 cells/well).
(5) Compound addition: The compound diluted with the Stimulation buffer was added to the assay plates described above at 7 µL/well.
(6) Reaction and incubation: After gentle shaking, the assay plates were incubated at 37 °C for 60 min.
(7) Detection reagent addition: IP1-cryptate and Anti-IP1-d2 were separately diluted according to a ratio of 1:12 with Lysis & detection buffer in the Cisbio IP-One detection kit, and the diluted IP1-cryptate and Anti-IP1-d2 described above were separately added to the assay plate at 3 µL/well. After shaking, the assay plates were left to stand at room temperature for 60 min.
(8) Experimental readings: The plate was read on Envision, readings from the 665 nm and 615 nm channels were taken, and the ratio of 665 nm/615 nm readings was calculated.

### 2.4 Data analysis

Based on the agonistic effect values of the compound samples at different concentration test points, data analysis and statistics (log(agonist) vs. normalized response -- Variable slope, n ≥ 2) were performed using the plotting software GraphPad Prism5, and EC₅₀ was calculated.

### 2.5 Experimental results

By way of example, the agonistic activity of compounds 1, 3, 4, 10, 11-b, and 31-b of the present disclosure on the α_{2A} receptor was comparable to that of dexmedetomidine hydrochloride, and the agonistic activity of compounds 2 and 8 on the α_{2A} receptor was superior to that of dexmedetomidine hydrochloride; the agonistic activity α_{1A/2A} of compounds 2, 4, 10, 11-b, 17, and 31-b was comparable to that of dexmedetomidine hydrochloride, and the agonistic activity α_{1A/2A} of compounds 3 and 8 was superior to that of dexmedetomidine hydrochloride. See Table 2 for details.

**Table 2. In vitro affinity and functional data**

| **Serial number** | **Binding (nM)** | | | | | **Function (nM)** | | | | |
|---|---|---|---|---|---|---|---|---|---|---|
| | **α_{1A/2A}** | **α_{1A}** | **α_{2A}** | **α_{2B}** | **α_{2C}** | **α_{1A/2A}** | **α_{1A}** | **α_{2A}** | **α_{2B}** | **α_{2C}** |
| Dexmedetomidine hydrochloride | 35.1 | 592.80 | 16.90 | 3.66 | 7.34 | 6.1 | 9.55 | 1.58 | 0.96 | 2.05 |
| 1 | 4.3 | 67.90 | 15.62^{b} | 59.08 | 29.31 | 2.5 | 3.16 | 1.28^{b} | 4.09 | 15.35 |
| 2 | 11.8 | 91.23 | 7.72^{a} | 11.68 | 13.81 | 10.1^{b} | 5.32 | 0.53^{a} | 3.03 | 18.42 |
| 3 | 39.0^{b} | 1134.85 | 29.12^{b} | 62.26 | 26.11 | 18.3^{a} | 57.01 | 3.12^{b} | 5.51 | 191.3 |
| 4 | 71.4^{a} | 2048.50 | 28.70^{b} | 64.61 | 46.07 | 7.0^{b} | 25.09 | 3.57^{b} | 54.43 | 278 |
| 8 | 104.4^{a} | 1131.50 | 10.84^{b} | 37.66 | 18.89 | 23.9^{a} | 21.75 | 0.91^{a} | 17.7 | 9.89 |
| 10 | 318.5^{a} | 765.95 | 2.40^{a} | 6.053 | 6.445 | 5.2^{b} | 24.13 | 4.63^{b} | 1.66 | 3.99 |
| 11-b | 3.2 | 671.45 | 211.22 | 54.8 | 23.78 | 4.0^{b} | 36.78 | 9.18^{b} | 2.55 | 0.87 |
| 12-b | 3.3 | 806.35 | 247.8 | 39.25 | 32.46 | 0.5 | 48.21 | 93.77 | 4.77 | 5.0 |
| 17 | N/A | >10000 | 142.6 | 244.3 | 593.6 | 3.2^{b} | 103.8 | 31.99 | 6.59 | 134.3 |
| 31-b | 18.1 ^{b} | 424.35 | 23.46 ^{b} | 32.85 | 10.78 | 1.8^{b} | 7.94 | 4.46^{b} | 1.08 | 1.78 |
| 32 | 27.7^{b} | 136.90 | 4.94^{a} | 28.14 | 26.07 | 0.0 | 4.73 | 353.45 | 2.23 | 8.13 |
| 38-b | 4.5 | 3158.00 | 701.25 | 49.06 | 36.43 | 0.7 | 9.75 | 13.21 | 3.84 | 2.21 |

| | | | | | | | | | | |
|---|---|---|---|---|---|---|---|---|---|---|
| Note: "^{a}" indicates superiority over dexmedetomidine hydrochloride, "^{b}" indicates comparability to dexmedetomidine hydrochloride, and "N/A" indicates not applicable. | | | | | | | | | | |

### Test Example 3. Hypnotic Efficacy

### 3.1. Experimental method

The sequential method was used to determine the ED₅₀ (median effective dose) for the loss of righting reflex (LORR) by the compounds of the present disclosure in rats: Male SD rats, weighing 200-300 grams, were used. Each compound was dissolved in normal saline and administered via tail vein injection at an initial dose at a constant injection rate over 10 s (one rat per dose). If a rat exhibited loss of righting reflex after administration of the initial dose, the next rat was given a lower dose (0.8 times the initial dose); if a rat did not exhibit loss of righting reflex after administration of the initial dose, the next rat was given a higher dose (1.25 times the initial dose). This process was repeated, and the results were recorded in the AOT425 Statpm software until the software could calculate the ED₅₀, at which point the experiment was terminated. The hypnotic efficacy of the compounds of the present disclosure was evaluated using ED₅₀ with lower ED₅₀ values indicating stronger efficacy.

Comparison of onset time and duration: Male SD rats, weighing 200-300 grams, were used. Each compound was dissolved in normal saline and administered via tail vein injection at a constant injection rate over 10 s, with 10 rats per group. The injection dose was twice the ED₅₀ (ED₅₀: the dose at which 50% of rats exhibit loss of righting reflex). The onset time and duration were recorded.

The onset time refers to the period from the completion of injection to when the rat is placed in a supine position, with the criterion for loss of righting reflex defined as the rat's inability to spontaneously revert to a prone position using its forelimbs and maintained for ≥10 s, which serves as an indicator of compound efficacy; the duration refers to the sustained period during which the rat remains in a state of losing righting reflex. The spontaneous recovery of the righting reflex in the rat is considered the cessation of the efficacy.

### 3.2. Experimental results

The results are shown in Table 3:

**Table 3. Hypnotic efficacy data**

| **Compound** | **ED₅₀ (µg/kg)** | **Dose of administration (µg/kg)** | **Onset time (S)** | **Duration (S)** |
|---|---|---|---|---|
| Dexmedetomidine hydrochloride | 10 | 20 | 77.80±28.56 | 3275.10±796.79 |
| 5 | 62.5 | 125 | 99.20±29.98 | 531.10±559.16** |
| 8 | 21.25 | 42.5 | 161.70±101.69 | 635.80±697.73** |
| 10 | 8.8 | 17.6 | 219.90±93.88** | 508.10±572.65** |
| 11-b | 25.6 | 50.0 | 45.20±7.38** | 666.00±376.66** |
| 12-b | 70.2 | 150.0 | 71.20±17.71 | 1751.50±672.18** |
| 17 | 276.3 | 552.6 | 101.80±154.86 | 1602.50±681.51** |
| 31-b | 26.8 | 60.0 | 100.44±49.16 | 1147.78±656.35** |
| 38-b | 32.0 | 64.0 | 102.44±52.34 | 228.22±158.93** |

| | | | | |
|---|---|---|---|---|
| Note: Compared with dexmedetomidine hydrochloride, ^{**}*p* < 0.01. | | | | |

The experimental results showed that the hypnotic onset times of compounds 5, 8, 10, 12-b, 17, 31-b, and 38-b of the present disclosure were comparable to or slightly longer than that of dexmedetomidine hydrochloride; however, all these compounds could induce sleep in rats within 5 min, exhibiting definite and reversible hypnotic effects. The hypnotic onset time of compound 11-b, as well as the duration of compounds 5, 8, 10, 11-b, 12-b, 17, 31-b, and 38-b, were all significantly shorter than those of dexmedetomidine hydrochloride, which expands other application scenarios of the compounds.

### 4. Fluctuations in blood pressure and heart rate after administration

### 4.1. Experimental method

Male SD rats, weighing 250-300 g, with 4 rats per group, were maintained under general anesthesia by isoflurane inhalation. Femoral artery cannulation was performed, and blood pressure and heart rate were monitored using a Powerlab data acquisition and analysis system. 30 min after the baseline values stabilized, the baseline mean arterial pressure (MAP₀) and baseline heart rate (HR₀) were measured. The rats were given a solution of each compound in normal saline via tail vein injection (the dose of each compound was the same as in Test Example 3, with rapid intravenous injection over 10 s). Blood pressure and heart rate were continuously recorded for 90 min. MAP⁺ (the difference between the maximum mean arterial pressure (MAPₘₐₓ) and MAP₀), MAP⁻(the difference between the minimum mean arterial pressure (MAPₘᵢₙ) and MAP₀), and HR⁻ (the difference between the minimum heart rate (HRₘᵢₙ) and HR₀) within 90 min were calculated. For calculation, the maximum increase rate of mean arterial pressure = MAP⁺/MAP₀ × 100%; the maximum decrease rate of mean arterial pressure = MAP⁻/MAP₀ × 100%; and the maximum decrease rate of heart rate = HR⁻/HR₀ × 100%. See Table 4 for details.

### 4.2. Experimental results

No significant fluctuations in blood pressure or heart rate were observed in rats after normal saline injection (with fluctuation amplitude less than 10%). After administration of dexmedetomidine hydrochloride, the animals exhibited a biphasic change in MAP, characterized by an initial increase followed by a decrease, with sustained reductions in MAP and HR for 90 min.

The maximum increase rates of MAP for compounds 5, 10, 11-b, and 31-b were lower than that of dexmedetomidine hydrochloride. No significant decreases greater than 10% in MAP were observed for compounds 5, 8, 10, 11-b, and 31-b. The maximum decrease rates of HR for compounds 5, 8, 10, 11-b, and 31-b were all lower than that of dexmedetomidine hydrochloride, and the recovery of HR was faster.

**Table 4. Fluctuations in blood pressure and heart rate after administration**

| **Compound** | **MAP increase** | | **MAP decrease** | | **HR decrease** | |
|---|---|---|---|---|---|---|
| | **Maximum increase rate (%)** | **Duration (min)** | **Maximum decrease rate (%)** | **Duration (min)** | **Maximum decrease rate (%)** | **Duration (min)** |
| Normal saline | 3.0±4.3 | / | 1.7±4.7 | / | / | / |
| Dexmedetomidine hydrochloride | 27.7±4.1 | 2 | 16.5±14.6 | 90 | 33.8±5.3 | 90 |
| 5 | 19.9±6.3 | 2 | 7.4±9.2 | / | 24.7±4.5* | 30 |
| 8 | 32.0±10.5 | 2 | 7.1±4.1 | / | 24.9±10.8 | 60 |
| 10 | 17.3±5.0* | 2 | 7.9±7.4 | / | 24.2±5.7* | 30 |
| 11-b | 24.1±4.96 | 2 | 6.7±4.02 | / | 31.2±3.19 | 60 |
| 31-b | 24.0±11.1 | 2 | 5.3±4.6 | / | 30.2±7.7 | 60 |

| | | | | | | |
|---|---|---|---|---|---|---|
| Note: "/" indicates no relevant effect or not applicable; compared with dexmedetomidine hydrochloride, ^{*}*p* < 0.05. | | | | | | |

## Claims

1. A compound represented by formula I or a pharmaceutically acceptable salt thereof, a stereoisomer thereof, or a tautomer thereof, 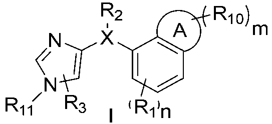
wherein X is selected from CH and N, and preferably, X is CH;
n is selected from integers of 1-3, and preferably, n is 1;
m is selected from integers of 1-8, preferably integers of 1-3;
each R₁ is independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl, and the optional substituent is independently selected from halogen and hydroxyl;
R₂ is selected from H, cyano, optionally substituted C₁-C₈ alkyl, optionally substituted C₂-C₈ alkenyl, and optionally substituted C₂-C₈ alkynyl, and the optional substituent is independently selected from halogen and hydroxyl;
R₃ is selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl, and the optional substituent is independently selected from halogen and hydroxyl;
ring A is selected from C₃-C₈ cycloalkyl, C₃-C₈ cycloalkenyl, 3- to 8-membered heterocyclyl containing 1-3 heteroatoms independently selected from oxygen, nitrogen, and sulfur, and 5- to 8-membered heteroaryl containing 1-3 heteroatoms independently selected from oxygen and sulfur, preferably C₃-C₈ cycloalkyl, 3- to 8-membered heterocyclyl containing 1 heteroatom selected from oxygen, nitrogen, and sulfur, and 5- to 8-membered heteroaryl containing 1 heteroatom selected from oxygen and sulfur; each R₁₀ is independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl;
R₁₁ is selected from H, optionally substituted C₁-C₈ alkyl, optionally substituted C₂-C₈ alkenyl, and optionally substituted C₂-C₈ alkynyl, preferably H and optionally substituted C₁-C₅ alkyl, and further preferably, R₁₁ is H; the optional substituent is selected from hydroxyl and halogen;
and the compound of formula I is not: 5-(2,3-dihydro-benzofuran-7-ylmethyl)-1-imidazole, 8-((1H-imidazol-5-yl)methyl)-1,2,3,4-tetrahydroquinoline, 5-(benzofuran-7-ylmethyl)-1H-imidazole, or 5-((5-bromobenzofuran-7-yl)methyl)-1H-imidazole.

2. The compound represented by formula I or the pharmaceutically acceptable salt thereof, the stereoisomer thereof, or the tautomer thereof according to claim 1, wherein one or more of the following conditions are met:
(1) ring A is selected from C₃-C₈ cycloalkyl, C₃-C₈ cycloalkenyl, 4- to 6-membered heterocyclyl containing 1 heteroatom selected from oxygen, nitrogen, and sulfur, and 5- to 6-membered heteroaryl containing 1 heteroatom selected from oxygen and sulfur, preferably azetidinyl, oxetanyl, thietanyl, thienyl, furanyl, thiopyranyl, pyranyl, tetrahydropyrrolyl, hexahydropyridinyl, dihydropyrrolyl, tetrahydropyridinyl, tetrahydrothienyl, dihydropyridinyl, dihydrothienyl, tetrahydrothiopyranyl, dihydrothiopyranyl, tetrahydrofuranyl, dihydrofuranyl, tetrahydropyranyl, dihydropyranyl, cyclopropyl, cyclobutyl, cyclopentyl, cyclohexyl, cyclobutenyl, cyclopentenyl, cyclopentadienyl, cyclohexenyl, and cyclohexadienyl, and further preferably tetrahydropyrrolyl, hexahydropyridinyl, dihydropyrrolyl, tetrahydropyridinyl, thienyl, furanyl, thiopyranyl, pyranyl, dihydropyridinyl, tetrahydrothiopyranyl, dihydrothiopyranyl, tetrahydrothienyl, dihydrothienyl, tetrahydrofuranyl, dihydrofuranyl, cyclobutyl, cyclobutenyl, cyclopentyl, cyclohexyl, cyclopentenyl, cyclopentadienyl, and cyclohexenyl;
(2) R₁ is selected from H, halogen, optionally substituted C₁-C₈ alkyl, optionally substituted C₁-C₈ alkoxy, hydroxyl, optionally substituted C₁-C₈ alkylthio, and optionally substituted C₃-C₆ cycloalkyl, preferably H, halogen, optionally substituted C₁-C₅ alkyl, optionally substituted C₁-C₅ alkoxy, hydroxyl, optionally substituted C₁-C₅ alkylthio, and optionally substituted C₃-C₆ cycloalkyl, further preferably H, halogen, optionally substituted C₁-C₅ alkyl, and hydroxyl, and further preferably H, -F, -Cl, and optionally substituted C₁-C₅ alkyl, and further preferably, R₁ is H or -F; the optional substituent is selected from hydroxyl and halogen, and preferably, the optional substituent is F;
(3) R₃ is selected from H, halogen, optionally substituted C₁-C₈ alkyl, optionally substituted C₁-C₈ alkoxy, hydroxyl, optionally substituted C₁-C₈ alkylthio, and optionally substituted C₃-C₆ cycloalkyl, preferably H, halogen, optionally substituted C₁-C₅ alkyl, optionally substituted C₁-C₅ alkoxy, hydroxyl, and optionally substituted C₃-C₆ cycloalkyl, and further preferably H, halogen, and optionally substituted C₁-C₅ alkyl, and still further preferably, R₃ is H; the optional substituent is selected from hydroxyl and halogen, and preferably, the optional substituent is F;
(4) each R₁₀ is independently selected from H, halogen, optionally substituted C₁-C₈ alkyl, optionally substituted C₁-C₈ alkoxy, hydroxyl, optionally substituted C₁-C₈ alkylthio, and optionally substituted C₃-C₆ cycloalkyl, preferably H, halogen, optionally substituted C₁-C₅ alkyl, optionally substituted C₁-C₅ alkoxy, hydroxyl, optionally substituted C₁-C₅ alkylthio, and optionally substituted C₃-C₆ cycloalkyl, and further preferably H, halogen, and optionally substituted C₁-C₅ alkyl; the optional substituent is independently selected from halogen, C₁-C₈ alkyl, and hydroxyl, preferably halogen, C₁-C₅ alkyl, and hydroxyl, and further preferably, the optional substituent is halogen, still further preferably F;
(5) R₂ is selected from H and optionally substituted C₁-C₈ alkyl, preferably H and optionally substituted C₁-C₅ alkyl, and further preferably optionally substituted C₁-C₅ alkyl; the optional substituent is independently selected from halogen and hydroxyl.

3. The compound represented by formula I or the pharmaceutically acceptable salt thereof, the stereoisomer thereof, or the tautomer thereof according to any one of claims 1-2, wherein the compound represented by formula I is represented by formula I-a' or formula I-b':

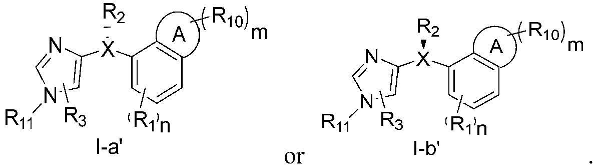

4. The compound represented by formula I or the pharmaceutically acceptable salt thereof, the stereoisomer thereof, or the tautomer thereof according to any one of claims 1-3, wherein:
ring A is selected from 4- to 6-membered heterocyclyl containing one oxygen atom and 5- to 6-membered heteroaryl containing one oxygen atom, preferably 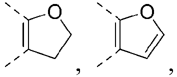 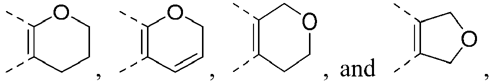 and further preferably 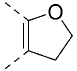 and 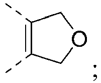 R₂ is selected from optionally substituted C₁-C₈ alkyl; the substituent is independently selected from halogen and hydroxyl.

5. The compound represented by formula I or the pharmaceutically acceptable salt thereof, the stereoisomer thereof, or the tautomer thereof according to any one of claims 1-3, wherein:
ring A is selected from 4- to 6-membered heterocyclyl containing one sulfur atom and 5- to 6-membered heteroaryl containing one sulfur atom, preferably 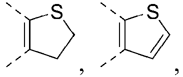 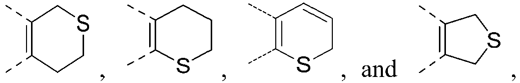 and further preferably

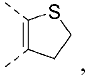

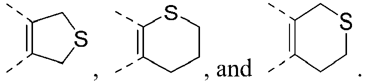

6. The compound represented by formula I or the pharmaceutically acceptable salt thereof, the stereoisomer thereof, or the tautomer thereof according to any one of claims 1-3, wherein:
ring A is selected from 4- to 6-membered heterocyclyl containing one nitrogen atom,
preferably 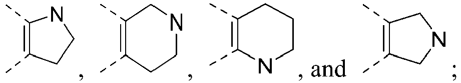
R₂ is selected from optionally substituted C₁-C₈ alkyl; the substituent is independently selected from halogen and hydroxyl.

7. The compound represented by formula I or the pharmaceutically acceptable salt thereof, the stereoisomer thereof, or the tautomer thereof according to any one of claims 1-3, wherein:
ring A is selected from C₃-C₈ cycloalkyl, preferably 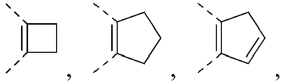 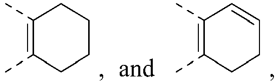 and further preferably 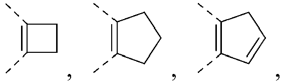 and 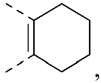 and still further preferably, ring A is

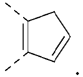

8. The compound represented by formula I or the pharmaceutically acceptable salt thereof, the stereoisomer thereof, or the tautomer thereof according to any one of claims 1-4, wherein the compound represented by formula I is represented by formula II or formula III: 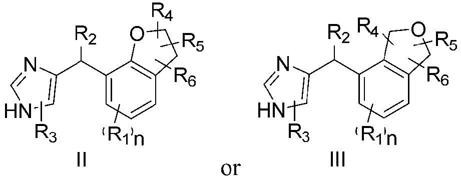
wherein R₂ is selected from optionally substituted C₁-C₈ alkyl, and the optional substituent is independently selected from halogen and hydroxyl;
n, R₁, and R₃ are as defined for the corresponding groups in claims 1-4;
R₄, R₅, and R₆ are each independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl.

9. The compound represented by formula I or the pharmaceutically acceptable salt thereof, the stereoisomer thereof, or the tautomer thereof according to any one of claims 1-3, and 7, wherein the compound represented by formula I is represented by formula V or formula VI: 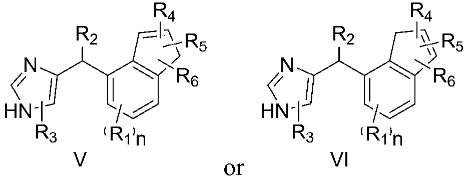
wherein n, R₁, R₂, and R₃ are as defined for the corresponding groups in claims 1-3 and 7;
R₄, R₅, and R₆ are each independently selected from H, halogen, hydroxyl, nitryl, cyano, optionally substituted C₁-C₈ alkyl, optionally substituted C₁-C₈ alkoxy, optionally substituted C₂-C₈ alkenyl, optionally substituted C₂-C₈ alkynyl, optionally substituted C₁-C₈ alkylthio, and optionally substituted C₃-C₈ cycloalkyl; the optional substituent is independently selected from halogen, C₁-C₈ alkyl, and hydroxyl.

10. The compound or the pharmaceutically acceptable salt thereof, the stereoisomer thereof, or the tautomer thereof according to any one of claims 1-3, wherein the compound or the pharmaceutically acceptable salt thereof, the stereoisomer thereof, or the tautomer thereof is selected from the following compounds: 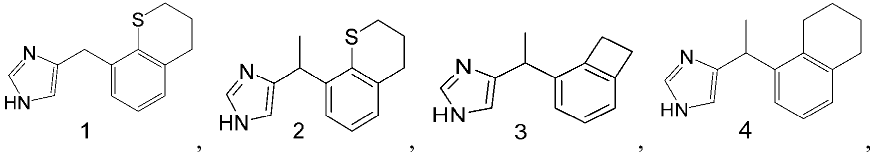 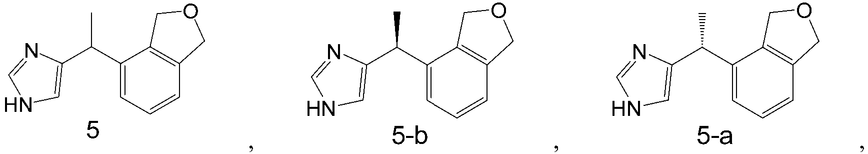 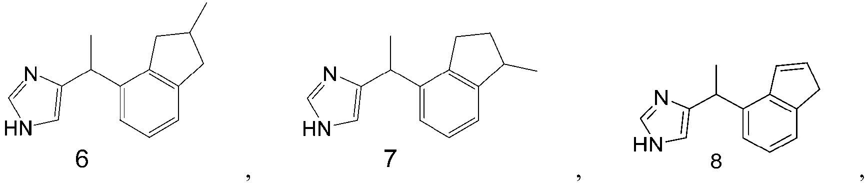 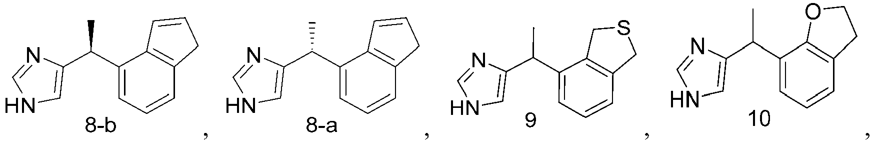 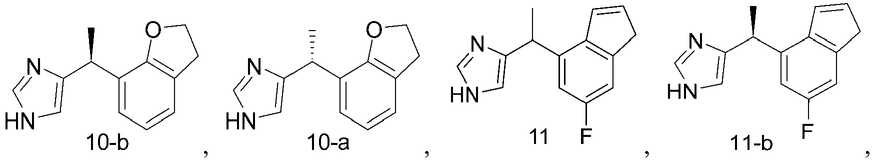 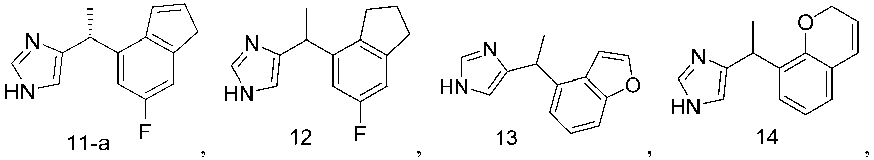 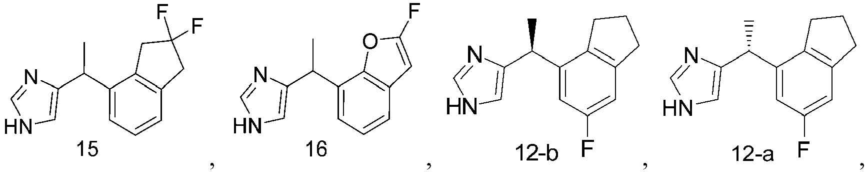 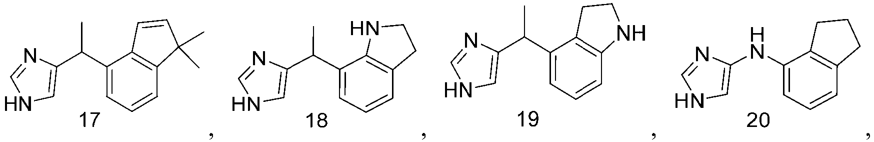 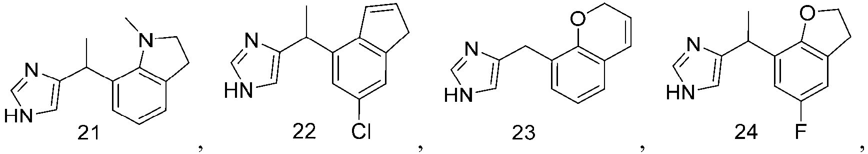 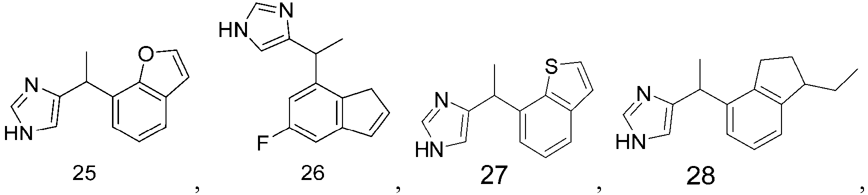 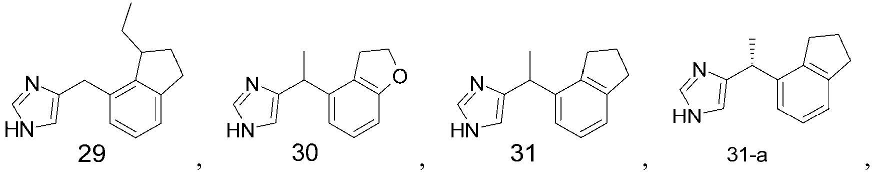 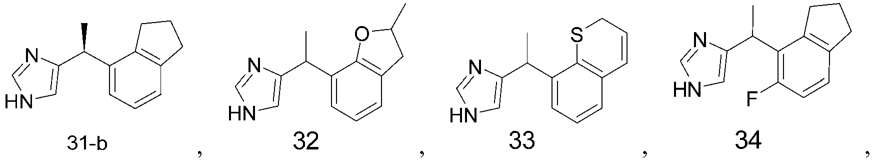 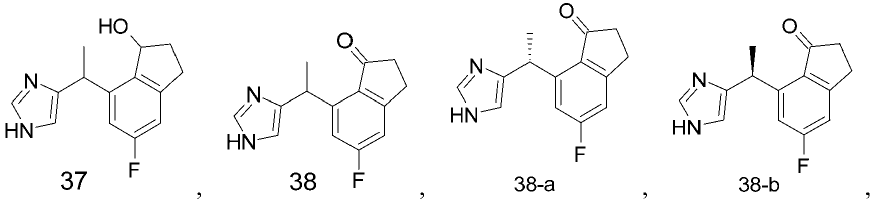 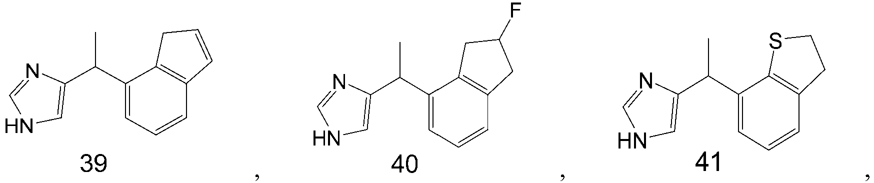 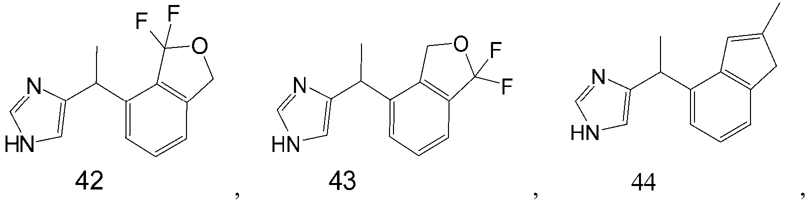 and

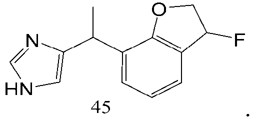

11. A pharmaceutical composition, comprising the compound or the pharmaceutically acceptable salt thereof, the stereoisomer thereof, or the tautomer thereof according to any one of claims 1-10, and a pharmaceutically acceptable carrier.

12. Use of the compound or the pharmaceutically acceptable salt thereof, the stereoisomer thereof, or the tautomer thereof according to any one of claims 1-10, or the pharmaceutical composition according to claim 11 in the preparation of a medicament associated with an α2 adrenoceptor agonist, preferably in the preparation of a medicament for producing sedative, and/or analgesic, and/or anesthetic effects, and/or for anti-anxiety, and/or for inhibiting sympathetic nerve activity, and/or for treating insomnia, and/or for treating a mental disorder in a human or an animal, and further preferably in the preparation of a medicament for producing sedative, and/or analgesic, and/or anesthetic effects, and/or for treating insomnia, and/or for treating a mental disorder in a human or an animal.

13. A compound represented by formula I-A, formula I-A', formula I-B, formula I-B', formula I-C, formula I-D, or formula I-D' or a salt thereof, 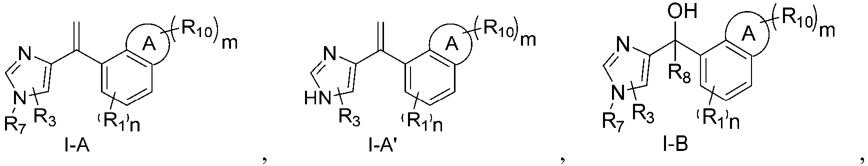 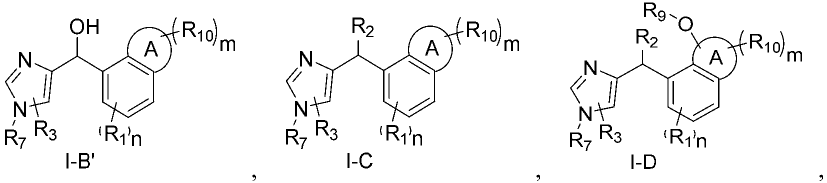 or 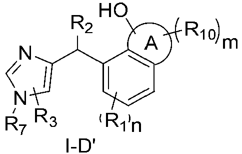 wherein
in formula I-A, n, ring A, R₁, R₃, m, and R₁₀ are as defined for the corresponding groups in claims 1-10;
in formula I-A', n, ring A, R₁, R₃, m, and R₁₀ are as defined for the corresponding groups in claims 1-10;
in formula I-B, n, ring A, R₁, R₃, m, and R₁₀ are as defined for the corresponding groups in claims 1-9;
R₈ is selected from C₁-C₅ alkyl, preferably methyl, ethyl, and propyl, and further preferably, R₈ is methyl;
in formula I-B', n, ring A, R₁, R₃, m, and R₁₀ are as defined for the corresponding groups in claims 1-9;
in formula I-C, n, ring A, R₁, R₂, R₃, m, and R₁₀ are as defined for the corresponding groups in claims 1-9;
in formula I-D, n, ring A, R₁, R₂, R₃, m, and R₁₀ are as defined for the corresponding groups in claims 1-9;
R₉ is selected from a hydroxyl protecting group, preferably a silyl ether hydroxyl protecting group, further preferably trimethylsilyl, tert-butyldiphenylsilyl, tertbutyldimethylsilyl, and triisopropylsilyl, and still further preferably tertbutyldimethylsilyl;
in formula I-D', n, ring A, R₁, R₂, R₃, m, and R₁₀ are as defined for the corresponding groups in claims 1-10;
R₇ in formula I-A, formula I-B, formula I-B', formula I-C, formula I-D, or formula I-D' is selected from an amino protecting group, and preferably, R₇ is trityl.

14. A preparation method for the compound according to any one of claims 1-10, comprising the following steps: subjecting an intermediate I-B-1 to a dehydration reaction to give an intermediate I-A, then performing a hydrogenation reduction reaction to give an intermediate I-C, and subjecting the intermediate I-C to removal of an amino protecting group to give a compound represented by formula I-1; or subjecting an intermediate I-B-1 to one-step removal of an amino protecting group and water to give an intermediate I-A', and then performing a hydrogenation reduction reaction to give a compound represented by formula I-1; or subjecting an intermediate I-B' to one-step removal of an amino protecting group and a reduction reaction simultaneously to give a compound represented by formula I-2; or subjecting an intermediate I-D to removal of a hydroxyl protecting group to give an intermediate I-D', and subjecting the intermediate I-D' to removal of an amino protecting group and dehydration to give a compound represented by formula I; 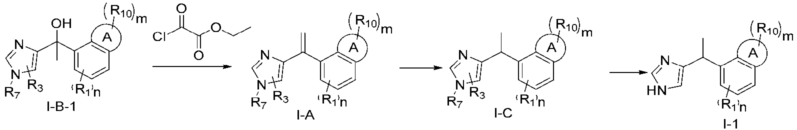 or 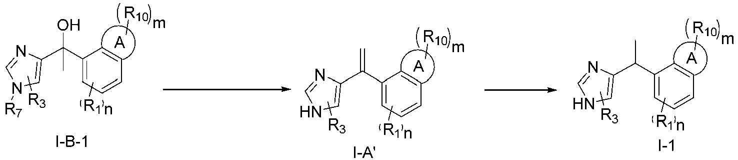 or 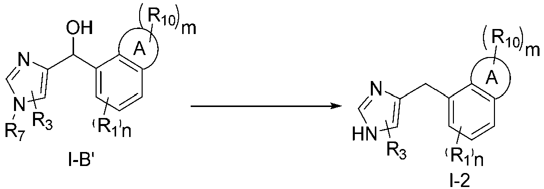 or 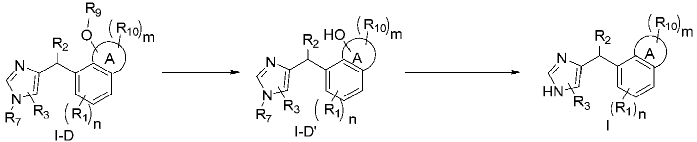
wherein n, ring A, R₁, R₂, R₃, m, and R₁₀ are as defined for the corresponding groups in claims 1-10;
R₇ is selected from an amino protecting group, and preferably, R₇ is trityl;
R₉ is selected from a hydroxyl protecting group, preferably a silyl ether hydroxyl protecting group, further preferably trimethylsilyl, tert-butyldiphenylsilyl, tertbutyldimethylsilyl, and triisopropylsilyl, and still further preferably tertbutyldimethylsilyl.
